# EUROPÄISCHE PATENTSCHRIFT

(11) **EP 1 414 977 B1**
(45) Veröffentlichungstag und Bekanntmachung des Hinweises auf die Patenterteilung: **02.01.2008**
(21) Anmeldenummer: 02794523.7
(22) Anmeldetag: 27.07.2002
(51) Int. Cl.: C12N 15/62, C12N 9/28, C11D 3/386

(54) **WASCH- UND REINIGUNGSMITTEL MIT HYBRID-ALPHA-AMYLASEN**
DETERGENT AND CLEANING AGENT WITH HYBRID ALPHA AMYLASES
AGENTS DETERGENTS ET NETTOYANTS CONTENANT DES ALPHA-AMYLASES HYBRIDES

(30) Priorität: 07.08.2001 DE 10138753
(43) Veröffentlichungstag der Anmeldung: 06.05.2004
(73) Patentinhaber: Henkel Kommanditgesellschaft auf Aktien, 40589 Düsseldorf (DE)
(72) Erfinder: KOTTWITZ, Beatrix, 40593 Düsseldorf (DE); BREVES, Roland, 40822 Mettmann (DE); MAURER, Karl-Heinz, 40699 Erkrath (DE)
(86) Internationale Anmeldenummer: PCT/EP2002/008391
(87) Internationale Veröffentlichungsnummer: WO 2003/014358

(56) Entgegenhaltungen:
- WO-A-00/60059
- WO-A-96/23874
- WO-A-97/41213
- CONRAD B ET AL: "HYBRID BACILLUS AMULOLIQUEFACIENS X BACILLUS LICHENIFORMIS ALPHA-AMYLASES CONSTRUCTION, PROPERTIES AND SEQUENCE DETERMINANTS" EUROPEAN JOURNAL OF BIOCHEMISTRY, BERLIN, DE, Bd. 230, Nr. 2, 1995, Seiten 481-490, XP000960424 ISSN: 0014-2956 in der Anmeldung erwähnt
- DATABASE EBI [Online] 21. Juli 1986 (1986-07-21) "Alpha-amylase precursor (EC 3.2.1.1)" Database accession no. P00692 XP002232998
- DATABASE EBI [Online] 1. Januar 1988 (1988-01-01) "Alpha-amylase precursor (EC 3.2.1.1)" Database accession no. P06278 XP002232999

### SEQUENZPROTOKOLL

<110> Henkel Kommanditgesellschaft auf Aktien
<120> Wasch- und Reinigungsmittel mit Hybrid-Alpha-Amylasen
<130> H 4714 PCT
<140>
   <141>
<150> DE 10138753.9-41
   <151> 2001-08-07
<160> 18
<170> PatentIn Ver. 2.1
<210> 1
   <211> 1452
   <212> DNA
   <213> Bacillus licheniformis
<220>
   <221> CDS
   <222> (1)..(1452)
<400> 1
   gca aat ctt aat ggg acg ctg atg cag tat ttt gaa tgg tac atg ccc      48
   Ala Asn Leu Asn Gly Thr Leu Met Gln Tyr Phe Glu Trp Tyr Met Pro
    1               5                  10                  15
   aat gac ggc caa cat tgg aag cgc ttg caa aac gac tcg gca tat ttg      96
   Asn Asp Gly Gln His Trp Lys Arg Leu Gln Asn Asp Ser Ala Tyr Leu
               20                  25                  30
   gct gaa cac ggt att act gcc gtc tgg att ccc ccg gca tat aag gga     144
   Ala Glu His Gly Ile Thr Ala Val Trp Ile Pro Pro Ala Tyr Lys Gly
           35                  40                  45
   acg agc caa gcg gat gtg ggc tac ggt gct tac gac ctt tat gat tta     192
   Thr Ser Gln Ala Asp Val Gly Tyr Gly Ala Tyr Asp Leu Tyr Asp Leu
       50                  55                  60
   ggg gag ttt cat caa aaa ggg acg gtt cgg aca aag tac ggc aca aaa     240
   Gly Glu Phe His Gln Lys Gly Thr Val Arg Thr Lys Tyr Gly Thr Lys
   65                  70                  75                  80
   gga gag ctg caa tct gcg atc aaa agt ctt cat tcc cgc gac att aac     288
   Gly Glu Leu Gln Ser Ala Ile Lys Ser Leu His Ser Arg Asp Ile Asn
                   85                  90                  95
   gtt tac ggg gat gtg gtc atc aac cac aaa ggc ggc gct gat gcg acc     336
   Val Tyr Gly Asp Val Val Ile Asn His Lys Gly Gly Ala Asp Ala Thr
               100                 105                 110
   gaa gat gta acc gcg gtt gaa gtc gat ccc gct gac cgc aac cgc gta     384
   Glu Asp Val Thr Ala Val Glu Val Asp Pro Ala Asp Arg Asn Arg Val
           115                 120                 125
   att tca gga gaa cac cga att aaa gcc tgg aca cat ttt cat ttt ccg    432
   Ile Ser Gly Glu His Arg Ile Lys Ala Trp Thr His Phe His Phe Pro
       130                 135                 140
   ggg cgc ggc agc aca tac agc gat ttt aaa tgg cat tgg tac cat ttt    480
   Gly Arg Gly Ser Thr Tyr Ser Asp Phe Lys Trp His Trp Tyr His Phe
   145                 150                 155                 160
   gac gga acc gat tgg gac gag tcc cga aag ctg aac cgc atc tat aag    528
   Asp Gly Thr Asp Trp Asp Glu Ser Arg Lys Leu Asn Arg Ile Tyr Lys
                   165                 170                 175
   ttt caa gga aag gct tgg gat tgg gaa gtt tcc aat gaa aac ggc aac    576
   Phe Gln Gly Lys Ala Trp Asp Trp Glu Val Ser Asn Glu Asn Gly Asn
               180                 185                 190
   tat gat tat ttg atg tat gcc gac atc gat tat gac cat cct gat gtc    624
   Tyr Asp Tyr Leu Met Tyr Ala Asp Ile Asp Tyr Asp His Pro Asp Val
           195                 200                 205
   gca gca gaa att aag aga tgg ggc act tgg tat gcc aat gaa ctg caa    672
   Ala Ala Glu Ile Lys Arg Trp Gly Thr Trp Tyr Ala Asn Glu Leu Gln
       210                 215                 220
   ttg gac ggt ttc cgt ctt gat gct gtc aaa cac att aaa ttt tct ttt    720
   Leu Asp Gly Phe Arg Leu Asp Ala Val Lys His Ile Lys Phe Ser Phe
   225                 230                 235                 240
   ttg cgg gat tgg gtt aat cat gtc agg gaa aaa acg ggg aag gaa atg    768
   Leu Arg Asp Trp Val Asn His Val Arg Glu Lys Thr Gly Lys Glu Met
                   245                 250                 255
   ttt acg gta gct gaa tat tgg cag aat gac ttg ggc gcg ctg gaa aac    816
   Phe Thr Val Ala Glu Tyr Trp Gln Asn Asp Leu Gly Ala Leu Glu Asn
               260                 265                 270
   tat ttg aac aaa aca aat ttt aat cat tca gtg ttt gac gtg ccg ctt    864
   Tyr Leu Asn Lys Thr Asn Phe Asn His Ser Val Phe Asp Val Pro Leu
           275                 280                 285
   cat tat cag ttc cat gct gca tcg aca cag gga ggc ggc tat gat atg    912
   His Tyr Gln Phe His Ala Ala Ser Thr Gln Gly Gly Gly Tyr Asp Met
       290                 295                 300
   agg aaa ttg ctg aac agt acg gtc gtt tcc aag cat ccg ttg aaa gcg    960
   Arg Lys Leu Leu Asn Ser Thr Val Val Ser Lys His Pro Leu Lys Ala
   305                 310                 315                 320
   gtt aca ttt gtc gat aac cat gat aca cag ccg ggg caa tcg ctt gag    1008
   Val Thr Phe Val Asp Asn His Asp Thr Gln Pro Gly Gln Ser Leu Glu
                   325                 330                 335
   tcg act gtc caa aca tgg ttt aag ccg ctt gct tac gct ttt att ctc    1056
   Ser Thr Val Gln Thr Trp Phe Lys Pro Leu Ala Tyr Ala Phe Ile Leu
               340                 345                 350
   aca agg gaa tct gga tac cct cag gtt ttc tac ggg gat atg tac ggg    1104
   Thr Arg Glu Ser Gly Tyr Pro Gln Val Phe Tyr Gly Asp Met Tyr Gly
           355                 360                 365
   acg aaa gga gac tcc cag cgc gaa att cct gcc ttg aaa cac aaa att    1152
   Thr Lys Gly Asp Ser Gln Arg Glu Ile Pro Ala Leu Lys His Lys Ile
       370                 375                 380
   gaa ccg atc tta aaa gcg aga aaa cag tat gcg tac gga gca cag cat    1200
   Glu Pro Ile Leu Lys Ala Arg Lys Gln Tyr Ala Tyr Gly Ala Gln His
   385                 390                 395                 400
   gat tat ttc gac cac cat gac att gtc ggc tgg aca agg gaa ggc gac    1248
   Asp Tyr Phe Asp His His Asp Ile Val Gly Trp Thr Arg Glu Gly Asp
                   405                 410                 415
   agc tcg gtt gca aat tca ggt ttg gcg gca tta ata aca gac gga ccc    1296
   Ser Ser Val Ala Asn Ser Gly Leu Ala Ala Leu Ile Thr Asp Gly Pro
               420                 425                 430
   ggt ggg gca aag cga atg tat gtc ggc cgg caa aac gcc ggt gag aca    1344
   Gly Gly Ala Lys Arg Met Tyr Val Gly Arg Gln Asn Ala Gly Glu Thr
           435                 440                 445
   tgg cat gac att acc gga aac cgt tcg gag ccg gtt gtc atc aat tcg    1392
   Trp His Asp Ile Thr Gly Asn Arg Ser Glu Pro Val Val Ile Asn Ser
       450                 455                 460
   gaa ggc tgg gga gag ttt cac gta aac ggc ggg tcg gtt tca att tat    1440
   Glu Gly Trp Gly Glu Phe His Val Asn Gly Gly Ser Val Ser Ile Tyr
   465                 470                 475                 480
   gtt caa aga tag                                                    1452
   Val Gln Arg
<210> 2
   <211> 483
   <212> PRT
   <213> Bacillus licheniformis
<400> 2
   Ala Asn Leu Asn Gly Thr Leu Met Gln Tyr Phe Glu Trp Tyr Met Pro
    1               5                  10                  15
   Asn Asp Gly Gln His Trp Lys Arg Leu Gln Asn Asp Ser Ala Tyr Leu
               20                  25                  30
   Ala Glu His Gly Ile Thr Ala Val Trp Ile Pro Pro Ala Tyr Lys Gly
           35                  40                  45
   Thr Ser Gln Ala Asp Val Gly Tyr Gly Ala Tyr Asp Leu Tyr Asp Leu
       50                  55                  60
   Gly Glu Phe His Gln Lys Gly Thr Val Arg Thr Lys Tyr Gly Thr Lys
   65                  70                  75                  80
   Gly Glu Leu Gln Ser Ala Ile Lys Ser Leu His Ser Arg Asp Ile Asn
                   85                  90                  95
   Val Tyr Gly Asp Val Val Ile Asn His Lys Gly Gly Ala Asp Ala Thr
               100                 105                 110
   Glu Asp Val Thr Ala Val Glu Val Asp Pro Ala Asp Arg Asn Arg Val
           115                 120                 125
   Ile Ser Gly Glu His Arg Ile Lys Ala Trp Thr His Phe His Phe Pro
       130                 135                 140
   Gly Arg Gly Ser Thr Tyr Ser Asp Phe Lys Trp His Trp Tyr His Phe
   145                 150                 155                 160
   Asp Gly Thr Asp Trp Asp Glu Ser Arg Lys Leu Asn Arg Ile Tyr Lys
                   165                 170                 175
   Phe Gln Gly Lys Ala Trp Asp Trp Glu Val Ser Asn Glu Asn Gly Asn
               180                 185                 190
   Tyr Asp Tyr Leu Met Tyr Ala Asp Ile Asp Tyr Asp His Pro Asp Val
           195                 200                 205
   Ala Ala Glu Ile Lys Arg Trp Gly Thr Trp Tyr Ala Asn Glu Leu Gln
       210                 215                 220
   Leu Asp Gly Phe Arg Leu Asp Ala Val Lys His Ile Lys Phe Ser Phe
   225                 230                 235                 240
   Leu Arg Asp Trp Val Asn His Val Arg Glu Lys Thr Gly Lys Glu Met
                   245                 250                 255
   Phe Thr Val Ala Glu Tyr Trp Gln Asn Asp Leu Gly Ala Leu Glu Asn
               260                 265                 270
   Tyr Leu Asn Lys Thr Asn Phe Asn His Ser Val Phe Asp Val Pro Leu
           275                 280                 285
   His Tyr Gln Phe His Ala Ala Ser Thr Gln Gly Gly Gly Tyr Asp Met
       290                 295                 300
   Arg Lys Leu Leu Asn Ser Thr Val Val Ser Lys His Pro Leu Lys Ala
   305                 310                 315                 320
   Val Thr Phe Val Asp Asn His Asp Thr Gln Pro Gly Gln Ser Leu Glu
                   325                 330                 335
   Ser Thr Val Gln Thr Trp Phe Lys Pro Leu Ala Tyr Ala Phe Ile Leu
               340                 345                 350
   Thr Arg Glu Ser Gly Tyr Pro Gln Val Phe Tyr Gly Asp Met Tyr Gly
           355                 360                 365
   Thr Lys Gly Asp Ser Gln Arg Glu Ile Pro Ala Leu Lys His Lys Ile
       370                 375                 380
   Glu Pro Ile Leu Lys Ala Arg Lys Gln Tyr Ala Tyr Gly Ala Gln His
   385                 390                 395                 400
   Asp Tyr Phe Asp His His Asp Ile Val Gly Trp Thr Arg Glu Gly Asp
                   405                 410                 415
   Ser Ser Val Ala Asn Ser Gly Leu Ala Ala Leu Ile Thr Asp Gly Pro
               420                 425                 430
   Gly Gly Ala Lys Arg Met Tyr Val Gly Arg Gln Asn Ala Gly Glu Thr
           435                 440                 445
   Trp His Asp Ile Thr Gly Asn Arg Ser Glu Pro Val Val Ile Asn Ser
       450                 455                 460
   Glu Gly Trp Gly Glu Phe His Val Asn Gly Gly Ser Val Ser Ile Tyr
   465                 470                 475                 480
   Val Gln Arg
<210> 3
   <211> 1452
   <212> DNA
   <213> Bacillus amyloliquefaciens
<220>
   <221> CDS
   <222> (1)..(1452)
<400> 3
   gta aat ggc acg ctg atg cag tat ttt gaa tgg tat acg ccg aac gac    48
   Val Asn Gly Thr Leu Met Gln Tyr Phe Glu Trp Tyr Thr Pro Asn Asp
    1               5                  10                  15
   ggc cag cat tgg aaa cga ttg cag aat gat gcg gaa cat tta tcg gat    96
   Gly Gln His Trp Lys Arg Leu Gln Asn Asp Ala Glu His Leu Ser Asp
                   20                  25                  30
   atc gga atc act gcc gtc tgg att cct ccc gca tac aaa gga ttg agc    144
   Ile Gly Ile Thr Ala Val Trp Ile Pro Pro Ala Tyr Lys Gly Leu Ser
               35                  40                  45
   caa tcc gat aac gga tac gga cct tat gat ttg tat gat tta gga gaa    192
   Gln Ser Asp Asn Gly Tyr Gly Pro Tyr Asp Leu Tyr Asp Leu Gly Glu
       50                  55                  60
   ttc cag caa aaa ggg acg gtc aga acg aaa tac ggc aca aaa tca gag    240
   Phe Gln Gln Lys Gly Thr Val Arg Thr Lys Tyr Gly Thr Lys Ser Glu
   65                  70                  75                      80
   ctt caa gat gcg atc ggc tca ctg cat tcc cgg aac gtc caa gta tac    288
   Leu Gln Asp Ala Ile Gly Ser Leu His Ser Arg Asn Val Gln Val Tyr
                   85                  90                  95
   gga gat gtg gtt ttg aat cat aag gct ggt gct gat gca aca gaa gat    336
   Gly Asp Val Val Leu Asn His Lys Ala Gly Ala Asp Ala Thr Glu Asp
               100                 105                 110
   gta act gcc gtc gaa gtc aat ccg gcc aat aga aat cag gaa act tcg    384
   Val Thr Ala Val Glu Val Asn Pro Ala Asn Arg Asn Gln Glu Thr Ser
           115                 120                 125
   gag gaa tat caa atc aaa gcg tgg acg gat ttt cgt ttt ccg ggc cgt    432
   Glu Glu Tyr Gln Ile Lys Ala Trp Thr Asp Phe Arg Phe Pro Gly Arg
       130                 135                 140
   gga aac acg tac agt gat ttt aaa tgg cat tgg tat cat ttc gac gga    480
   Gly Asn Thr Tyr Ser Asp Phe Lys Trp His Trp Tyr His Phe Asp Gly
   145                 150                 155                 160
   gcg gac tgg gat gaa tcc cgg aag atc agc cgc atc ttt aag ttt cgt    528
   Ala Asp Trp Asp Glu Ser Arg Lys Ile Ser Arg Ile Phe Lys Phe Arg
                   165                 170                 175
   ggg gaa gga aaa gcg tgg gat tgg gaa gta tca agt gaa aac ggc aac    576
   Gly Glu Gly Lys Ala Trp Asp Trp Glu Val Ser Ser Glu Asn Gly Asn
               180                 185                 190
   tat gac tat tta atg tat gct gat gtt gac tac gac cac cct gat gtc    624
   Tyr Asp Tyr Leu Met Tyr Ala Asp Val Asp Tyr Asp His Pro Asp Val
           195                 200                 205
   gtg gca gag aca aaa aaa tgg ggt atc tgg tat gcg aat gaa ctg tca    672
   Val Ala Glu Thr Lys Lys Trp Gly Ile Trp Tyr Ala Asn Glu Leu Ser
       210                 215                 220
   tta gac ggc ttc cgt att gat gcc gcc aaa cat att aaa ttt tca ttt    720
   Leu Asp Gly Phe Arg Ile Asp Ala Ala Lys His Ile Lys Phe Ser Phe
   225                 230                 235                 240
   ctg cgt gat tgg gtt cag gcg gtc aga cag gcg acg gga aaa gaa atg    768
   Leu Arg Asp Trp Val Gln Ala Val Arg Gln Ala Thr Gly Lys Glu Met
                   245                 250                 255
   ttt acg gtt gcg gag tat tgg cag aat aat gcc ggg aaa ctc gaa aac    816
   Phe Thr Val Ala Glu Tyr Trp Gln Asn Asn Ala Gly Lys Leu Glu Asn
               260                 265                 270
   tac ttg aat aaa aca agc ttt aat caa tcc gtg ttt gat gtt ccg ctt    864
   Tyr Leu Asn Lys Thr Ser Phe Asn Gln Ser Val Phe Asp Val Pro Leu
           275                 280                 285
   cat ttc aat tta cag gcg gct tcc tca caa gga ggc gga tat gat atg    912
   His Phe Asn Leu Gln Ala Ala Ser Ser Gln Gly Gly Gly Tyr Asp Met
       290                 295                 300
   agg cgt ttg ctg gac ggt acc gtt gtg tcc agg cat ccg gaa aag gcg    960
   Arg Arg Leu Leu Asp Gly Thr Val Val Ser Arg His Pro Glu Lys Ala
   305                 310                 315                 320
   gtt aca ttt gtt gaa aat cat gac aca cag ccg gga cag tca ttg gaa   1008
   Val Thr Phe Val Glu Asn His Asp Thr Gln Pro Gly Gln Ser Leu Glu
                   325                 330                 335
   tcg aca gtc caa act tgg ttt aaa ccg ctt gca tac gcc ttt att ttg   1056
   Ser Thr Val Gln Thr Trp Phe Lys Pro Leu Ala Tyr Ala Phe Ile Leu
               340                 345                 350
   aca aga gaa tcc ggt tat cct cag gtg ttc tat ggg gat atg tac ggg   1104
   Thr Arg Glu Ser Gly Tyr Pro Gln Val Phe Tyr Gly Asp Met Tyr Gly
           355                 360                 365
   aca aaa ggg aca tcg cca aag gaa att ccc tca ctg aaa gat aat ata   1152
   Thr Lys Gly Thr Ser Pro Lys Glu Ile Pro Ser Leu Lys Asp Asn Ile
       370                 375                 380
   gag ccg att tta aaa gcg cgt aag gag tac gca tac ggg ccc cag cac   1200
   Glu Pro Ile Leu Lys Ala Arg Lys Glu Tyr Ala Tyr Gly Pro Gln His
   385                 390                 395                 400
   gat tat att gac cac ccg gat gtg atc gga tgg acg agg gaa ggt gac   1248
   Asp Tyr Ile Asp His Pro Asp Val Ile Gly Trp Thr Arg Glu Gly Asp
                   405                 410                 415
   agc tcc gcc gcc aaa tca ggt ttg gcc gct tta atc acg gac gga ccc   1296
   Ser Ser Ala Ala Lys Ser Gly Leu Ala Ala Leu Ile Thr Asp Gly Pro
               420                 425                 430
   ggc gga tca aag cgg atg tat gcc ggc ctg aaa aat gcc ggc gag aca   1344
   Gly Gly Ser Lys Arg Met Tyr Ala Gly Leu Lys Asn Ala Gly Glu Thr
           435                 440                 445
   tgg tat gac ata acg ggc aac cgt tca gat act gta aaa atc gga tct   1392
   Trp Tyr Asp Ile Thr Gly Asn Arg Ser Asp Thr Val Lys Ile Gly Ser
       450                 455                 460
   gac ggc tgg gga gag ttt cat gta aac gat ggg tcc gtc tcc att tat   1440
   Asp Gly Trp Gly Glu Phe His Val Asn Asp Gly Ser Val Ser Ile Tyr
   465                 470                 475                 480
   gtt cag aaa taa                                                   1452
   Val Gln Lys
<210> 4
   <211> 483
   <212> PRT
   <213> Bacillus amyloliquefaciens
<400> 4
   Val Asn Gly Thr Leu Met Gln Tyr Phe Glu Trp Tyr Thr Pro Asn Asp
    1               5                  10                  15
   Gly Gln His Trp Lys Arg Leu Gln Asn Asp Ala Glu His Leu Ser Asp
               20                  25                  30
   Ile Gly Ile Thr Ala Val Trp Ile Pro Pro Ala Tyr Lys Gly Leu Ser
           35                  40                  45
   Gln Ser Asp Asn Gly Tyr Gly Pro Tyr Asp Leu Tyr Asp Leu Gly Glu
       50                  55                  60
   Phe Gln Gln Lys Gly Thr Val Arg Thr Lys Tyr Gly Thr Lys Ser Glu
    65                  70                  75                  80
   Leu Gln Asp Ala Ile Gly Ser Leu His Ser Arg Asn Val Gln Val Tyr
                   85                  90                  95
   Gly Asp Val Val Leu Asn His Lys Ala Gly Ala Asp Ala Thr Glu Asp
               100                 105                 110
   Val Thr Ala Val Glu Val Asn Pro Ala Asn Arg Asn Gln Glu Thr Ser
           115                 120                 125
   Glu Glu Tyr Gln Ile Lys Ala Trp Thr Asp Phe Arg Phe Pro Gly Arg
       130                 135                 140
   Gly Asn Thr Tyr Ser Asp Phe Lys Trp His Trp Tyr His Phe Asp Gly
   145                 150                 155                 160
   Ala Asp Trp Asp Glu Ser Arg Lys Ile Ser Arg Ile Phe Lys Phe Arg
                   165                 170                 175
   Gly Glu Gly Lys Ala Trp Asp Trp Glu Val Ser Ser Glu Asn Gly Asn
               180                 185                 190
   Tyr Asp Tyr Leu Met Tyr Ala Asp Val Asp Tyr Asp His Pro Asp Val
           195                 200                 205
   Val Ala Glu Thr Lys Lys Trp Gly Ile Trp Tyr Ala Asn Glu Leu Ser
       210                 215                 220
   Leu Asp Gly Phe Arg Ile Asp Ala Ala Lys His Ile Lys Phe Ser Phe
   225                 230                 235                 240
   Leu Arg Asp Trp Val Gln Ala Val Arg Gln Ala Thr Gly Lys Glu Met
                   245                 250                 255
   Phe Thr Val Ala Glu Tyr Trp Gln Asn Asn Ala Gly Lys Leu Glu Asn
               260                 265                 270
   Tyr Leu Asn Lys Thr Ser Phe Asn Gln Ser Val Phe Asp Val Pro Leu
           275                 280                 285
   His Phe Asn Leu Gln Ala Ala Ser Ser Gln Gly Gly Gly Tyr Asp Met
       290                 295                 300
   Arg Arg Leu Leu Asp Gly Thr Val Val Ser Arg His Pro Glu Lys Ala
   305                 310                 315                 320
   Val Thr Phe Val Glu Asn His Asp Thr Gln Pro Gly Gln Ser Leu Glu
                   325                 330                 335
   Ser Thr Val Gln Thr Trp Phe Lys Pro Leu Ala Tyr Ala Phe Ile Leu
               340                 345                 350
   Thr Arg Glu Ser Gly Tyr Pro Gln Val Phe Tyr Gly Asp Met Tyr Gly
           355                 360                 365
   Thr Lys Gly Thr Ser Pro Lys Glu Ile Pro Ser Leu Lys Asp Asn Ile
       370                 375                 380
   Glu Pro Ile Leu Lys Ala Arg Lys Glu Tyr Ala Tyr Gly Pro Gln His
   385                 390                 395                 400
   Asp Tyr Ile Asp His Pro Asp Val Ile Gly Trp Thr Arg Glu Gly Asp
                   405                     410                     415
   Ser Ser Ala Ala Lys Ser Gly Leu Ala Ala Leu Ile Thr Asp Gly Pro
               420                     425                     430
   Gly Gly Ser Lys Arg Met Tyr Ala Gly Leu Lys Asn Ala Gly Glu Thr
           435                     440                     445
   Trp Tyr Asp Ile Thr Gly Asn Arg Ser Asp Thr Val Lys Ile Gly Ser
       450                     455                     460
   Asp Gly Trp Gly Glu Phe His Val Asn Asp Gly Ser Val Ser Ile Tyr
   465                     470                     475                     480
   Val Gln Lys
<210> 5
   <211> 1446
   <212> DNA
   <213> Künstliche Sequenz
<220>
   <223> Beschreibung der künstlichen Sequenz:Fusion der Alpha-Amylase-Gene von B. licheniformis und B. amyloliquefaciens (AL34).
<220>
   <221> CDS
   <222> (1)..(1446)
<400> 5
   gta aat ggc acg ctg atg cag tat ttt gaa tgg tat acg ccg aac gac    48
   Val Asn Gly Thr Leu Met Gln Tyr Phe Glu Trp Tyr Thr Pro Asn Asp
    1               5                  10                  15
   ggc cag cat tgg aaa cga ttg cag aat gat gcg gaa cat tta tcg gat    96
   Gly Gln His Trp Lys Arg Leu Gln Asn Asp Ala Glu His Leu Ser Asp
               20                  25                  30
   atc ggt att act gcc gtc tgg att ccc ccg gca tat aag gga acg agc   144
   Ile Gly Ile Thr Ala Val Trp Ile Pro Pro Ala Tyr Lys Gly Thr Ser
           35                  40                  45
   caa gcg gat gtg ggc tac ggt gct tac gac ctt tat gat tta ggg gag   192
   Gln Ala Asp Val Gly Tyr Gly Ala Tyr Asp Leu Tyr Asp Leu Gly Glu
       50                  55                  60
   ttt cat caa aaa ggg acg gtt cgg aca aag tac ggc aca aaa gga gag   240
   Phe His Gln Lys Gly Thr Val Arg Thr Lys Tyr Gly Thr Lys Gly Glu
   65                  70                  75                  80
   ctg caa tct gcg atc aaa agt ctt cat tcc cgc gac att aac gtt tac   288
   Leu Gln Ser Ala Ile Lys Ser Leu His Ser Arg Asp Ile Asn Val Tyr
                   85                  90                  95
   ggg gat gtg gtc atc aac cac aaa ggc ggc gct gat gcg acc gaa gat   336
   Gly Asp Val Val Ile Asn His Lys Gly Gly Ala Asp Ala Thr Glu Asp
               100                 105                 110
   gta acc gcg gtt gaa gtc gat ccc gct gac cgc aac cgc gta att tca   384
   Val Thr Ala Val Glu Val Asp Pro Ala Asp Arg Asn Arg Val Ile Ser
           115                 120                 125
   gga gaa cac cga att aaa gcc tgg aca cat ttt cat ttt ccg ggg cgc    432
   Gly Glu His Arg Ile Lys Ala Trp Thr His Phe His Phe Pro Gly Arg
       130                 135                 140
   ggc agc aca tac agc gat ttt aaa tgg cat tgg tac cat ttt gac gga    480
   Gly Ser Thr Tyr Ser Asp Phe Lys Trp His Trp Tyr His Phe Asp Gly
   145                 150                 155                 160
   acc gat tgg gac gag tcc cga aag ctg aac cgc atc tat aag ttt caa    528
   Thr Asp Trp Asp Glu Ser Arg Lys Leu Asn Arg Ile Tyr Lys Phe Gln
                   165                 170                 175
   gga aag gct tgg gat tgg gaa gtt tcc aat gaa aac ggc aac tat gat    576
   Gly Lys Ala Trp Asp Trp Glu Val Ser Asn Glu Asn Gly Asn Tyr Asp
               180                 185                 190
   tat ttg atg tat gcc gac atc gat tat gac cat cct gat gtc gca gca    624
   Tyr Leu Met Tyr Ala Asp Ile Asp Tyr Asp His Pro Asp Val Ala Ala
           195                 200                 205
   gaa att aag aga tgg ggc act tgg tat gcc aat gaa ctg caa ttg gac    672
   Glu Ile Lys Arg Trp Gly Thr Trp Tyr Ala Asn Glu Leu Gln Leu Asp
       210                 215                 220
   ggt ttc cgt ctt gat gct gtc aaa cac att aaa ttt tct ttt ttg cgg    720
   Gly Phe Arg Leu Asp Ala Val Lys His Ile Lys Phe Ser Phe Leu Arg
   225                 230                 235                 240
   gat tgg gtt aat cat gtc agg gaa aaa acg ggg aag gaa atg ttt acg    768
   Asp Trp Val Asn His Val Arg Glu Lys Thr Gly Lys Glu Met Phe Thr
                   245                 250                 255
   gta gct gaa tat tgg cag aat gac ttg ggc gcg ctg gaa aac tat ttg    816
   Val Ala Glu Tyr Trp Gln Asn Asp Leu Gly Ala Leu Glu Asn Tyr Leu
               260                 265                 270
   aac aaa aca aat ttt aat cat tca gtg ttt gac gtg ccg ctt cat tat    864
   Asn Lys Thr Asn Phe Asn His Ser Val Phe Asp Val Pro Leu His Tyr
           275                 280                 285
   cag ttc cat gct gca tcg aca cag gga ggc ggc tat gat atg agg aaa    912
   Gln Phe His Ala Ala Ser Thr Gln Gly Gly Gly Tyr Asp Met Arg Lys
       290                 295                 300
   ttg ctg aac agt acg gtc gtt tcc aag cat ccg ttg aaa gcg gtt aca    960
   Leu Leu Asn Ser Thr Val Val Ser Lys His Pro Leu Lys Ala Val Thr
   305                 310                 315                 320
   ttt gtc gat aac cat gat aca cag ccg ggg caa tcg ctt gag tcg act    1008
   Phe Val Asp Asn His Asp Thr Gln Pro Gly Gln Ser Leu Glu Ser Thr
                   325                 330                 335
   gtc caa aca tgg ttt aag ccg ctt gct tac gct ttt att ctc aca agg    1056
   Val Gln Thr Trp Phe Lys Pro Leu Ala Tyr Ala Phe Ile Leu Thr Arg
               340                 345                 350
   gaa tct gga tac cct cag gtt ttc tac ggg gat atg tac ggg acg aaa    1104
   Glu Ser Gly Tyr Pro Gln Val Phe Tyr Gly Asp Met Tyr Gly Thr Lys
           355                 360                 365
   gga gac tcc cag cgc gaa att cct gcc ttg aaa cac aaa att gaa ccg     1152
   Gly Asp Ser Gln Arg Glu Ile Pro Ala Leu Lys His Lys Ile Glu Pro
       370                 375                 380
   atc tta aaa gcg aga aaa cag tat gcg tac gga gca cag cat gat tat     1200
   Ile Leu Lys Ala Arg Lys Gln Tyr Ala Tyr Gly Ala Gln His Asp Tyr
   385                 390                 395                 400
   ttc gac cac cat gac att gtc ggc tgg aca agg gaa ggc gac agc tcg     1248
   Phe Asp His His Asp Ile Val Gly Trp Thr Arg Glu Gly Asp Ser Ser
                   405                 410                 415
   gtt gca aat tca ggt ttg gcg gca tta ata aca gac gga ccc ggt ggg     1296
   Val Ala Asn Ser Gly Leu Ala Ala Leu Ile Thr Asp Gly Pro Gly Gly
               420                 425                 430
   gca aag cga atg tat gtc ggc cgg caa aac gcc ggt gag aca tgg cat     1344
   Ala Lys Arg Met Tyr Val Gly Arg Gln Asn Ala Gly Glu Thr Trp His
           435                 440                 445
   gac att acc gga aac cgt tcg gag ccg gtt gtc atc aat tcg gaa ggc     1392
   Asp Ile Thr Gly Asn Arg Ser Glu Pro Val Val Ile Asn Ser Glu Gly
       450                 455                 460
   tgg gga gag ttt cac gta aac ggc ggg tcg gtt tca att tat gtt caa     1440
   Trp Gly Glu Phe His Val Asn Gly Gly Ser Val Ser Ile Tyr Val Gln
   465                 470                 475                 480
   aga tag                                                             1446
   Arg
<210> 6
   <211> 481
   <212> PRT
   <213> Künstliche Sequenz
   <223> Beschreibung der künstlichen Sequenz:Fusion der Alpha-Amylase-Gene von B. licheniformis und B. amyloliquefaciens (AL34).
<400> 6
   Val Asn Gly Thr Leu Met Gln Tyr Phe Glu Trp Tyr Thr Pro Asn Asp
   1 5 10 15
   Gly Gln His Trp Lys Arg Leu Gln Asn Asp Ala Glu His Leu Ser Asp
   20 25 30
   Ile Gly Ile Thr Ala Val Trp Ile Pro Pro Ala Tyr Lys Gly Thr Ser
   35 40 45
   Gln Ala Asp Val Gly Tyr Gly Ala Tyr Asp Leu Tyr Asp Leu Gly Glu
   50 55 60
   Phe His Gln Lys Gly Thr Val Arg Thr Lys Tyr Gly Thr Lys Gly Glu
   65 70 75 80
   Leu Gln Ser Ala Ile Lys Ser Leu His Ser Arg Asp Ile Asn Val Tyr
   85 90 95
   Gly Asp Val Val Ile Asn His Lys Gly Gly Ala Asp Ala Thr Glu Asp
   100 105 110
   Val Thr Ala Val Glu Val Asp Pro Ala Asp Arg Asn Arg Val Ile Ser
   115 120 125
   Gly Glu His Arg Ile Lys Ala Trp Thr His Phe His Phe Pro Gly Arg
       130                 135                 140
   Gly Ser Thr Tyr Ser Asp Phe Lys Trp His Trp Tyr His Phe Asp Gly
   145                 150                 155                 160
   Thr Asp Trp Asp Glu Ser Arg Lys Leu Asn Arg Ile Tyr Lys Phe Gln
                   165                 170                 175
   Gly Lys Ala Trp Asp Trp Glu Val Ser Asn Glu Asn Gly Asn Tyr Asp
               180                 185                 190
   Tyr Leu Met Tyr Ala Asp Ile Asp Tyr Asp His Pro Asp Val Ala Ala
           195                 200                 205
   Glu Ile Lys Arg Trp Gly Thr Trp Tyr Ala Asn Glu Leu Gln Leu Asp
       210                 215                 220
   Gly Phe Arg Leu Asp Ala Val Lys His Ile Lys Phe Ser Phe Leu Arg
   225                 230                 235                 240
   Asp Trp Val Asn His Val Arg Glu Lys Thr Gly Lys Glu Met Phe Thr
                   245                 250                 255
   Val Ala Glu Tyr Trp Gln Asn Asp Leu Gly Ala Leu Glu Asn Tyr Leu
               260                 265                 270
   Asn Lys Thr Asn Phe Asn His Ser Val Phe Asp Val Pro Leu His Tyr
           275                 280                 285
   Gln Phe His Ala Ala Ser Thr Gln Gly Gly Gly Tyr Asp Met Arg Lys
       290                 295                 300
   Leu Leu Asn Ser Thr Val Val Ser Lys His Pro Leu Lys Ala Val Thr
   305                 310                 315                 320
   Phe Val Asp Asn His Asp Thr Gln Pro Gly Gln Ser Leu Glu Ser Thr
                   325                 330                 335
   Val Gln Thr Trp Phe Lys Pro Leu Ala Tyr Ala Phe Ile Leu Thr Arg
               340                 345                 350
   Glu Ser Gly Tyr Pro Gln Val Phe Tyr Gly Asp Met Tyr Gly Thr Lys
           355                 360                 365
   Gly Asp Ser Gln Arg Glu Ile Pro Ala Leu Lys His Lys Ile Glu Pro
       370                 375                 380
   Ile Leu Lys Ala Arg Lys Gln Tyr Ala Tyr Gly Ala Gln His Asp Tyr
   385                 390                 395                 400
   Phe Asp His His Asp Ile Val Gly Trp Thr Arg Glu Gly Asp Ser Ser
                   405                 410                 415
   Val Ala Asn Ser Gly Leu Ala Ala Leu Ile Thr Asp Gly Pro Gly Gly
               420                 425                 430
   Ala Lys Arg Met Tyr Val Gly Arg Gln Asn Ala Gly Glu Thr Trp His
           435                 440                 445
   Asp Ile Thr Gly Asn Arg Ser Glu Pro Val Val Ile Asn Ser Glu Gly
       450                 455                 460
   Trp Gly Glu Phe His Val Asn Gly Gly Ser Val Ser Ile Tyr Val Gln
   465                 470                 475                 480
   Arg
<210> 7
   <211> 1446
   <212> DNA
   <213> Künstliche Sequenz
<220>
<223> Beschreibung der künstlichen Sequenz:Fusion der Alpha-Amylase-Gene von B. licheniformis und B. amyloliquefaciens (AL76).
<220>
   <221> CDS
   <222> (1).. (1446)
<400> 7
   gta aat ggc acg ctg atg cag tat ttt gaa tgg tat acg ccg aac gac    48
   Val Asn Gly Thr Leu Met Gln Tyr Phe Glu Trp Tyr Thr Pro Asn Asp
    1               5                  10                  15
   ggc cag cat tgg aaa cga ttg cag aat gat gcg gaa cat tta tcg gat    96
   Gly Gln His Trp Lys Arg Leu Gln Asn Asp Ala Glu His Leu Ser Asp
               20                  25                  30
   atc gga atc act gcc gtc tgg att cct ccc gca tac aaa gga ttg agc    144
   Ile Gly Ile Thr Ala Val Trp Ile Pro Pro Ala Tyr Lys Gly Leu Ser
           35                  40                  45
   caa tcc gat aac gga tac gga cct tat gat ttg tat gat tta gga gaa    192
   Gln Ser Asp Asn Gly Tyr Gly Pro Tyr Asp Leu Tyr Asp Leu Gly Glu
       50                  55                  60
   ttc cag caa aaa ggg acg gtc aga acg aaa tac ggc aca aaa gga gag    240
   Phe Gln Gln Lys Gly Thr Val Arg Thr Lys Tyr Gly Thr Lys Gly Glu
   65                  70                  75                  80
   ctg caa tct gcg atc aaa agt ctt cat tcc cgc gac att aac gtt tac    288
   Leu Gln Ser Ala Ile Lys Ser Leu His Ser Arg Asp Ile Asn Val Tyr
                   85                  90                  95
   ggg gat gtg gtc atc aac cac aaa ggc ggc gct gat gcg acc gaa gat    336
   Gly Asp Val Val Ile Asn His Lys Gly Gly Ala Asp Ala Thr Glu Asp
               100                 105                 110
   gta acc gcg gtt gaa gtc gat ccc gct gac cgc aac cgc gta att tca    384
   Val Thr Ala Val Glu Val Asp Pro Ala Asp Arg Asn Arg Val Ile Ser
           115                 120                 125
   gga gaa cac cga att aaa gcc tgg aca cat ttt cat ttt ccg ggg cgc    432
   Gly Glu His Arg Ile Lys Ala Trp Thr His Phe His Phe Pro Gly Arg
       130                 135                 140
   ggc agc aca tac agc gat ttt aaa tgg cat tgg tac cat ttt gac gga    480
   Gly Ser Thr Tyr Ser Asp Phe Lys Trp His Trp Tyr His Phe Asp Gly
   145                 150                 155                 160
   acc gat tgg gac gag tcc cga aag ctg aac cgc atc tat aag ttt caa    528
   Thr Asp Trp Asp Glu Ser Arg Lys Leu Asn Arg Ile Tyr Lys Phe Gln
                   165                 170                 175
   gga aag gct tgg gat tgg gaa gtt tcc aat gaa aac ggc aac tat gat    576
   Gly Lys Ala Trp Asp Trp Glu Val Ser Asn Glu Asn Gly Asn Tyr Asp
               180                 185                 190
   tat ttg atg tat gcc gac atc gat tat gac cat cct gat gtc gca gca    624
   Tyr Leu Met Tyr Ala Asp Ile Asp Tyr Asp His Pro Asp Val Ala Ala
           195                 200                 205
   gaa att aag aga tgg ggc act tgg tat gcc aat gaa ctg caa ttg gac    672
   Glu Ile Lys Arg Trp Gly Thr Trp Tyr Ala Asn Glu Leu Gln Leu Asp
       210                 215                 220
   ggt ttc cgt ctt gat gct gtc aaa cac att aaa ttt tct ttt ttg cgg     720
   Gly Phe Arg Leu Asp Ala Val Lys His Ile Lys Phe Ser Phe Leu Arg
   225                 230                 235                 240
   gat tgg gtt aat cat gtc agg gaa aaa acg ggg aag gaa atg ttt acg     768
   Asp Trp Val Asn His Val Arg Glu Lys Thr Gly Lys Glu Met Phe Thr
                   245                 250                 255
   gta gct gaa tat tgg cag aat gac ttg ggc gcg ctg gaa aac tat ttg     816
   Val Ala Glu Tyr Trp Gln Asn Asp Leu Gly Ala Leu Glu Asn Tyr Leu
               260                 265                 270
   aac aaa aca aat ttt aat cat tca gtg ttt gac gtg ccg ctt cat tat     864
   Asn Lys Thr Asn Phe Asn His Ser Val Phe Asp Val Pro Leu His Tyr
           275                 280                 285
   cag ttc cat gct gca tcg aca cag gga ggc ggc tat gat atg agg aaa     912
   Gln Phe His Ala Ala Ser Thr Gln Gly Gly Gly Tyr Asp Met Arg Lys
       290                 295                 300
   ttg ctg aac agt acg gtc gtt tcc aag cat ccg ttg aaa gcg gtt aca     960
   Leu Leu Asn Ser Thr Val Val Ser Lys His Pro Leu Lys Ala Val Thr
   305                 310                 315                 320
   ttt gtc gat aac cat gat aca cag ccg ggg caa tcg ctt gag tcg act    1008
   Phe Val Asp Asn His Asp Thr Gln Pro Gly Gln Ser Leu Glu Ser Thr
                   325                 330                 335
   gtc caa aca tgg ttt aag ccg ctt gct tac gct ttt att ctc aca agg    1056
   Val Gln Thr Trp Phe Lys Pro Leu Ala Tyr Ala Phe Ile Leu Thr Arg
               340                 345                 350
   gaa tct gga tac cct cag gtt ttc tac ggg gat atg tac ggg acg aaa    1104
   Glu Ser Gly Tyr Pro Gln Val Phe Tyr Gly Asp Met Tyr Gly Thr Lys
           355                 360                 365
   gga gac tcc cag cgc gaa att cct gcc ttg aaa cac aaa att gaa ccg    1152
   Gly Asp Ser Gln Arg Glu Ile Pro Ala Leu Lys His Lys Ile Glu Pro
       370                 375                 380
   atc tta aaa gcg aga aaa cag tat gcg tac gga gca cag cat gat tat    1200
   Ile Leu Lys Ala Arg Lys Gln Tyr Ala Tyr Gly Ala Gln His Asp Tyr
   385                 390                 395                 400
   ttc gac cac cat gac att gtc ggc tgg aca agg gaa ggc gac agc tcg    1248
   Phe Asp His His Asp Ile Val Gly Trp Thr Arg Glu Gly Asp Ser Ser
                   405                 410                 415
   gtt gca aat tca ggt ttg gcg gca tta ata aca gac gga ccc ggt ggg    1296
   Val Ala Asn Ser Gly Leu Ala Ala Leu Ile Thr Asp Gly Pro Gly Gly
               420                 425                 430
   gca aag cga atg tat gtc ggc cgg caa aac gcc ggt gag aca tgg cat    1344
   Ala Lys Arg Met Tyr Val Gly Arg Gln Asn Ala Gly Glu Thr Trp His
           435                 440                 445
   gac att acc gga aac cgt tcg gag ccg gtt gtc atc aat tcg gaa ggc    1392
   Asp Ile Thr Gly Asn Arg Ser Glu Pro Val Val Ile Asn Ser Glu Gly
       450                 455                 460
   tgg gga gag ttt cac gta aac ggc ggg tcg gtt tca att tat gtt caa    1440
   Trp Gly Glu Phe His Val Asn Gly Gly Ser Val Ser Ile Tyr Val Gln
   465                 470                 475                 480
   aga tag                                                            1446
   Arg
<210> 8
   <211> 481
   <212> PRT
   <213> Künstliche Sequenz
   <223> Beschreibung der künstlichen Sequenz:Fusion der Alpha-Amylase-Gene von B. licheniformis und B. amyloliquefaciens (AL76).
<400> 8
   Val Asn Gly Thr Leu Met Gln Tyr Phe Glu Trp Tyr Thr Pro Asn Asp
    1               5                  10                  15
   Gly Gln His Trp Lys Arg Leu Gln Asn Asp Ala Glu His Leu Ser Asp
               20                  25                  30
   Ile Gly Ile Thr Ala Val Trp Ile Pro Pro Ala Tyr Lys Gly Leu Ser
           35                  40                  45
   Gln Ser Asp Asn Gly Tyr Gly Pro Tyr Asp Leu Tyr Asp Leu Gly Glu
       50                  55                  60
   Phe Gln Gln Lys Gly Thr Val Arg Thr Lys Tyr Gly Thr Lys Gly Glu
   65                  70                  75                  80
   Leu Gln Ser Ala Ile Lys Ser Leu His Ser Arg Asp Ile Asn Val Tyr
                   85                  90                  95
   Gly Asp Val Val Ile Asn His Lys Gly Gly Ala Asp Ala Thr Glu Asp
               100                 105                 110
   Val Thr Ala Val Glu Val Asp Pro Ala Asp Arg Asn Arg Val Ile Ser
           115                 120                 125
   Gly Glu His Arg Ile Lys Ala Trp Thr His Phe His Phe Pro Gly Arg
       130                 135                 140
   Gly Ser Thr Tyr Ser Asp Phe Lys Trp His Trp Tyr His Phe Asp Gly
   145                 150                 155                 160
   Thr Asp Trp Asp Glu Ser Arg Lys Leu Asn Arg Ile Tyr Lys Phe Gln
                   165                 170                 175
   Gly Lys Ala Trp Asp Trp Glu Val Ser Asn Glu Asn Gly Asn Tyr Asp
               180                 185                 190
   Tyr Leu Met Tyr Ala Asp Ile Asp Tyr Asp His Pro Asp Val Ala Ala
           195                 200                 205
   Glu Ile Lys Arg Trp Gly Thr Trp Tyr Ala Asn Glu Leu Gln Leu Asp
       210                 215                 220
   Gly Phe Arg Leu Asp Ala Val Lys His Ile Lys Phe Ser Phe Leu Arg
   225                 230                 235                 240
   Asp Trp Val Asn His Val Arg Glu Lys Thr Gly Lys Glu Met Phe Thr
                   245                 250                 255
   Val Ala Glu Tyr Trp Gln Asn Asp Leu Gly Ala Leu Glu Asn Tyr Leu
               260                 265                 270
   Asn Lys Thr Asn Phe Asn His Ser Val Phe Asp Val Pro Leu His Tyr
           275                 280                 285
   Gln Phe His Ala Ala Ser Thr Gln Gly Gly Gly Tyr Asp Met Arg Lys
       290                 295                 300
   Leu Leu Asn Ser Thr Val Val Ser Lys His Pro Leu Lys Ala Val Thr
   305                 310                 315                 320
   Phe Val Asp Asn His Asp Thr Gln Pro Gly Gln Ser Leu Glu Ser Thr
                   325                 330                 335
   Val Gln Thr Trp Phe Lys Pro Leu Ala Tyr Ala Phe Ile Leu Thr Arg
               340                 345                 350
   Glu Ser Gly Tyr Pro Gln Val Phe Tyr Gly Asp Met Tyr Gly Thr Lys
           355                 360                 365
   Gly Asp Ser Gln Arg Glu Ile Pro Ala Leu Lys His Lys Ile Glu Pro
       370                 375                 380
   Ile Leu Lys Ala Arg Lys Gln Tyr Ala Tyr Gly Ala Gln His Asp Tyr
   385                 390                 395                 400
   Phe Asp His His Asp Ile Val Gly Trp Thr Arg Glu Gly Asp Ser Ser
                   405                 410                 415
   Val Ala Asn Ser Gly Leu Ala Ala Leu Ile Thr Asp Gly Pro Gly Gly
               420                 425                 430
   Ala Lys Arg Met Tyr Val Gly Arg Gln Asn Ala Gly Glu Thr Trp His
           435                 440                 445
   Asp Ile Thr Gly Asn Arg Ser Glu Pro Val Val Ile Asn Ser Glu Gly
       450                 455                 460
   Trp Gly Glu Phe His Val Asn Gly Gly Ser Val Ser Ile Tyr Val Gln
   465                 470                 475                 480
   Arg
<210> 9
   <211> 1446
   <212> DNA
   <213> Künstliche Sequenz
<220>
   <223> Beschreibung der künstlichen Sequenz:Fusion der Alpha-Amylase-Gene von B. licheniformis und B. amyloliquefaciens (AL112).
<220>
   <221> CDS
   <222> (1)..(1446)
<400> 9
   gta aat ggc acg ctg atg cag tat ttt gaa tgg tat acg ccg aac gac    48
   Val Asn Gly Thr Leu Met Gln Tyr Phe Glu Trp Tyr Thr Pro Asn Asp
   1               5                   10                  15
   ggc cag cat tgg aaa cga ttg cag aat gat gcg gaa cat tta tcg gat    96
   Gly Gln His Trp Lys Arg Leu Gln Asn Asp Ala Glu His Leu Ser Asp
               20                  25                  30
   atc gga atc act gcc gtc tgg att cct ccc gca tac aaa gga ttg agc    144
   Ile Gly Ile Thr Ala Val Trp Ile Pro Pro Ala Tyr Lys Gly Leu Ser
           35                  40                  45
   caa tcc gat aac gga tac gga cct tat gat ttg tat gat tta gga gaa    192
   Gln Ser Asp Asn Gly Tyr Gly Pro Tyr Asp Leu Tyr Asp Leu Gly Glu
       50                  55                  60
   ttc cag caa aaa ggg acg gtc aga acg aaa tac ggc aca aaa tca gag    240
   Phe Gln Gln Lys Gly Thr Val Arg Thr Lys Tyr Gly Thr Lys Ser Glu
   65                  70                  75                  80
   ctt caa gat gcg atc ggc tca ctg cat tcc cgg aac gtc caa gta tac      288
   Leu Gln Asp Ala Ile Gly Ser Leu His Ser Arg Asn Val Gln Val Tyr
                   85                  90                  95
   gga gat gtg gtt ttg aat cat aag gct ggt gct gat gca aca gaa gat      336
   Gly Asp Val Val Leu Asn His Lys Ala Gly Ala Asp Ala Thr Glu Asp
               100                 105                 110
   gta acc gcg gtt gaa gtc gat ccc gct gac cgc aac cgc gta att tca      384
   Val Thr Ala Val Glu Val Asp Pro Ala Asp Arg Asn Arg Val Ile Ser
           115                 120                 125
   gga gaa cac cga att aaa gcc tgg aca cat ttt cat ttt ccg ggg cgc      432
   Gly Glu His Arg Ile Lys Ala Trp Thr His Phe His Phe Pro Gly Arg
       130                 135                 140
   ggc agc aca tac agc gat ttt aaa tgg cat tgg tac cat ttt gac gga      480
   Gly Ser Thr Tyr Ser Asp Phe Lys Trp His Trp Tyr His Phe Asp Gly
   145                 150                 155                 160
   acc gat tgg gac gag tcc cga aag ctg aac cgc atc tat aag ttt caa      528
   Thr Asp Trp Asp Glu Ser Arg Lys Leu Asn Arg Ile Tyr Lys Phe Gln
                   165                 170                 175
   gga aag gct tgg gat tgg gaa gtt tcc aat gaa aac ggc aac tat gat      576
   Gly Lys Ala Trp Asp Trp Glu Val Ser Asn Glu Asn Gly Asn Tyr Asp
               180                 185                 190
   tat ttg atg tat gcc gac atc gat tat gac cat cct gat gtc gca gca      624
   Tyr Leu Met Tyr Ala Asp Ile Asp Tyr Asp His Pro Asp Val Ala Ala
           195                 200                 205
   gaa att aag aga tgg ggc act tgg tat gcc aat gaa ctg caa ttg gac      672
   Glu Ile Lys Arg Trp Gly Thr Trp Tyr Ala Asn Glu Leu Gln Leu Asp
       210                 215                 220
   ggt ttc cgt ctt gat gct gtc aaa cac att aaa ttt tct ttt ttg cgg      720
   Gly Phe Arg Leu Asp Ala Val Lys His Ile Lys Phe Ser Phe Leu Arg
   225                 230                 235                 240
   gat tgg gtt aat cat gtc agg gaa aaa acg ggg aag gaa atg ttt acg      768
   Asp Trp Val Asn His Val Arg Glu Lys Thr Gly Lys Glu Met Phe Thr
                   245                 250                 255
   gta gct gaa tat tgg cag aat gac ttg ggc gcg ctg gaa aac tat ttg      816
   Val Ala Glu Tyr Trp Gln Asn Asp Leu Gly Ala Leu Glu Asn Tyr Leu
               260                 265                 270
   aac aaa aca aat ttt aat cat tca gtg ttt gac gtg ccg ctt cat tat      864
   Asn Lys Thr Asn Phe Asn His Ser Val Phe Asp Val Pro Leu His Tyr
           275                 280                 285
   cag ttc cat gct gca tcg aca cag gga ggc ggc tat gat atg agg aaa      912
   Gln Phe His Ala Ala Ser Thr Gln Gly Gly Gly Tyr Asp Met Arg Lys
       290                 295                 300
   ttg ctg aac agt acg gtc gtt tcc aag cat ccg ttg aaa gcg gtt aca      960
   Leu Leu Asn Ser Thr Val Val Ser Lys His Pro Leu Lys Ala Val Thr
   305                 310                 315                 320
   ttt gtc gat aac cat gat aca cag ccg ggg caa tcg ctt gag tcg act     1008
   Phe Val Asp Asn His Asp Thr Gln Pro Gly Gln Ser Leu Glu Ser Thr
                   325                     330                     335
   gtc caa aca tgg ttt aag ccg ctt gct tac gct ttt att ctc aca agg     1056
   Val Gln Thr Trp Phe Lys Pro Leu Ala Tyr Ala Phe Ile Leu Thr Arg
               340                     345                     350
   gaa tct gga tac cct cag gtt ttc tac ggg gat atg tac ggg acg aaa     1104
   Glu Ser Gly Tyr Pro Gln Val Phe Tyr Gly Asp Met Tyr Gly Thr Lys
           355                     360                     365
   gga gac tcc cag cgc gaa att cct gcc ttg aaa cac aaa att gaa ccg     1152
   Gly Asp Ser Gln Arg Glu Ile Pro Ala Leu Lys His Lys Ile Glu Pro
       370                     375                     380
   atc tta aaa gcg aga aaa cag tat gcg tac gga gca cag cat gat tat     1200
   Ile Leu Lys Ala Arg Lys Gln Tyr Ala Tyr Gly Ala Gln His Asp Tyr
   385                     390                     395                     400
   ttc gac cac cat gac att gtc ggc tgg aca agg gaa ggc gac agc tcg     1248
   Phe Asp His His Asp Ile Val Gly Trp Thr Arg Glu Gly Asp Ser Ser
                   405                     410                     415
   gtt gca aat tca ggt ttg gcg gca tta ata aca gac gga ccc ggt ggg     1296
   Val Ala Asn Ser Gly Leu Ala Ala Leu Ile Thr Asp Gly Pro Gly Gly
               420                     425                     430
   gca aag cga atg tat gtc ggc cgg caa aac gcc ggt gag aca tgg cat     1344
   Ala Lys Arg Met Tyr Val Gly Arg Gln Asn Ala Gly Glu Thr Trp His
           435                     440                     445
   gac att acc gga aac cgt tcg gag ccg gtt gtc atc aat tcg gaa ggc     1392
   Asp Ile Thr Gly Asn Arg Ser Glu Pro Val Val Ile Asn Ser Glu Gly
       450                     455                     460
   tgg gga gag ttt cac gta aac ggc ggg tcg gtt tca att tat gtt caa     1440
   Trp Gly Glu Phe His Val Asn Gly Gly Ser Val Ser Ile Tyr Val Gln
   465                     470                     475                     480
   aga tag                                                             1446
   Arg
<210> 10
   <211> 481
   <212> PRT
   <213> Künstliche Sequenz
   <223> Beschreibung der künstlichen Sequenz:Fusion der Alpha-Amylase-Gene von B. licheniformis und B. amyloliquefaciens (AL112).
<400> 10
   Val Asn Gly Thr Leu Met Gln Tyr Phe Glu Trp Tyr Thr Pro Asn Asp
    1               5                  10                  15
   Gly Gln His Trp Lys Arg Leu Gln Asn Asp Ala Glu His Leu Ser Asp
               20                  25                  30
   Ile Gly Ile Thr Ala Val Trp Ile Pro Pro Ala Tyr Lys Gly Leu Ser
           35                      40                      45
   Gln Ser Asp Asn Gly Tyr Gly Pro Tyr Asp Leu Tyr Asp Leu Gly Glu
       50                      55                      60
   Phe Gln Gln Lys Gly Thr Val Arg Thr Lys Tyr Gly Thr Lys Ser Glu
   65                      70                      75                      80
   Leu Gln Asp Ala Ile Gly Ser Leu His Ser Arg Asn Val Gln Val Tyr
                   85                      90                      95
   Gly Asp Val Val Leu Asn His Lys Ala Gly Ala Asp Ala Thr Glu Asp
               100                     105                     110
   Val Thr Ala Val Glu Val Asp Pro Ala Asp Arg Asn Arg Val Ile Ser
           115                     120                     125
   Gly Glu His Arg Ile Lys Ala Trp Thr His Phe His Phe Pro Gly Arg
       130                     135                     140
   Gly Ser Thr Tyr Ser Asp Phe Lys Trp His Trp Tyr His Phe Asp Gly
   145                     150                     155                     160
   Thr Asp Trp Asp Glu Ser Arg Lys Leu Asn Arg Ile Tyr Lys Phe Gln
                   165                     170                     175
   Gly Lys Ala Trp Asp Trp Glu Val Ser Asn Glu Asn Gly Asn Tyr Asp
               180                     185                     190
   Tyr Leu Met Tyr Ala Asp Ile Asp Tyr Asp His Pro Asp Val Ala Ala
           195                     200                     205
   Glu Ile Lys Arg Trp Gly Thr Trp Tyr Ala Asn Glu Leu Gln Leu Asp
       210                     215                     220
   Gly Phe Arg Leu Asp Ala Val Lys His Ile Lys Phe Ser Phe Leu Arg
   225                     230                     235                     240
   Asp Trp Val Asn His Val Arg Glu Lys Thr Gly Lys Glu Met Phe Thr
                   245                     250                     255
   Val Ala Glu Tyr Trp Gln Asn Asp Leu Gly Ala Leu Glu Asn Tyr Leu
               260                     265                     270
   Asn Lys Thr Asn Phe Asn His Ser Val Phe Asp Val Pro Leu His Tyr
           275                     280                     285
   Gln Phe His Ala Ala Ser Thr Gln Gly Gly Gly Tyr Asp Met Arg Lys
       290                     295                     300
   Leu Leu Asn Ser Thr Val Val Ser Lys His Pro Leu Lys Ala Val Thr
   305                     310                     315                     320
   Phe Val Asp Asn His Asp Thr Gln Pro Gly Gln Ser Leu Glu Ser Thr
                   325                     330                     335
   Val Gln Thr Trp Phe Lys Pro Leu Ala Tyr Ala Phe Ile Leu Thr Arg
               340                     345                     350
   Glu Ser Gly Tyr Pro Gln Val Phe Tyr Gly Asp Met Tyr Gly Thr Lys
           355                     360                     365
   Gly Asp Ser Gln Arg Glu Ile Pro Ala Leu Lys His Lys Ile Glu Pro
       370                     375                     380
   Ile Leu Lys Ala Arg Lys Gln Tyr Ala Tyr Gly Ala Gln His Asp Tyr
   385                     390                     395                     400
   Phe Asp His His Asp Ile Val Gly Trp Thr Arg Glu Gly Asp Ser Ser
                   405                     410                     415
   Val Ala Asn Ser Gly Leu Ala Ala Leu Ile Thr Asp Gly Pro Gly Gly
               420                     425                     430
   Ala Lys Arg Met Tyr Val Gly Arg Gln Asn Ala Gly Glu Thr Trp His
           435                     440                     445
   Asp Ile Thr Gly Asn Arg Ser Glu Pro Val Val Ile Asn Ser Glu Gly
       450                     455                     460
   Trp Gly Glu Phe His Val Asn Gly Gly Ser Val Ser Ile Tyr Val Gln
   465                     470                     475                     480
   Arg
<210> 11
   <211> 1452
   <212> DNA
   <213> Künstliche Sequenz
<220>
   <223> Beschreibung der künstlichen Sequenz:Fusion der Alpha-Amylase-Gene von B. licheniformis und B. amyloliquefaciens (AL256).
<220>
   <221> CDS
   <222> (1)..(1452)
<400> 11
   gta aat ggc acg ctg atg cag tat ttt gaa tgg tat acg ccg aac gac      48
   Val Asn Gly Thr Leu Met Gln Tyr Phe Glu Trp Tyr Thr Pro Asn Asp
   1               5                   10                  15
   ggc cag cat tgg aaa cga ttg cag aat gat gcg gaa cat tta tcg gat      96
   Gly Gln His Trp Lys Arg Leu Gln Asn Asp Ala Glu His Leu Ser Asp
               20                  25                  30
   atc gga atc act gcc gtc tgg att cct ccc gca tac aaa gga ttg agc      144
   Ile Gly Ile Thr Ala Val Trp Ile Pro Pro Ala Tyr Lys Gly Leu Ser
           35                  40                  45
   caa tcc gat aac gga tac gga cct tat gat ttg tat gat tta gga gaa      192
   Gln Ser Asp Asn Gly Tyr Gly Pro Tyr Asp Leu Tyr Asp Leu Gly Glu
       50                  55                  60
   ttc cag caa aaa ggg acg gtc aga acg aaa tac ggc aca aaa tca gag      240
   Phe Gln Gln Lys Gly Thr Val Arg Thr Lys Tyr Gly Thr Lys Ser Glu
   65                  70                  75                  80
   ctt caa gat gcg atc ggc tca ctg cat tcc cgg aac gtc caa gta tac      288
   Leu Gln Asp Ala Ile Gly Ser Leu His Ser Arg Asn Val Gln Val Tyr
                   85                  90                  95
   gga gat gtg gtt ttg aat cat aag gct ggt gct gat gca aca gaa gat      336
   Gly Asp Val Val Leu Asn His Lys Ala Gly Ala Asp Ala Thr Glu Asp
               100                 105                 110
   gta act gcc gtc gaa gtc aat ccg gcc aat aga aat cag gaa act tcg      384
   Val Thr Ala Val Glu Val Asn Pro Ala Asn Arg Asn Gln Glu Thr Ser
           115                 120                 125
   gag gaa tat caa atc aaa gcg tgg acg gat ttt cgt ttt ccg ggc cgt      432
   Glu Glu Tyr Gln Ile Lys Ala Trp Thr Asp Phe Arg Phe Pro Gly Arg
       130                 135                 140
   gga aac acg tac agt gat ttt aaa tgg cat tgg tat cat ttc gac gga      480
   Gly Asn Thr Tyr Ser Asp Phe Lys Trp His Trp Tyr His Phe Asp Gly
   145                 150                 155                 160
   gcg gac tgg gat gaa tcc cgg aag atc agc cgc atc ttt aag ttt cgt      528
   Ala Asp Trp Asp Glu Ser Arg Lys Ile Ser Arg Ile Phe Lys Phe Arg
                   165                 170                 175
   ggg gaa gga aaa gcg tgg gat tgg gaa gta tca agt gaa aac ggc aac      576
   Gly Glu Gly Lys Ala Trp Asp Trp Glu Val Ser Ser Glu Asn Gly Asn
               180                 185                 190
   tat gac tat tta atg tat gct gat gtt gac tac gac cac cct gat gtc      624
   Tyr Asp Tyr Leu Met Tyr Ala Asp Val Asp Tyr Asp His Pro Asp Val
           195                 200                 205
   gtg gca gag aca aaa aaa tgg ggt atc tgg tat gcg aat gaa ctg tca      672
   Val Ala Glu Thr Lys Lys Trp Gly Ile Trp Tyr Ala Asn Glu Leu Ser
       210                 215                 220
   tta gac ggc ttc cgt att gat gcc gcc aaa cat att aaa ttt tca ttt      720
   Leu Asp Gly Phe Arg Ile Asp Ala Ala Lys His Ile Lys Phe Ser Phe
   225                 230                 235                 240
   ctg cgt gat tgg gtt cag gcg gtc aga cag gcg acg gga aaa gaa atg      768
   Leu Arg Asp Trp Val Gln Ala Val Arg Gln Ala Thr Gly Lys Glu Met
                   245                 250                 255
   ttt acg gta gct gaa tat tgg cag aat gac ttg ggc gcg ctg gaa aac      816
   Phe Thr Val Ala Glu Tyr Trp Gln Asn Asp Leu Gly Ala Leu Glu Asn
               260                 265                 270
   tat ttg aac aaa aca aat ttt aat cat tca gtg ttt gac gtg ccg ctt      864
   Tyr Leu Asn Lys Thr Asn Phe Asn His Ser Val Phe Asp Val Pro Leu
           275                 280                 285
   cat tat cag ttc cat gct gca tcg aca cag gga ggc ggc tat gat atg      912
   His Tyr Gln Phe His Ala Ala Ser Thr Gln Gly Gly Gly Tyr Asp Met
       290                 295                 300
   agg aaa ttg ctg aac agt acg gtc gtt tcc aag cat ccg ttg aaa gcg      960
   Arg Lys Leu Leu Asn Ser Thr Val Val Ser Lys His Pro Leu Lys Ala
   305                 310                 315                 320
   gtt aca ttt gtc gat aac cat gat aca cag ccg ggg caa tcg ctt gag     1008
   Val Thr Phe Val Asp Asn His Asp Thr Gln Pro Gly Gln Ser Leu Glu
                   325                 330                 335
   tcg act gtc caa aca tgg ttt aag ccg ctt gct tac gct ttt att ctc     1056
   Ser Thr Val Gln Thr Trp Phe Lys Pro Leu Ala Tyr Ala Phe Ile Leu
               340                 345                 350
   aca agg gaa tct gga tac cct cag gtt ttc tac ggg gat atg tac ggg     1104
   Thr Arg Glu Ser Gly Tyr Pro Gln Val Phe Tyr Gly Asp Met Tyr Gly
           355                 360                 365
   acg aaa gga gac tcc cag cgc gaa att cct gcc ttg aaa cac aaa att     1152
   Thr Lys Gly Asp Ser Gln Arg Glu Ile Pro Ala Leu Lys His Lys Ile
       370                 375                 380
   gaa ccg atc tta aaa gcg aga aaa cag tat gcg tac gga gca cag cat     1200
   Glu Pro Ile Leu Lys Ala Arg Lys Gln Tyr Ala Tyr Gly Ala Gln His
   385                 390                 395                 400
   gat tat ttc gac cac cat gac att gtc ggc tgg aca agg gaa ggc gac     1248
   Asp Tyr Phe Asp His His Asp Ile Val Gly Trp Thr Arg Glu Gly Asp
                   405                 410                 415
   agc tcg gtt gca aat tca ggt ttg gcg gca tta ata aca gac gga ccc    1296
   Ser Ser Val Ala Asn Ser Gly Leu Ala Ala Leu Ile Thr Asp Gly Pro
               420                 425                 430
   ggt ggg gca aag cga atg tat gtc ggc cgg caa aac gcc ggt gag aca    1344
   Gly Gly Ala Lys Arg Met Tyr Val Gly Arg Gln Asn Ala Gly Glu Thr
           435                 440                 445
   tgg cat gac att acc gga aac cgt tcg gag ccg gtt gtc atc aat tcg    1392
   Trp His Asp Ile Thr Gly Asn Arg Ser Glu Pro Val Val Ile Asn Ser
       450                 455                 460
   gaa ggc tgg gga gag ttt cac gta aac ggc ggg tcg gtt tca att tat    1440
   Glu Gly Trp Gly Glu Phe His Val Asn Gly Gly Ser Val Ser Ile Tyr
   465                 470                 475                 480
   gtt caa aga tag                                                    1452
   Val Gln Arg
<210> 12
   <211> 483
   <212> PRT
   <213> Künstliche Sequenz
   <223> Beschreibung der künstlichen Sequenz:Fusion der Alpha-Amylase-Gene von B. licheniformis und B. amyloliquefaciens (AL256).
<400> 12
   Val Asn Gly Thr Leu Met Gln Tyr Phe Glu Trp Tyr Thr Pro Asn Asp
    1               5                  10                  15
   Gly Gln His Trp Lys Arg Leu Gln Asn Asp Ala Glu His Leu Ser Asp
               20                  25                  30
   Ile Gly Ile Thr Ala Val Trp Ile Pro Pro Ala Tyr Lys Gly Leu Ser
           35                  40                  45
   Gln Ser Asp Asn Gly Tyr Gly Pro Tyr Asp Leu Tyr Asp Leu Gly Glu
       50                  55                  60
   Phe Gln Gln Lys Gly Thr Val Arg Thr Lys Tyr Gly Thr Lys Ser Glu
   65                  70                  75                      80
   Leu Gln Asp Ala Ile Gly Ser Leu His Ser Arg Asn Val Gln Val Tyr
                   85                  90                  95
   Gly Asp Val Val Leu Asn His Lys Ala Gly Ala Asp Ala Thr Glu Asp
               100                 105                 110
   Val Thr Ala Val Glu Val Asn Pro Ala Asn Arg Asn Gln Glu Thr Ser
           115                 120                 125
   Glu Glu Tyr Gln Ile Lys Ala Trp Thr Asp Phe Arg Phe Pro Gly Arg
       130                 135                 140
   Gly Asn Thr Tyr Ser Asp Phe Lys Trp His Trp Tyr His Phe Asp Gly
   145                 150                 155                     160
   Ala Asp Trp Asp Glu Ser Arg Lys Ile Ser Arg Ile Phe Lys Phe Arg
                   165                 170                 175
   Gly Glu Gly Lys Ala Trp Asp Trp Glu Val Ser Ser Glu Asn Gly Asn
               180                 185                 190
   Tyr Asp Tyr Leu Met Tyr Ala Asp Val Asp Tyr Asp His Pro Asp Val
           195                 200                 205
   Val Ala Glu Thr Lys Lys Trp Gly Ile Trp Tyr Ala Asn Glu Leu Ser
       210                 215                 220
   Leu Asp Gly Phe Arg Ile Asp Ala Ala Lys His Ile Lys Phe Ser Phe
   225                 230                 235                 240
   Leu Arg Asp Trp Val Gln Ala Val Arg Gln Ala Thr Gly Lys Glu Met
                   245                 250                 255
   Phe Thr Val Ala Glu Tyr Trp Gln Asn Asp Leu Gly Ala Leu Glu Asn
               260                 265                 270
   Tyr Leu Asn Lys Thr Asn Phe Asn His Ser Val Phe Asp Val Pro Leu
           275                 280                 285
   His Tyr Gln Phe His Ala Ala Ser Thr Gln Gly Gly Gly Tyr Asp Met
       290                 295                 300
   Arg Lys Leu Leu Asn Ser Thr Val Val Ser Lys His Pro Leu Lys Ala
   305                 310                 315                 320
   Val Thr Phe Val Asp Asn His Asp Thr Gln Pro Gly Gln Ser Leu Glu
                   325                 330                 335
   Ser Thr Val Gln Thr Trp Phe Lys Pro Leu Ala Tyr Ala Phe Ile Leu
               340                 345                 350
   Thr Arg Glu Ser Gly Tyr Pro Gln Val Phe Tyr Gly Asp Met Tyr Gly
           355                 360                 365
   Thr Lys Gly Asp Ser Gln Arg Glu Ile Pro Ala Leu Lys His Lys Ile
       370                 375                 380
   Glu Pro Ile Leu Lys Ala Arg Lys Gln Tyr Ala Tyr Gly Ala Gln His
   385                 390                 395                 400
   Asp Tyr Phe Asp His His Asp Ile Val Gly Trp Thr Arg Glu Gly Asp
                   405                 410                 415
   Ser Ser Val Ala Asn Ser Gly Leu Ala Ala Leu Ile Thr Asp Gly Pro
               420                 425                 430
   Gly Gly Ala Lys Arg Met Tyr Val Gly Arg Gln Asn Ala Gly Glu Thr
           435                 440                 445
   Trp His Asp Ile Thr Gly Asn Arg Ser Glu Pro Val Val Ile Asn Ser
       450                 455                 460
   Glu Gly Trp Gly Glu Phe His Val Asn Gly Gly Ser Val Ser Ile Tyr
   465                 470                 475                 480
   Val Gln Arg
<210> 13
   <211> 1452
   <212> DNA
   <213> Künstliche Sequenz
<220>
   <223> Beschreibung der künstlichen Sequenz:Fusion der Alpha-Amylase-Gene von B. licheniformis und B. amyloliquefaciens (ALA34-84).
<220>
   <221> CDS
   <222> (1)..(1952)
<400> 13
   gta aat ggc acg ctg atg cag tat ttt gaa tgg tat acg ccg aac gac    48
   Val Asn Gly Thr Leu Met Gln Tyr Phe Glu Trp Tyr Thr Pro Asn Asp
     1               5                  10                  15
   ggc cag cat tgg aaa cga ttg cag aat gat gcg gaa cat tta tcg gat    96
   Gly Gln His Trp Lys Arg Leu Gln Asn Asp Ala Glu His Leu Ser Asp
                20                  25                  30
   atc ggt att act gcc gtc tgg att ccc ccg gca tat aag gga acg agc   144
   Ile Gly Ile Thr Ala Val Trp Ile Pro Pro Ala Tyr Lys Gly Thr Ser
            35                  40                  45
   caa gcg gat gtg ggc tac ggt gct tac gac ctt tat gat tta ggg gag      192
   Gln Ala Asp Val Gly Tyr Gly Ala Tyr Asp Leu Tyr Asp Leu Gly Glu
        50                  55                  60
   ttt cat caa aaa ggg acg gtt cgg aca aag tac ggc aca aaa gga gag      240
   Phe His Gln Lys Gly Thr Val Arg Thr Lys Tyr Gly Thr Lys Gly Glu
    65                  70                  75                  80
   ctg caa tct gcg atc ggc tca ctg cat tcc cgg aac gtc caa gta tac      288
   Leu Gln Ser Ala Ile Gly Ser Leu His Ser Arg Asn Val Gln Val Tyr
                    85                  90                  95
   gga gat gtg gtt ttg aat cat aag gct ggt gct gat gca aca gaa gat      336
   Gly Asp Val Val Leu Asn His Lys Ala Gly Ala Asp Ala Thr Glu Asp
               100                 105                 110
   gta act gcc gtc gaa gtc aat ccg gcc aat aga aat cag gaa act tcg      384
   Val Thr Ala Val Glu Val Asn Pro Ala Asn Arg Asn Gln Glu Thr Ser
           115                 120                 125
   gag gaa tat caa atc aaa gcg tgg acg gat ttt cgt ttt ccg ggc cgt      432
   Glu Glu Tyr Gln Ile Lys Ala Trp Thr Asp Phe Arg Phe Pro Gly Arg
       130                 135                 140
   gga aac acg tac agt gat ttt aaa tgg cat tgg tat cat ttc gac gga      480
   Gly Asn Thr Tyr Ser Asp Phe Lys Trp His Trp Tyr His Phe Asp Gly
   145                 150                 155                 160
   gcg gac tgg gat gaa tcc cgg aag atc agc cgc atc ttt aag ttt cgt      528
   Ala Asp Trp Asp Glu Ser Arg Lys Ile Ser Arg Ile Phe Lys Phe Arg
                   165                 170                 175
   ggg gaa gga aaa gcg tgg gat tgg gaa gta tca agt gaa aac ggc aac      576
   Gly Glu Gly Lys Ala Trp Asp Trp Glu Val Ser Ser Glu Asn Gly Asn
               180                 185                 190
   tat gac tat tta atg tat gct gat gtt gac tac gac cac cct gat gtc      624
   Tyr Asp Tyr Leu Met Tyr Ala Asp Val Asp Tyr Asp His Pro Asp Val
           195                 200                 205
   gtg gca gag aca aaa aaa tgg ggt atc tgg tat gcg aat gaa ctg tca      672
   Val Ala Glu Thr Lys Lys Trp Gly Ile Trp Tyr Ala Asn Glu Leu Ser
       210                 215                 220
   tta gac ggc ttc cgt att gat gcc gcc aaa cat att aaa ttt tca ttt      720
   Leu Asp Gly Phe Arg Ile Asp Ala Ala Lys His Ile Lys Phe Ser Phe
   225                 230                 235                 240
   ctg cgt gat tgg gtt cag gcg gtc aga cag gcg acg gga aaa gaa atg      768
   Leu Arg Asp Trp Val Gln Ala Val Arg Gln Ala Thr Gly Lys Glu Met
                   245                 250                 255
   ttt acg gtt gcg gag tat tgg cag aat aat gcc ggg aaa ctc gaa aac      816
   Phe Thr Val Ala Glu Tyr Trp Gln Asn Asn Ala Gly Lys Leu Glu Asn
               260                 265                 270
   tac ttg aat aaa aca agc ttt aat caa tcc gtg ttt gat gtt ccg ctt      864
   Tyr Leu Asn Lys Thr Ser Phe Asn Gln Ser Val Phe Asp Val Pro Leu
           275                 280                 285
   cat ttc aat tta cag gcg gct tcc tca caa gga ggc gga tat gat atg      912
   His Phe Asn Leu Gln Ala Ala Ser Ser Gln Gly Gly Gly Tyr Asp Met
       290                 295                 300
   agg cgt ttg ctg gac ggt acc gtt gtg tcc agg cat ccg gaa aag gcg      960
   Arg Arg Leu Leu Asp Gly Thr Val Val Ser Arg His Pro Glu Lys Ala
   305                 310                 315                 320
   gtt aca ttt gtt gaa aat cat gac aca cag ccg gga cag tca ttg gaa      1008
   Val Thr Phe Val Glu Asn His Asp Thr Gln Pro Gly Gln Ser Leu Glu
                   325                 330                 335
   tcg aca gtc caa act tgg ttt aaa ccg ctt gca tac gcc ttt att ttg      1056
   Ser Thr Val Gln Thr Trp Phe Lys Pro Leu Ala Tyr Ala Phe Ile Leu
               340                 345                 350
   aca aga gaa tcc ggt tat cct cag gtg ttc tat ggg gat atg tac ggg      1104
   Thr Arg Glu Ser Gly Tyr Pro Gln Val Phe Tyr Gly Asp Met Tyr Gly
           355                 360                 365
   aca aaa ggg aca tcg cca aag gaa att ccc tca ctg aaa gat aat ata      1152
   Thr Lys Gly Thr Ser Pro Lys Glu Ile Pro Ser Leu Lys Asp Asn Ile
       370                 375                 380
   gag ccg att tta aaa gcg cgt aag gag tac gca tac ggg ccc cag cac      1200
   Glu Pro Ile Leu Lys Ala Arg Lys Glu Tyr Ala Tyr Gly Pro Gln His
   385                 390                 395                 400
   gat tat att gac cac ccg gat gtg atc gga tgg acg agg gaa ggt gac      1248
   Asp Tyr Ile Asp His Pro Asp Val Ile Gly Trp Thr Arg Glu Gly Asp
                   405                 410                 415
   agc tcc gcc gcc aaa tca ggt ttg gcc gct tta atc acg gac gga ccc      1296
   Ser Ser Ala Ala Lys Ser Gly Leu Ala Ala Leu Ile Thr Asp Gly Pro
               420                 425                 430
   ggc gga tca aag cgg atg tat gcc ggc ctg aaa aat gcc ggc gag aca      1344
   Gly Gly Ser Lys Arg Met Tyr Ala Gly Leu Lys Asn Ala Gly Glu Thr
           435                 440                 445
   tgg tat gac ata acg ggc aac cgt tca gat act gta aaa atc gga tct      1392
   Trp Tyr Asp Ile Thr Gly Asn Arg Ser Asp Thr Val Lys Ile Gly Ser
       450                 455                 460
   gac ggc tgg gga gag ttt cat gta aac gat ggg tcc gtc tcc att tat      1440
   Asp Gly Trp Gly Glu Phe His Val Asn Asp Gly Ser Val Ser Ile Tyr
   465                 470                 475                 480
   gtt cag aaa taa                                                      1452
   Val Gln Lys
<210> 14
   <211> 483
   <212> PRT
   <213> Künstliche Sequenz
   <223> Beschreibung der künstlichen Sequenz:Fusion der Alpha-Amylase-Gene von B. licheniformis und B. amyloliquefaciens (ALA34-84).
<400> 14
   Val Asn Gly Thr Leu Met Gln Tyr Phe Glu Trp Tyr Thr Pro Asn Asp
   1               5                   10                  15
   Gly Gln His Trp Lys Arg Leu Gln Asn Asp Ala Glu His Leu Ser Asp
               20                  25                  30
   Ile Gly Ile Thr Ala Val Trp Ile Pro Pro Ala Tyr Lys Gly Thr Ser
           35                  40                  45
   Gln Ala Asp Val Gly Tyr Gly Ala Tyr Asp Leu Tyr Asp Leu Gly Glu
       50                  55                  60
   Phe His Gln Lys Gly Thr Val Arg Thr Lys Tyr Gly Thr Lys Gly Glu
   65                  70                  75                  80
   Leu Gln Ser Ala Ile Gly Ser Leu His Ser Arg Asn Val Gln Val Tyr
                   85                  90                  95
   Gly Asp Val Val Leu Asn His Lys Ala Gly Ala Asp Ala Thr Glu Asp
               100                 105                 110
   Val Thr Ala Val Glu Val Asn Pro Ala Asn Arg Asn Gln Glu Thr Ser
           115                 120                 125
   Glu Glu Tyr Gln Ile Lys Ala Trp Thr Asp Phe Arg Phe Pro Gly Arg
       130                 135                 140
   Gly Asn Thr Tyr Ser Asp Phe Lys Trp His Trp Tyr His Phe Asp Gly
   145                 150                 155                 160
   Ala Asp Trp Asp Glu Ser Arg Lys Ile Ser Arg Ile Phe Lys Phe Arg
                   165                 170                 175
   Gly Glu Gly Lys Ala Trp Asp Trp Glu Val Ser Ser Glu Asn Gly Asn
               180                 185                 190
   Tyr Asp Tyr Leu Met Tyr Ala Asp Val Asp Tyr Asp His Pro Asp Val
           195                 200                 205
   Val Ala Glu Thr Lys Lys Trp Gly Ile Trp Tyr Ala Asn Glu Leu Ser
       210                 215                 220
   Leu Asp Gly Phe Arg Ile Asp Ala Ala Lys His Ile Lys Phe Ser Phe
   225                 230                 235                 240
   Leu Arg Asp Trp Val Gln Ala Val Arg Gln Ala Thr Gly Lys Glu Met
                   245                 250                 255
   Phe Thr Val Ala Glu Tyr Trp Gln Asn Asn Ala Gly Lys Leu Glu Asn
               260                 265                 270
   Tyr Leu Asn Lys Thr Ser Phe Asn Gln Ser Val Phe Asp Val Pro Leu
           275                 280                 285
   His Phe Asn Leu Gln Ala Ala Ser Ser Gln Gly Gly Gly Tyr Asp Met
       290                 295                 300
   Arg Arg Leu Leu Asp Gly Thr Val Val Ser Arg His Pro Glu Lys Ala
   305                 310                 315                 320
   Val Thr Phe Val Glu Asn His Asp Thr Gln Pro Gly Gln Ser Leu Glu
                   325                 330                 335
   Ser Thr Val Gln Thr Trp Phe Lys Pro Leu Ala Tyr Ala Phe Ile Leu
               340                 345                 350
   Thr Arg Glu Ser Gly Tyr Pro Gln Val Phe Tyr Gly Asp Met Tyr Gly
           355                 360                 365
   Thr Lys Gly Thr Ser Pro Lys Glu Ile Pro Ser Leu Lys Asp Asn Ile
       370                 375                 380
   Glu Pro Ile Leu Lys Ala Arg Lys Glu Tyr Ala Tyr Gly Pro Gln His
   385                 390                 395                 400
   Asp Tyr Ile Asp His Pro Asp Val Ile Gly Trp Thr Arg Glu Gly Asp
                   405                 410                 415
   Ser Ser Ala Ala Lys Ser Gly Leu Ala Ala Leu Ile Thr Asp Gly Pro
               420                 425                 430
   Gly Gly Ser Lys Arg Met Tyr Ala Gly Leu Lys Asn Ala Gly Glu Thr
           435                 440                 445
   Trp Tyr Asp Ile Thr Gly Asn Arg Ser Asp Thr Val Lys Ile Gly Ser
       450                 455                 460
   Asp Gly Trp Gly Glu Phe His Val Asn Asp Gly Ser Val Ser Ile Tyr
   465                 470                 475                 480
   Val Gln Lys
<210> 15
   <211> 1458
   <212> DNA
   <213> Künstliche Sequenz
<220>
   <223> Beschreibung der künstlichen Sequenz:Fusion der Alpha-Amylase-Gene von B. licheniformis und B. amyloliquefaciens (LAL19-433).
<220>
   <221> CDS
   <222> (1)..(1458)
<400> 15
   gca aat ctt aat ggg acg ctg atg cag tat ttt gaa tgg tac atg ccc    48
   Ala Asn Leu Asn Gly Thr Leu Met Gln Tyr Phe Glu Trp Tyr Met Pro
    1               5                  10                  15
   aat gac ggc cag cat tgg aaa cga ttg cag aat gat gcg gaa cat tta    96
   Asn Asp Gly Gln His Trp Lys Arg Leu Gln Asn Asp Ala Glu His Leu
               20                  25                  30
   tcg gat atc gga atc act gcc gtc tgg att cct ccc gca tac aaa gga   144
   Ser Asp Ile Gly Ile Thr Ala Val Trp Ile Pro Pro Ala Tyr Lys Gly
           35                  40                  45
   ttg agc caa tcc gat aac gga tac gga cct tat gat ttg tat gat tta   192
   Leu Ser Gln Ser Asp Asn Gly Tyr Gly Pro Tyr Asp Leu Tyr Asp Leu
       50                  55                  60
   gga gaa ttc cag caa aaa ggg acg gtc aga acg aaa tac ggc aca aaa   240
   Gly Glu Phe Gln Gln Lys Gly Thr Val Arg Thr Lys Tyr Gly Thr Lys
   65                  70                  75                  80
   tca gag ctt caa gat gcg atc ggc tca ctg cat tcc cgg aac gtc caa   288
   Ser Glu Leu Gln Asp Ala Ile Gly Ser Leu His Ser Arg Asn Val Gln
                   85                  90                  95
   gta tac gga gat gtg gtt ttg aat cat aag gct ggt gct gat gca aca   336
   Val Tyr Gly Asp Val Val Leu Asn His Lys Ala Gly Ala Asp Ala Thr
               100                 105                 110
   gaa gat gta act gcc gtc gaa gtc aat ccg gcc aat aga aat cag gaa   384
   Glu Asp Val Thr Ala Val Glu Val Asn Pro Ala Asn Arg Asn Gln Glu
           115                 120                 125
   act tcg gag gaa tat caa atc aaa gcg tgg acg gat ttt cgt ttt ccg   432
   Thr Ser Glu Glu Tyr Gln Ile Lys Ala Trp Thr Asp Phe Arg Phe Pro
       130                 135                 140
   ggc cgt gga aac acg tac agt gat ttt aaa tgg cat tgg tat cat ttc     480
   Gly Arg Gly Asn Thr Tyr Ser Asp Phe Lys Trp His Trp Tyr His Phe
   145                 150                 155                 160
   gac gga gcg gac tgg gat gaa tcc cgg aag atc agc cgc atc ttt aag     528
   Asp Gly Ala Asp Trp Asp Glu Ser Arg Lys Ile Ser Arg Ile Phe Lys
                   165                 170                 175
   ttt cgt ggg gaa gga aaa gcg tgg gat tgg gaa gta tca agt gaa aac     576
   Phe Arg Gly Glu Gly Lys Ala Trp Asp Trp Glu Val Ser Ser Glu Asn
               180                 185                 190
   ggc aac tat gac tat tta atg tat gct gat gtt gac tac gac cac cct     624
   Gly Asn Tyr Asp Tyr Leu Met Tyr Ala Asp Val Asp Tyr Asp His Pro
           195                 200                 205
   gat gtc gtg gca gag aca aaa aaa tgg ggt atc tgg tat gcg aat gaa     672
   Asp Val Val Ala Glu Thr Lys Lys Trp Gly Ile Trp Tyr Ala Asn Glu
       210                 215                 220
   ctg tca tta gac ggc ttc cgt att gat gcc gcc aaa cat att aaa ttt     720
   Leu Ser Leu Asp Gly Phe Arg Ile Asp Ala Ala Lys His Ile Lys Phe
   225                 230                 235                 240
   tca ttt ctg cgt gat tgg gtt cag gcg gtc aga cag gcg acg gga aaa     768
   Ser Phe Leu Arg Asp Trp Val Gln Ala Val Arg Gln Ala Thr Gly Lys
                   245                 250                 255
   gaa atg ttt acg gtt gcg gag tat tgg cag aat aat gcc ggg aaa ctc     816
   Glu Met Phe Thr Val Ala Glu Tyr Trp Gln Asn Asn Ala Gly Lys Leu
               260                 265                 270
   gaa aac tac ttg aat aaa aca agc ttt aat caa tcc gtg ttt gat gtt     864
   Glu Asn Tyr Leu Asn Lys Thr Ser Phe Asn Gln Ser Val Phe Asp Val
           275                 280                 285
   ccg ctt cat ttc aat tta cag gcg gct tcc tca caa gga ggc gga tat     912
   Pro Leu His Phe Asn Leu Gln Ala Ala Ser Ser Gln Gly Gly Gly Tyr
       290                 295                 300
   gat atg agg cgt ttg ctg gac ggt acc gtt gtg tcc agg cat ccg gaa     960
   Asp Met Arg Arg Leu Leu Asp Gly Thr Val Val Ser Arg His Pro Glu
   305                 310                 315                 320
   aag gcg gtt aca ttt gtt gaa aat cat gac aca cag ccg gga cag tca    1008
   Lys Ala Val Thr Phe Val Glu Asn His Asp Thr Gln Pro Gly Gln Ser
                   325                 330                 335
   ttg gaa tcg aca gtc caa act tgg ttt aaa ccg ctt gca tac gcc ttt    1056
   Leu Glu Ser Thr Val Gln Thr Trp Phe Lys Pro Leu Ala Tyr Ala Phe
               340                 345                 350
   att ttg aca aga gaa tcc ggt tat cct cag gtg ttc tat ggg gat atg    1104
   Ile Leu Thr Arg Glu Ser Gly Tyr Pro Gln Val Phe Tyr Gly Asp Met
           355                 360                 365
   tac ggg aca aaa ggg aca tcg cca aag gaa att ccc tca ctg aaa gat   1152
   Tyr Gly Thr Lys Gly Thr Ser Pro Lys Glu Ile Pro Ser Leu Lys Asp
       370                 375                 380
   aat ata gag ccg att tta aaa gcg cgt aag gag tac gca tac ggg ccc   1200
   Asn Ile Glu Pro Ile Leu Lys Ala Arg Lys Glu Tyr Ala Tyr Gly Pro
   385                 390                 395                 400
   cag cac gat tat att gac cac ccg gat gtg atc gga tgg acg agg gaa   1248
   Gln His Asp Tyr Ile Asp His Pro Asp Val Ile Gly Trp Thr Arg Glu
                   405                 410                 415
   ggt gac agc tcc gcc gcc aaa tca ggt ttg gcc gct tta atc acg gac   1296
   Gly Asp Ser Ser Ala Ala Lys Ser Gly Leu Ala Ala Leu Ile Thr Asp
               420                 425                 430
   gga ccc ggt ggg gca aag cga atg tat gtc ggc cgg caa aac gcc ggt   1344
   Gly Pro Gly Gly Ala Lys Arg Met Tyr Val Gly Arg Gln Asn Ala Gly
           435                 440                 445
   gag aca tgg cat gac att acc gga aac cgt tcg gag ccg gtt gtc atc   1392
   Glu Thr Trp His Asp Ile Thr Gly Asn Arg Ser Glu Pro Val Val Ile
       450                 455                 460
   aat tcg gaa ggc tgg gga gag ttt cac gta aac ggc ggg tcg gtt tca   1440
   Asn Ser Glu Gly Trp Gly Glu Phe His Val Asn Gly Gly Ser Val Ser
   465                 470                 475                 480
   att tat gtt caa aga tag                                           1458
   Ile Tyr Val Gln Arg
                   485
<210> 16
   <211> 485
   <212> PRT
   <213> Künstliche Sequenz
   <223> Beschreibung der künstlichen Sequenz:Fusion der Alpha-Amylase-Gene von B. licheniformis und B. amyloliquefaciens (LAL19-433).
<400> 16
   Ala Asn Leu Asn Gly Thr Leu Met Gln Tyr Phe Glu Trp Tyr Met Pro
    1               5                   10                  15
   Asn Asp Gly Gln His Trp Lys Arg Leu Gln Asn Asp Ala Glu His Leu
               20                  25                  30
   Ser Asp Ile Gly Ile Thr Ala Val Trp Ile Pro Pro Ala Tyr Lys Gly
           35                  40                  45
   Leu Ser Gln Ser Asp Asn Gly Tyr Gly Pro Tyr Asp Leu Tyr Asp Leu
       50                  55                  60
   Gly Glu Phe Gln Gln Lys Gly Thr Val Arg Thr Lys Tyr Gly Thr Lys
   65                  70                  75                  80
   Ser Glu Leu Gln Asp Ala Ile Gly Ser Leu His Ser Arg Asn Val Gln
                   85                  90                  95
   Val Tyr Gly Asp Val Val Leu Asn His Lys Ala Gly Ala Asp Ala Thr
               100                 105                 110
   Glu Asp Val Thr Ala Val Glu Val Asn Pro Ala Asn Arg Asn Gln Glu
           115                 120                 125
   Thr Ser Glu Glu Tyr Gln Ile Lys Ala Trp Thr Asp Phe Arg Phe Pro
           130                 135                 140
   Gly Arg Gly Asn Thr Tyr Ser Asp Phe Lys Trp His Trp Tyr His Phe
   145                 150                 155                 160
   Asp Gly Ala Asp Trp Asp Glu Ser Arg Lys Ile Ser Arg Ile Phe Lys
                   165                 170                 175
   Phe Arg Gly Glu Gly Lys Ala Trp Asp Trp Glu Val Ser Ser Glu Asn
               180                 185                 190
   Gly Asn Tyr Asp Tyr Leu Met Tyr Ala Asp Val Asp Tyr Asp His Pro
           195                 200                 205
   Asp Val Val Ala Glu Thr Lys Lys Trp Gly Ile Trp Tyr Ala Asn Glu
       210                 215                 220
   Leu Ser Leu Asp Gly Phe Arg Ile Asp Ala Ala Lys His Ile Lys Phe
   225                 230                 235                 240
   Ser Phe Leu Arg Asp Trp Val Gln Ala Val Arg Gln Ala Thr Gly Lys
                   245                 250                 255
   Glu Met Phe Thr Val Ala Glu Tyr Trp Gln Asn Asn Ala Gly Lys Leu
               260                 265                 270
   Glu Asn Tyr Leu Asn Lys Thr Ser Phe Asn Gln Ser Val Phe Asp Val
           275                 280                 285
   Pro Leu His Phe Asn Leu Gln Ala Ala Ser Ser Gln Gly Gly Gly Tyr
       290                 295                 300
   Asp Met Arg Arg Leu Leu Asp Gly Thr Val Val Ser Arg His Pro Glu
   305                 310                 315                 320
   Lys Ala Val Thr Phe Val Glu Asn His Asp Thr Gln Pro Gly Gln Ser
                   325                 330                 335
   Leu Glu Ser Thr Val Gln Thr Trp Phe Lys Pro Leu Ala Tyr Ala Phe
               340                 345                 350
   Ile Leu Thr Arg Glu Ser Gly Tyr Pro Gln Val Phe Tyr Gly Asp Met
           355                 360                 365
   Tyr Gly Thr Lys Gly Thr Ser Pro Lys Glu Ile Pro Ser Leu Lys Asp
       370                 375                 380
   Asn Ile Glu Pro Ile Leu Lys Ala Arg Lys Glu Tyr Ala Tyr Gly Pro
   385                 390                 395                 400
   Gln His Asp Tyr Ile Asp His Pro Asp Val Ile Gly Trp Thr Arg Glu
                   405                 410                 415
   Gly Asp Ser Ser Ala Ala Lys Ser Gly Leu Ala Ala Leu Ile Thr Asp
               420                 425                 430
   Gly Pro Gly Gly Ala Lys Arg Met Tyr Val Gly Arg Gln Asn Ala Gly
           435                 440                 445
   Glu Thr Trp His Asp Ile Thr Gly Asn Arg Ser Glu Pro Val Val Ile
       450                 455                 460
   Asn Ser Glu Gly Trp Gly Glu Phe His Val Asn Gly Gly Ser Val Ser
   465                 470                 475                 480
   Ile Tyr Val Gln Arg
                   485
<210> 17
   <211> 1452
   <212> DNA
   <213> Künstliche Sequenz
<220>
   <223> Beschreibung der künstlichen Sequenz:Fusion der Alpha-Amylase-Gene von B. licheniformis und B. amyloliquefaciens (LAL19-153).
<220>
   <221> CDS
   <222> (1)..(1452)
<400> 17
   gca aat ctt aat ggg acg ctg atg cag tat ttt gaa tgg tac atg ccc      48
   Ala Asn Leu Asn Gly Thr Leu Met Gln Tyr Phe Glu Trp Tyr Met Pro
    1               5                  10                  15
   aat gac ggc cag cat tgg aaa cga ttg cag aat gat gcg gaa cat tta      96
   Asn Asp Gly Gln His Trp Lys Arg Leu Gln Asn Asp Ala Glu His Leu
               20                  25                  30
   tcg gat atc gga atc act gcc gtc tgg att cct ccc gca tac aaa gga     144
   Ser Asp Ile Gly Ile Thr Ala Val Trp Ile Pro Pro Ala Tyr Lys Gly
           35                  40                  45
   ttg agc caa tcc gat aac gga tac gga cct tat gat ttg tat gat tta     192
   Leu Ser Gln Ser Asp Asn Gly Tyr Gly Pro Tyr Asp Leu Tyr Asp Leu
       50                  55                  60
   gga gaa ttc cag caa aaa ggg acg gtc aga acg aaa tac ggc aca aaa     240
   Gly Glu Phe Gln Gln Lys Gly Thr Val Arg Thr Lys Tyr Gly Thr Lys
   65                  70                  75                  80
   tca gag ctt caa gat gcg atc ggc tca ctg cat tcc cgg aac gtc caa     288
   Ser Glu Leu Gln Asp Ala Ile Gly Ser Leu His Ser Arg Asn Val Gln
                   85                  90                  95
   gta tac gga gat gtg gtt ttg aat cat aag gct ggt gct gat gca aca     336
   Val Tyr Gly Asp Val Val Leu Asn His Lys Ala Gly Ala Asp Ala Thr
               100                 105                 110
   gaa gat gta act gcc gtc gaa gtc aat ccg gcc aat aga aat cag gaa     384
   Glu Asp Val Thr Ala Val Glu Val Asn Pro Ala Asn Arg Asn Gln Glu
           115                 120                 125
   act tcg gag gaa tat caa atc aaa gcg tgg acg gat ttt cgt ttt ccg     432
   Thr Ser Glu Glu Tyr Gln Ile Lys Ala Trp Thr Asp Phe Arg Phe Pro
       130                 135                 140
   ggc cgt gga aac acg tac agt gat ttt aaa tgg cat tgg tac cat ttt     480
   Gly Arg Gly Asn Thr Tyr Ser Asp Phe Lys Trp His Trp Tyr His Phe
   145                 150                 155                 160
   gac gga acc gat tgg gac gag tcc cga aag ctg aac cgc atc tat aag     528
   Asp Gly Thr Asp Trp Asp Glu Ser Arg Lys Leu Asn Arg Ile Tyr Lys
                   165                 170                 175
   ttt caa gga aag gct tgg gat tgg gaa gtt tcc aat gaa aac ggc aac     576
   Phe Gln Gly Lys Ala Trp Asp Trp Glu Val Ser Asn Glu Asn Gly Asn
               180                 185                 190
   tat gat tat ttg atg tat gcc gac atc gat tat gac cat cct gat gtc     624
   Tyr Asp Tyr Leu Met Tyr Ala Asp Ile Asp Tyr Asp His Pro Asp Val
           195                 200                 205
   gca gca gaa att aag aga tgg ggc act tgg tat gcc aat gaa ctg caa     672
   Ala Ala Glu Ile Lys Arg Trp Gly Thr Trp Tyr Ala Asn Glu Leu Gln
       210                 215                 220
   ttg gac ggt ttc cgt ctt gat gct gtc aaa cac att aaa ttt tct ttt     720
   Leu Asp Gly Phe Arg Leu Asp Ala Val Lys His Ile Lys Phe Ser Phe
   225                 230                 235                 240
   ttg cgg gat tgg gtt aat cat gtc agg gaa aaa acg ggg aag gaa atg     768
   Leu Arg Asp Trp Val Asn His Val Arg Glu Lys Thr Gly Lys Glu Met
                   245                 250                 255
   ttt acg gta gct gaa tat tgg cag aat gac ttg ggc gcg ctg gaa aac     816
   Phe Thr Val Ala Glu Tyr Trp Gln Asn Asp Leu Gly Ala Leu Glu Asn
               260                 265                 270
   tat ttg aac aaa aca aat ttt aat cat tca gtg ttt gac gtg ccg ctt     864
   Tyr Leu Asn Lys Thr Asn Phe Asn His Ser Val Phe Asp Val Pro Leu
           275                 280                 285
   cat tat cag ttc cat gct gca tcg aca cag gga ggc ggc tat gat atg     912
   His Tyr Gln Phe His Ala Ala Ser Thr Gln Gly Gly Gly Tyr Asp Met
       290                 295                 300
   agg aaa ttg ctg aac agt acg gtc gtt tcc aag cat ccg ttg aaa gcg     960
   Arg Lys Leu Leu Asn Ser Thr Val Val Ser Lys His Pro Leu Lys Ala
   305                 310                 315                 320
   gtt aca ttt gtc gat aac cat gat aca cag ccg ggg caa tcg ctt gag    1008
   Val Thr Phe Val Asp Asn His Asp Thr Gln Pro Gly Gln Ser Leu Glu
                   325                 330                 335
   tcg act gtc caa aca tgg ttt aag ccg ctt gct tac gct ttt att ctc    1056
   Ser Thr Val Gln Thr Trp Phe Lys Pro Leu Ala Tyr Ala Phe Ile Leu
               340                 345                 350
   aca agg gaa tct gga tac cct cag gtt ttc tac ggg gat atg tac ggg    1104
   Thr Arg Glu Ser Gly Tyr Pro Gln Val Phe Tyr Gly Asp Met Tyr Gly
           355                 360                 365
   acg aaa gga gac tcc cag cgc gaa att cct gcc ttg aaa cac aaa att    1152
   Thr Lys Gly Asp Ser Gln Arg Glu Ile Pro Ala Leu Lys His Lys Ile
       370                 375                 380
   gaa ccg atc tta aaa gcg aga aaa cag tat gcg tac gga gca cag cat    1200
   Glu Pro Ile Leu Lys Ala Arg Lys Gln Tyr Ala Tyr Gly Ala Gln His
   385                 390                 395                 400
   gat tat ttc gac cac cat gac att gtc ggc tgg aca agg gaa ggc gac    1248
   Asp Tyr Phe Asp His His Asp Ile Val Gly Trp Thr Arg Glu Gly Asp
                   405                 410                 415
   agc tcg gtt gca aat tca ggt ttg gcg gca tta ata aca gac gga ccc    1296
   Ser Ser Val Ala Asn Ser Gly Leu Ala Ala Leu Ile Thr Asp Gly Pro
               420                 425                 430
   ggt ggg gca aag cga atg tat gtc ggc cgg caa aac gcc ggt gag aca    1344
   Gly Gly Ala Lys Arg Met Tyr Val Gly Arg Gln Asn Ala Gly Glu Thr
           435                 440                 445
   tgg cat gac att acc gga aac cgt tcg gag ccg gtt gtc atc aat tcg    1392
   Trp His Asp Ile Thr Gly Asn Arg Ser Glu Pro Val Val Ile Asn Ser
       450                 455                 460
   gaa ggc tgg gga gag ttt cac gta aac ggc ggg tcg gtt tca att tat    1440
   Glu Gly Trp Gly Glu Phe His Val Asn Gly Gly Ser Val Ser Ile Tyr
   465                 470                 475                 480
   gtt caa aga tag                                                    1452
   Val Gln Arg
<210> 18
   <211> 483
   <212> PRT
   <213> Künstliche Sequenz
   <223> Beschreibung der künstlichen Sequenz:Fusion der Alpha-Amylase-Gene von B. licheniformis und B. amyloliquefaciens (LAL19-153).
<400> 18
   Ala Asn Leu Asn Gly Thr Leu Met Gln Tyr Phe Glu Trp Tyr Met Pro
    1               5                  10                  15
   Asn Asp Gly Gln His Trp Lys Arg Leu Gln Asn Asp Ala Glu His Leu
               20                  25                  30
   Ser Asp Ile Gly Ile Thr Ala Val Trp Ile Pro Pro Ala Tyr Lys Gly
           35                  40                  45
   Leu Ser Gln Ser Asp Asn Gly Tyr Gly Pro Tyr Asp Leu Tyr Asp Leu
       50                  55                  60
   Gly Glu Phe Gln Gln Lys Gly Thr Val Arg Thr Lys Tyr Gly Thr Lys
   65                  70                  75                  80
   Ser Glu Leu Gln Asp Ala Ile Gly Ser Leu His Ser Arg Asn Val Gln
                   85                  90                  95
   Val Tyr Gly Asp Val Val Leu Asn His Lys Ala Gly Ala Asp Ala Thr
               100                 105                 110
   Glu Asp Val Thr Ala Val Glu Val Asn Pro Ala Asn Arg Asn Gln Glu
           115                 120                 125
   Thr Ser Glu Glu Tyr Gln Ile Lys Ala Trp Thr Asp Phe Arg Phe Pro
       130                 135                 140
   Gly Arg Gly Asn Thr Tyr Ser Asp Phe Lys Trp His Trp Tyr His Phe
   145                 150                 155                 160
   Asp Gly Thr Asp Trp Asp Glu Ser Arg Lys Leu Asn Arg Ile Tyr Lys
                   165                 170                 175
   Phe Gln Gly Lys Ala Trp Asp Trp Glu Val Ser Asn Glu Asn Gly Asn
               180                 185                 190
   Tyr Asp Tyr Leu Met Tyr Ala Asp Ile Asp Tyr Asp His Pro Asp Val
           195                 200                 205
   Ala Ala Glu Ile Lys Arg Trp Gly Thr Trp Tyr Ala Asn Glu Leu Gln
       210                 215                 220
   Leu Asp Gly Phe Arg Leu Asp Ala Val Lys His Ile Lys Phe Ser Phe
   225                 230                 235                 240
   Leu Arg Asp Trp Val Asn His Val Arg Glu Lys Thr Gly Lys Glu Met
                   245                 250                 255
   Phe Thr Val Ala Glu Tyr Trp Gln Asn Asp Leu Gly Ala Leu Glu Asn
               260                 265                 270
   Tyr Leu Asn Lys Thr Asn Phe Asn His Ser Val Phe Asp Val Pro Leu
           275                 280                 285
   His Tyr Gln Phe His Ala Ala Ser Thr Gln Gly Gly Gly Tyr Asp Met
       290                 295                 300
   Arg Lys Leu Leu Asn Ser Thr Val Val Ser Lys His Pro Leu Lys Ala
   305                 310                 315                 320
   Val Thr Phe Val Asp Asn His Asp Thr Gln Pro Gly Gln Ser Leu Glu
                   325                 330                 335
   Ser Thr Val Gln Thr Trp Phe Lys Pro Leu Ala Tyr Ala Phe Ile Leu
               340                 345                 350
   Thr Arg Glu Ser Gly Tyr Pro Gln Val Phe Tyr Gly Asp Met Tyr Gly
           355                 360                 365
   Thr Lys Gly Asp Ser Gln Arg Glu Ile Pro Ala Leu Lys His Lys Ile
       370                 375                 380
   Glu Pro Ile Leu Lys Ala Arg Lys Gln Tyr Ala Tyr Gly Ala Gln His
   385                 390                 395                 400
   Asp Tyr Phe Asp His His Asp Ile Val Gly Trp Thr Arg Glu Gly Asp
                   405                 410                 415
   Ser Ser Val Ala Asn Ser Gly Leu Ala Ala Leu Ile Thr Asp Gly Pro
               420                 425                 430
   Gly Gly Ala Lys Arg Met Tyr Val Gly Arg Gln Asn Ala Gly Glu Thr
           435                 440                 445
   Trp His Asp Ile Thr Gly Asn Arg Ser Glu Pro Val Val Ile Asn Ser
       450                 455                 460
   Glu Gly Trp Gly Glu Phe His Val Asn Gly Gly Ser Val Ser Ile Tyr
   465                 470                 475                 480
   Val Gln Arg

## Beschreibung

Die vorliegende Erfindung betrifft Wasch- und Reinigungsmittel mit hybriden α-Amylasen, die von den α-Amylasen der Bakterienspezies *Bacillus amyloliquefaciens* und *Bacillus licheniformis* abgeleitet sind, Verfahren zur Reinigung von Textilien oder harten Oberflächen unter Beteiligung solcher Proteine oder entsprechender Mittel und die Verwendung solcher Proteine oder entsprechender Mittel zur Reinigung von Textilien oder harten Oberflächen. Sie betrifft ferner ein Verfahren, um Wasch- und Reinigungsmittel in ihrer Leistung zu verbessern, indem Hybrid-Amylasen der α-Amylasen der Bakterienspezies *B. amyloliquefaciens* und *B. licheniformis* gebildet und den betreffenden Mitteln zugegeben werden.

α-Amylasen (E.C. 3.2.1.1) hydrolysieren interne α-1,4-glycosidische Bindungen von Stärke und stärkeähnlichen Polymeren, wie beispielsweise Amylose, Amylopektin oder Glykogen, unter Bildung von Dextrinen und β-1,6-verzweigten Oligosacchariden. Sie gehören zu den wichtigsten industriell genutzten Enzymen überhaupt. Dies aus zwei Gründen: Denn zum einen werden sie von Mikroorganismen in das umgebende Medium abgegeben, so daß sie durch Fermentation und Aufreinigung aus dem Kulturmedium mit vergleichsweise geringem Aufwand in industriellem Maßstab gewonnen werden können. Zum anderen werden Amylasen für ein breites Anwendungsspektrum benötigt.

Eine wichtige technische Verwendung von α-Amylase ist ihr Einsatz als aktive Komponente in Wasch- und Reinigungsmitteln. Ihr Beitrag zur Wasch-, beziehungsweise Reinigungsleistung des betreffenden Mittels ist der Abbau von stärkehaltigen Anschmutzungen. Die Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es günstigenfalls zu Synergieeffekten zwischen dem Enzym und den übrigen Bestandteilen dieser Mittel kommt.

Es findet eine intensive Suche nach α-Amylase aus natürlichen Quellen statt, die sich für den Einsatz in Wasch- und Reinigungsmitteln eignen könnten. Identifiziert wurden stärkespaltende Enzyme beispielsweise aus *Pimelobacter, Pseudomonas* und *Thermus* für die Lebensmittelherstellung, Kosmetik und Pharmaka (EP 636693), ebensolche aus *Rhizobium, Arthrobacter, Brevibacterium* und *Micrococcus* (EP 628630), aus *Pyrococcus* (WO 94/19454) und *Sulfolobus* zur Stärkeverflüssigung bei hohen Temperaturen, beziehungsweise stark sauren Reaktionsbedingungen (EP 727485 und WO 96/02633). Für den Einsatz bei alkalischen pH-Werten sind Amylasen aus *Bacillus sp.* (WO 95/26397 und WO 97/00324) gefunden worden. Wegen ihrer geringen Empfindlichkeit gegenüber Detergenzien eignen sich andere Amylasen aus verschiedenen *Bacilli* (EP 670367) zur Verwendung in Wasch- oder Reinigungsmitteln. Und die Amylase aus *Thermoalcalibacter* (WO 98/13481) ist weitgehend unempfindlich gegenüber Calcium-lonen, so daß sie von vornherein gute Voraussetzungen für die Verwendung in Waschmitteln aufweist.

Eine in Wasch- und Reinigungsmitteln häufig eingesetzte α-Amylase ist die aus *Bacillus licheniformis.* Das entsprechende Produkt der Fa. Novozymes A/S, Bagsværd, Dänemark, beispielsweise, trägt den Handelsnamen Termamyl^{®}; von der Fa. Genencor Int., Rochester, New York, USA, heißt es Purastar^{®}. Die von *B. amyloliquefaciens* gebildete, und beispielsweise gemäß US 1227374 durch *B*. *subtilis* fermentativ herstellbare α-Amylase wird von Novozymes A/S unter dem Namen BAN^{®} vertrieben. Die Sequenzen der α-Amylasen aus *B. licheniformis, B. amyloliquefaciens* sowie die der α-Amylase aus *B. stearothermophilus* werden beispielsweise in der Anmeldung WO 96/23874 angegeben.

Diese Amylase-Moleküle, beziehungsweise deren nahen Verwandte, sind in zahlreichen Erfindungen, insbesondere über molekularbiologische Modifikationen weiterentwickelt worden, um sie in Hinblick auf spezifische Anwendungen, insbesondere ihrer Verwendung in Wasch- und Reinigungsmitteln hin zu optimieren. Solche Optimierungen können beispielsweise die Substratspezifitäten, die Stabilität des Enzyms unter verschiedenen Bedingungen oder die enzymatische Aktivität selbst betreffen.

Ein Prinzip dieser Weiterentwicklungen besteht darin, die Eigenschaften durch Punktmutationen zu verbessern. Beispielsweise aus der Anmeldung WO 96/23873 gehen für den Einsatz in Wasch- und Reinigungsmitteln verbesserte Varianten von solchen α-Amylasen hervor, die als Wildtypenzyme bekannt waren oder aus Mikroorganismen stammen, die zuvor als Produzenten von alkalischen Proteasen beschrieben worden waren. Beispielsweise das Mutagenese-Verfahren der Anmeldung WO 99/20768 führt zu α-Amylase-Varianten, die in Gegenwart von Reinigungsmittelbestandteilen besonders stabil sind. Praktisch immer wirkt sich bei derartigen Modifikationen eine Änderung einzelner enzymatischer Eigenschaften auch auf andere Eigenschaften und auf die Leistung des betreffenden Enzyms aus. Ein Beispiel für ein auf solche Weise erhaltenes, inzwischen kommerziell vermarktetes Optimierungsprodukt ist Duramyl^{®} (WO 94/02597) mit verringerter Oxidationsempfindlichkeit (Fa. Novozymes; SÖFW-Journal 123, (1997), S. 723-731).

Weitere Beispiele sind: Die Amylasen der Anmeldung WO 99/02702 sind bei höheren Temperaturen stabiler als das Ausgangsmolekül. Die Enzyme der Anmeldung WO 99/23211 sind bei hohen pH-Werten, in Gegenwart von Calcium-lonen und bei höheren Temperaturen stabil. Die α-Amylasen der Anmeldung WO 97/43424 zeigen ein geändertes Calciumionen-Bindungsverhalten und damit geänderte enzymatische Eigenschaften.

Eine andere oder zusätzlich einzusetzede Optimierungsmethode besteht beispielsweise in chemischen Modifikationen (DE 4013142).

Beispielsweise in der Patentanmeldung WO 99/43793 wird ein weiteres Prinzip zur Weiterentwicklung bekannter α-Amylasen angewandt. Darin werden Sequenzähnlichkeiten zwischen Novamyl^{®} und bekannten Cyclodextringlucanotransferasen (CGTasen) ausgenutzt, um mithilfe molekularbiologischer Techniken größere Molekülteile neu zu kombinieren und somit eine Schar verwandter Moleküle zu konstruieren. Bei diesen handelt es sich um α-Amylasen mit zusätzlichen CGTasespezifischen Consensus-Sequenzen (Boxen) und Funktionen oder, umgekehrt, um CGTasen mit zusätzlichen für α-Amylasen typischen Bereichen und Funktionen oder um Chimären beider Moleküle.

In der Anmeldung WO 99/57250 wird eine vergleichbare Methode offenbart, wie Waschmittelenzyme in ihrer Waschleistung gesteigert werden können. Das dort beschriebene Prinzip besteht darin, daß die betreffenden Enzyme über einen nicht-Aminosäure-Linker an Cellulose-Bindungsdomänen (CBD) bakteriellen Ursprungs kovalent gebunden werden. Diese sorgen dafür, daß das Enzym verstärkt auf der Oberfläche des Textils wirksam wird. Mit der Schrift WO 99/57252 werden in dieses Konzept andere mögliche Linker miteinbezogen, und mit der Schrift WO 99/57254 andere Enzyme, wie beispielsweise Glykosyl-Transferasen oder Acyl-Transferasen, die entweder unter Bildung eines chimären Proteins oder über die in WO 99/57252 erwähnten Linker an die CBD gebunden werden.

Die Notwendigkeit all dieser Weiterentwicklungen der für den Einsatz in Wasch- und Reinigungsmitteln etablierten α-Amylasen ergibt sich beispielsweise aus neuentwickelten Inhaltsstoffen, die, wie beispielsweise die Bleichmittel, die jeweiligen Bedingungen drastisch beeinflussen und die Enzyme in ihrer Leistungsfähigkeit beeinträchtigen. Sie ergibt sich aber auch aus den verschiedenen Reingungszwecken, aus den sich ändernden Gewohnheiten und Ansprüchen der Verbraucher, nach denen, beispielsweise, ein zunehmender Bedarf nach Waschmitteln für die Reinigung bei niedrigen und mittleren Temperaturen besteht.

Die Technik zur Herstellung von Fusions- oder Hybridproteinen ist im Stand der Technik gut etabliert. So wird beispielsweise in der Anmeldung EP 208491 ein *In vivo*-Verfahren zur Herstellung von Hybridproteinen beschrieben, das darauf beruht, daß die zugehörigen DNA-Abschnitte hintereinander in einen Vektor kloniert werden. Darunter sind beispielsweise auch Hybridamylasen, deren N-terminaler Bereich dem der α-Amylase aus *B. stearothermophilus* und deren C-terminaler Bereich dem der α-Amylase aus *B. licheniformis* entspricht.

In der Anmeldung WO 96/23874 werden Hybride aus den α-Amylasen aus *Bacillus licheniformis, B. amyloliquefaciens, B. stearothermophilus* und *Aspergillus oryzae* offenbart. Darunter ist konkret eine, deren Aminosäuren 1-300 aus der α-Amylase von *B. amyloliquefaciens* und 301-483 aus der α-Amylase von *B. licheniformis* stammen; nach der Terminologie der vorliegenden Anmeldung (siehe unten) könnte sie also als AL300 bezeichnet werden. Auch eine Variante AL37 wird darin benannt. Solche Hybrid-Amylasen können nach der Lehre dieser Anmeldung zur Ermittlung der dreidimensionalen Struktur der Termamyl-ähnlichen Amylasen hergestellt werden, um anhand derer solche Positionen zu erkennen, die für die enzymatische Aktivität wichtig sind. Diese können dann durch ortsgerichtete Mutagenese gezielt verändert und entsprechenden Anwendungen zugeführt werden. Dementsprechend werden sowohl einzelne Positionen als auch konkrete Austausche für diese Positionen benannt, mit denen die enzymatischen Eigenschaften variiert werden können. Für die nicht punktmutierten Hybridamylasen selbst werden in dieser Schrift keine weiteren technischen Einsatzmöglichkeiten offenbart.

Auf diesen Erkenntnissen aufbauend werden weitere, durch Punktmutagenese zu erhaltende Varianten sowohl von den Wildtypenzymen als auch von einzelnen Hybridamylasen, darunter AL37, auch zum Einsatz in Wasch- und Reinigungsmitteln in WO 97/41213 offenbart.

In der Anmeldung WO 00/60059 werden Varianten offenbart, die hinsichtlich eines veränderten Spaltungsmusters am Substrat Stärke entwickelt worden sind und sich deshalb besonders für die Stärke-Verarbeitung eignen; hierfür sei die Erzeugung von langen verzweigten Oligosacchariden vorteilhafter als die von kürzeren verzweigten Oligosacchariden. In dieser Schrift werden zahlreiche Punktmutationen sowohl von nativen α-Amylasen, als auch von Hybridamylasen, wie beispielsweise AL33 und AL37 (in ihrer Sequenz identisch) offenbart, die unter anderem auch eine Mutation in der Position 412 (nach der Zählung von *B. licheniformis),* vorzugsweise T412A, enthalten können; daneben muß aber mindestens noch eine zweite Mutation in einer der Positionen 13, 48 bis 54, 57, 107, 108, 111, 168 und 197 vorliegen; bevorzugt sind Mehrfachmutanten mit noch weiteren Austauschen.

In keiner der zuletzt genannten drei Schriften werden als Fusionspunkte in der Numerierung nach B. *amyloliquefaciens* die Positionen 17, 34 (entsprechend 36 in der Numerierung nach *B. licheniformis),* 76, 108, 112, 142, 147, 149, 151, 163, 174, 179, 185, 191, 198, 207, 231, 234, 244, 256, 263, 276, 431, 84, 99, 429, 201, 19, 433 oder 153 offenbart. Es werden ebenfalls keine Hybridamylasen mit Sequenzvariationen in den Positionen 134 oder 320 (in der Zählung nach *B. licheniformis)* offenbart, und die Offenbarung einer Mutation in Position 412 erfolgte in Kombination mit weiteren definierten Variation und im Zusammenhang mit einer speziellen Änderung der enzymatischen Aktivität.

Es stellte sich somit die Aufgabe, Wasch- und Reinigungsmittel mit neuen oder zumeindest für dieses Einsatzgebiet zuvor noch nicht bekannten amylolytischen Enzymen zur Verfügung zu stellen, vorzugsweise mit solchen, die bessere Wasch- oder Reinigungsleistungen zeigen als die etablierten α-Amylasen.

Eine Teilaufgabe bestand darin, Verfahren zur Reinigung von Textilien oder von harten Oberflächen zur Verfügung zu stellen, die durch derartige Amylasen hinsichtlich der Wasch-, beziehungsweise Reinigungsleistung verbesserte Ergebnisse aufweisen.

Eine zweite Teilaufgabe bestand darin, entsprechend leistungsverbesserte Verwendungsmöglichkeiten aufzuzeigen.

Eine dritte Teilaufgabe bestand darin, Verfahren aufzuzeigen, nach denen die Leistung eines Wasch- und Reinigungsmittels durch Entwicklung neuer Amylasen verbessert werden kann.

Diese Aufgabe wird durch die Bereitstellung von Hybrid-Proteinen gelöst, die über Fusionierung entsprechend zusammenpassender Teile aus den α-Amylasen aus den Bakterienspezies *Bacillus amyloliquefaciens* und *Bacillus licheniformis* gebildet werden können.

Das Überraschende dieser Lösung besteht darin, daß bereits allein durch Neukombination der Sequenzen von natürlichen und lang etablierten α-Amylasen leistungsfähigere Wasch-, beziehungsweise Reinigungsmittelamylasen erhalten werden können. Diese Hybridamylasen können als Ausgangspunkt für gezielte Weiterentwicklungen hinsichtlich dieses Einsatzgebiets dienen.

Einen Erfindungsgegenstand stellen somit Wasch- oder Reinigungsmittel dar, die dadurch gekennzeichnet sind, daß sie ein amylolytisches Hybrid-Protein enthalten, dessen Aminosäure-Sequenz in jeweils homologer Lage mindestens eine mehr als eine Aminosäure umfassende Teilsequenz enthält, die mit der der α-Amylase aus *Bacillus amyloliquefaciens* identisch ist, und in jeweils homologer Lage mindestens eine mehr als eine Aminosäure umfassende Teilsequenz enthält, die mit der der α-Amylase aus *Bacillus licheniformis* identisch ist. Hierunter sind solche Mittel bevorzugt, bei denen die auf die Ausgangsmoleküle zurückzuführenden Teilsequenzen der Hybridamylasen länger als 7, vorzugsweise länger als 14, besonders bevorzugt 21 bis 462 Aminosäuren lang sind und/oder, bei denen das Hybridprotein aus 3 oder aus 2 entsprechend den Ausgangssequenzen einander ergänzenden Teilsequenzen zusammengesetzt ist.

Bevorzugte Lösungen dieser Aufgabe stellen Mittel dar, deren Hybrid-Amylasen Fusionspunkte nahe den Positionen 17, 34, 76, 108, 112, 142, 147, 149, 151, 163, 174, 179, 185, 191, 198, 207, 231, 234, 244, 256, 263, 276, 431, 84, 99, 429, 201, 19, 433 und 153 gemäß der Numerierung der α-Amylase aus *B*. *amyloliquefaciens* (SEQ ID NO. 4) aufweisen; und zwar zunehmend bevorzugt innerhalb von Bereichen von 10, beziehungsweise 5 Aminosäuren vor und nach diesen Positionen, beziehungsweise genau an diesen Positionen. Hierunter sind besonders die Hybridamylasen AL17, AL108, AL142, AL147, AL149, AL151, AL163, AL174, AL179, AL185, AL191, AL198, AL207, AL231, AL234, AL244, AL263, AL276, AL431, ALA17-151, ALA76-151, ALA99-429, ALA12-151, ALA112-201, LA19 und LA431 bevorzugt.

Besonders bevorzugte Lösungen dieser Aufgabe sind Mittel, deren Hybrid-Amylasen Fusionspunkte nahe den Positionen 34, 256, 84, 19 und 153 gemäß der Numerierung der α-Amylase aus *B*. *amyloliquefaciens* (SEQ ID NO. 4) aufweisen; und zwar zunehmend bevorzugt innerhalb von Bereichen von 10, beziehungsweise 5 Aminosäuren vor und nach diesen Positionen, beziehungsweise genau an diesen Positionen. Hierunter sind besonders die Hybridamylasen AL34 (SEQ ID NO. 6), AL256 (SEQ ID NO. 12), ALA34-84 (SEQ ID NO. 14) und LAL19-153 (SEQ ID NO. 18) bevorzugt.

Ganz besonders bevorzugte Lösungen dieser Aufgabe sind Mittel, deren Hybrid-Amylasen als Teilsequenz die Teilsequenz der Aminosäure-Positionen 19 bis 76 der α-Amylase aus *Bacillus amyloliquefaciens* (SEQ ID NO. 4) und als weitere Teilsequenz die Teilsequenz der Aminosäure-Positionen 433 bis 483 der α-Amylase aus *Bacillus licheniformis* (SEQ ID NO. 2) aufweisen. Hierunter sind besonders die Hybrid-Amylasen AL76 (SEQ ID NO. 8), AL112 (SEQ ID NO. 10) und LAL19-433 (SEQ ID NO. 16) bevorzugt, sowie die Moleküle, die jeweils mindestens zu 98%, vorzugsweise zu 99%, besonders bevorzugt zu 100% mit diesen identisch sind. Hierzu zählen durch Punktmutagenese, beispielsweise durch Substituion einzelner Aminosäuren erhältliche Varianten.

Weitere Lösungen dieser Aufgabe sind Mittel, deren Hybrid-Amylasen durch Deletionen kurzer, nicht mehr als 5 Aminosäuren umfassender Bereiche, Insertionsmutagenese oder Derivatisierung erhalten werden können oder eine durch die Hybridbildung entstandene antigene Determinate mit den zuvorgenannten Proteinen gemeinsam haben.

Vorzugsweise enthalten die Wasch- oder Reinigungsmittel dieses Erfindungsgegenstands die Hybrid-Amylase in Anteilen von 0,000001 Gewichts-Prozent bis 5 Gew.-%, insbesondere 0,00001 bis 3 Gew.-% und/oder weitere Enzyme; sie können in an sich bekannten Darreichungsformen oder Aggregaten oder mehreren Phasen vorliegen, oder darin kann die amylolytische Aktivität eine Funktion für die Freisetzung der Inhaltsstoffe des Mittels erfüllen oder selbst kontrolliert sein.

Eine Lösung der Teilaufgabe und somit den zweiten Erfindungsgegenstand bilden Verfahren zur Reinigung von Textilien oder von harten Oberflächen, die dadurch gekennzeichnet sind, daß in wenigstens einem der Verfahrensschritte eine Hybrid-Amylase, beziehungsweise ein Mittel des ersten Erfindungsgegenstands eingesetzt wird. Vorzugsweise kommt die Hybrid-Amylase in dem betreffenden Verfahrensschritt in einer Menge von 0,01 mg bis 400 mg, vorzugsweise von 0,02 mg bis 200 mg, besonders bevorzugt von 0,02 bis 100 mg pro Anwendung zum Einsatz.

Eine Lösung der zweiten Teilaufgabe und somit den dritten Erfindungsgegenstand bilden entsprechende Verwendungsmöglichkeiten der erfindungsrelevanten Hybrid-Amylasen zur Reinigung von Textilien oder von harten Oberflächen oder zur Freisetzung der Inhaltsstoffe entsprechender Mittel; vorzugsweise pro Anwendung in einer Geschirrspülmaschine oder einer Waschmaschine, in einer Menge von 0,01 mg bis 400 mg, vorzugsweise von 0,02 mg bis 200 mg, besonders bevorzugt von 0,02 bis 100 mg pro Anwendung zum Einsatz.

Lösungen der dritten Teilaufgabe und einen eigenen Erfindungsgegenstand stellen alle Verfahren zur Leistungsverbesserung von Wasch- oder Reinigungsmitteln dar, die auf der Entwicklung von Hybrid-Amylasen beruhen, die ihrerseits durch Fusion von jeweils mindestens mehr als eine Aminosäure umfassende Teilsequenzen der α-Amylasen aus *Bacillus amyloliquefaciens* und *Bacillus licheniformis* in jeweils homologer Lage zu einer amylolytisch wirksamen Hybrid-Amylase fusioniert und dem Mittel zugegeben werden.

Entsprechend bevorzugt sind jene Lösungen, die auf den zuvor diskutierten Hybrid-Amylasen beruhen, und ganz besonders auf den mit der vorliegenden Anmeldung bereitgestellten Sequenzinformationen über die natürlichen Gene aus *B. amyloliquefaciens* und *B. licheniformis* (SEQ ID NO. 3 und SEQ ID NO. 1).

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.

Unter einem Enzym ist im Sinne der vorliegenden Anmeldung ein Protein zu verstehen, das eine bestimmte biochemische Funktion ausübt. Unter amylolytischen Proteinen oder Enzymen mit amylolytischer Funktion sind solche zu verstehen, die α-1,4-glykosidische Bindungen von Polysacchariden hydrolysieren, insbesondere solche, die im Inneren der Polysaccharide liegen. Sie werden deshalb auch als α-1,4-Amylasen (E.C. 3.2.1.1) oder kurz: **α-Amylasen** bezeichnet.

Zahlreiche Proteine werden als sogenannte Präproteine (Precursor-Proteine), also zusammen mit einem Signal- oder Leaderpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Für technische Anwendungen, beispielsweise auch im Rahmen der vorliegenden Erfindung, sind demgegenüber aber aufgrund ihrer enzymatischen Aktivität die maturen Peptide, das heißt die nach ihrer Herstellung prozessierten Enzyme gegenüber den Präproteinen bevorzugt.

Unter Nukleinsäuren sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet.

Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions- Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen. Mutationen werden in der vorliegenden Anmeldung über die üblichen Einbuchstabencodes angegeben. So handelt es sich bei der Punktmutation T410A, beispielsweise, um einen Austausch in der Position 410 des betreffenden Proteins, bei dem die Aminosäure Threonin gegen die Aminosäure Alanin substituiert worden ist.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder amylolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Den Fragmenten entsprechen auf Nukleinsäure-Ebene die Teilsequenzen.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion kann beispielsweise darin bestehen, mithilfe des heranfusionierten Proteinteils eine bestimmte enzymatische Funktion herbeizuführen oder zu modifizieren oder zu Enzymen zu gelangen, die in irgendeiner Hinsicht verbesserte Eigenschaften aufweisen.

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Proteine gebunden werden. Ebenso ist unter Derivatisierung die kovalente Bindung an einen makromolekularen Träger zu verstehen.

Proteine können auch über die Reaktion mit einem Antiserum oder einem bestimmten Antikörper zu Gruppen immunologisch verwandter Proteine zusammengefaßt werden. Die Angehörigen einer Gruppe zeichnen sich dadurch aus, daß sie dieselbe, von einem Antikörper erkannte antigene Determinante aufweisen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Durch Vergleich mit bekannten Enzymen, die beispielsweise in allgemein zugänglichen Datenbanken hinterlegt sind, lassen sich aus der Aminosäure- oder Nukleotid-Sequenz charakteristische Molekülteile wie beispielsweise Strukturelemente oder die enzymatische Aktivität eines betrachteten Enzyms folgern.

Solch ein Vergleich geschieht dadurch, daß ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben. Eine Zusammenstellung aller in den verglichenen Sequenzen übereinstimmenden Positionen wird als Consensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von Prozent Ähnlichkeit die Rede. Solche Aussagen können über ganze Proteine oder Gene oder nur über einzelne Bereiche getroffen werden.

Homologe Bereiche von verschiedenen Proteinen sind zumeist solche mit gleichen Strukturelementen und/oder Funktionen, die sich durch Übereinstimmungen in der primären Aminosäuresequenz erkennen lassen. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Die Identifizierung homologer Bereiche zwischen mindestens zwei Proteinen ist die Grundlage dafür, diese zu Fusions- oder Hybrid-Proteinen mit vergleichbarer Funktion zusammenzusetzen. Dabei wird mindestens ein Bereich eines Proteins durch den homologen Bereich des anderen Proteins substituiert. Es müssen also die fusionierten Bereiche unter Erhalt der prinzipiellen Funktion des Gesamtproteins zusammenpassen. Handelt es sich beispielsweise um die Fusion von Teilsequenzen zweier Amylasen, so wird erfindungsgemäß dann eine Hybrid-Amylase erhalten, wenn das Fusionsprodukt selbst insgesamt eine amylolytische Aktivität aufweist.

Die enzymatische Aktivität kann durch andere Bereiche des Proteins, die nicht an der eigentlichen Reaktion beteiligt sind, qualitativ oder quantitativ modifiziert werden. Dies betrifft beispielsweise die Enzymstabilität, die Aktivität, die Reaktionsbedingungen oder die Substratspezifität.

Unter dem Begriff eines erfindungsgemäßen amylolytischen Proteins ist somit nicht allein eines mit der reinen, die Hydrolyse von α-1,4-glycosidischen Bindungen durchführenden Funktion zu verstehen, die auf die wenigen Aminosäurereste eines vermutlichen katalytisch aktiven Zentrums zurückzuführen sind. Er umfaßt darüberhinaus alle die Hydrolyse einer α-1,4-glycosidischen Bindung unterstützenden Funktionen.

Unter der Leistung eines Enzyms wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Faktoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien. So wird beispielsweise bei der Untersuchung, ob sich ein Enzym für den Einsatz in Wasch- oder Reinigungsmitteln eignet, dessen Beitrag zur Wasch- oder Reinigungsleistung eines mit weiteren Bestandteilen formulierten Mittels betrachtet.

Die erfindungswesentlichen Proteine werden erfindungsgemäß in Wasch- oder Reinigungsmitteln eingesetzt. Darin werden bevorzugt solche eingesetzt, deren enzymatische Aktivität dazu beiträgt, die Leistung mindestens einer Wasch- oder Reinigungsmittelrezeptur an mindestens einer stärkehaltigen oder stärkeähnlichen Anschmutzung auf wenigstens einer Oberfläche zu verbessern. Besonders bevorzugt sind solche, die bei mehr als einem Reinigungsproblem einen Beitrag zur Wasch-, beziehungsweise Reinigungsleistung des/der betreffenden Mittel leisten.

Bei diesen Oberflächen handelt es sich vorzugsweise um Textilien oder um harte Oberflächen. Die hierfür zu wählenden Bedingungen, wie beispielsweise Temperatur, pH-Wert, Ionenstärke, Redox-Verhältnisse oder mechanische Einflüsse sollten für das jeweilige Reinigungsproblem optimiert werden, also in Bezug auf die Anschmutzung und auf das Trägermaterial. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Vorzugsweise werden auch die Inhaltsstoffe der betreffenden Mittel aufeinander abgestimmt.

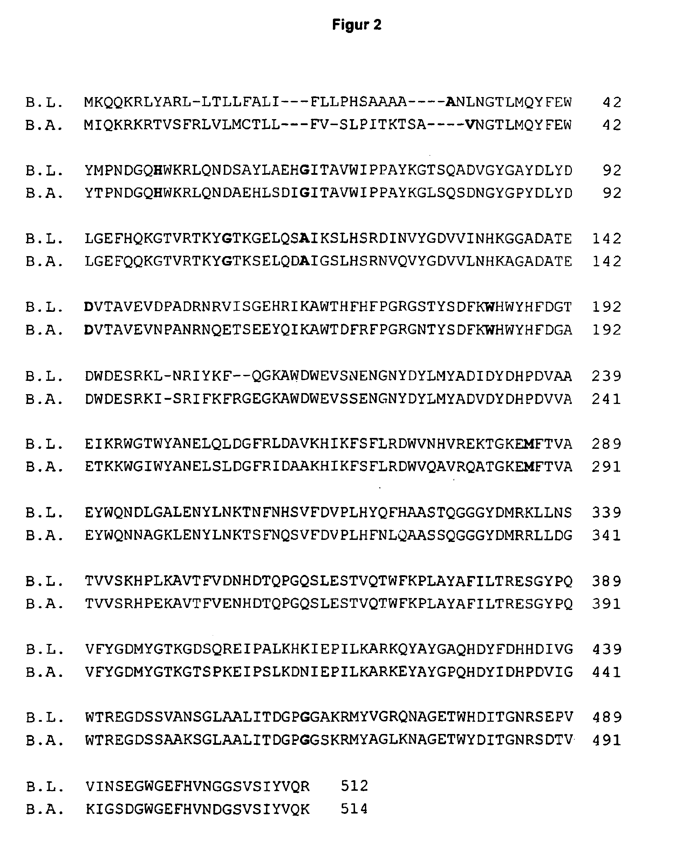

Die Hybrid-Amylasen, die die erfindungsgemäßen Mittel kennzeichnen, setzen sich in homologer Ergänzung aus Teilsequenzen der α-Amylasen von *Bacillus amyloliquefaciens* und von *Bacillus licheniformis* zusammen. Das heißt, jede Aminosäure der betreffenden Hybrid-Amylasen liegt in einem mindestens zwei Aminosäuren umfassenden Teilbereich, der in homologer Position entweder in der einen oder anderen Ausgangssequenz gefunden werden kann. Dies ist beispielsweise anhand des Alignments der Figur 2 nachvollziehbar.

Die Sequenzen dieser beiden Ausgangsenzyme können unter den Bezeichnungen amyA (für die α-Amylase aus *B. amyloliquefaciens*), beziehungsweise amyL (für die α-Amylase aus *B. licheniformis)* aus öffentlich zugänglichen Datenbanken erhalten werden. Beispielsweise in der Datenbank Swiss-Prot (Geneva Bioinformatics (GeneBio) S.A., Genf, Schweiz; http://www.genebio.com/sprot.html) sind sie unter den Eintragungsnummern P00692 (für die α-Amylase amyA aus *B. amyloliquefaciens)* und P06278 (für die α-Amylase amyL aus *B. licheniformis)* aufgeführt. Sie sind zusätzlich im Sequenzprotokoll der vorliegenden Anmeldung unter SEQ ID NO. 4, beziehungsweise SEQ ID NO. 2 angegeben; zusammen mit den zugehörigen Nukleotidsequenzen unter SEQ ID NO. 3, beziehungsweise SEQ ID NO. 1.

Beide Bacillus-Spezies sind in der Literatur ausführlich beschrieben und sind auch über Stammsammlungen allgemein zugänglich. So ist *B. amyloliquefaciens* beispielsweise unter der Bezeichnung DSM 7 bei der Deutschen Sammlung von Mikroorganismen und Zellkulturen GmbH, Mascheroder Weg 1b, 38124 Braunschweig (http://www.dsmz.de) oder unter der Bezeichnung ATCC 23350 bei der American Type Culture Collection, 10801 University Boulevard, Manassas, VA 20110-2209, USA (http://www.atcc.org) erhältlich. *B. licheniformis* kann über dieselben Stellen beispielsweise unter den Bezeichnungen DSM 13, beziehungsweise ATCC 14580 bezogen werden.

Aus diesen Stämmen können die zugehörigen α-Amylase-Gene beispielsweise über PCR mit solchen Primern erhalten werden, die anhand des Sequenzprotokolls der vorliegenden Anmeldung (SEQ ID NO. 3, beziehungsweise SEQ ID NO. 1) synthetisiert worden sind. Die erhaltenen PCR-Produkte können nach an sich bekannten Methoden kloniert und auf jede zweckmäßige Weise (siehe unten) weiterprozessiert werden.

Erfindungsgemäß können entsprechende Hybridklassen definiert und anhand der Ausgangssequenzen benannt werden. Wählt man für die Amylase aus *B. licheniformis* den Buchstaben L und für die aus *B amyloliquefaciens* den Buchstaben A, so gehört ein Enzym, das N-terminal die Sequenz der α-Amylase aus *B*. *licheniformis* und C-terminal die Sequenz der α-Amylase aus *B amyloliquefaciens* aufweist, zur Hybridklasse LA. Bei umgekehrter Zusammensetzung gehörte es zur Hybridklasse AL, und bei Fusion von drei Teilen entsprechend zu den Hybridklassen LAL oder ALA, je nachdem, welche Teilsequenzen der Ausgangsenzyme kombiniert worden sind.

Zusätzliche Nummern kennzeichnen das Molekül eindeutig dahingehend, an welcher Stelle, d.h. C-terminal von welcher Aminosäure die Fusion erfolgt ist. Maßgeblich ist dabei im Zweifel die Zählung der α-Amylase aus *B. amyloliquefaciens* (SEQ ID NO. 4). So besitzt beispielsweise das Molekül AL76 die N-terminalen 76 Aminosäuren der α-Amylase aus *B. amyloliquefaciens* und daran anschließend bis zum C-Terminus die homologen Aminosäuren der α-Amylase aus *B licheniformis,* also ab dem Tyrosin, das in Position 79 gemäß der Numerierung der α-Amylase aus *B.licheniformis* liegt. Beispielsweise die Hybrid-Amylase LAL19-433 besteht aus den N-terminalen 21 Aminosäuren der α-Amylase von *B*. *licheniformis,* gefolgt von dem homologen Bereich der α-Amylase von *B*. *amyloliquefaciens,* also beginnend mit dem in homologer Ergänzung auf das Histidin folgenden Tryptophan bis zu dem Glycin, das nach beiden Zählungen der Position 433 entspricht, und schließlich den restlichen Aminosäuren der α-Amylase von *B*. *licheniformis,* also dem Bereich der Aminosäuren 434 bis 483. Die Fusionspunkte sind auch in Figur 2 hervorgehoben.

Sollten sich bezüglich der Numerierung Unklarheiten aus der Betrachtung der Aminosäuresequenzen ergeben, so entscheidet die zugehörige Nukleotidsequenz, wie sie im Sequenzprotokoll offenbart ist, weil die Bildung von Hybridproteinen günstigerweise auf der Ebene der zugehörigen DNA erfolgt. Die Nummer des Fusionspunkts ergibt sich damit aus der Nummer des Codons, hinter dessen erster, zweiter oder dritter Nukleobase der Wechsel zur anderen DNA erfolgt ist, jeweils wiederum bezogen auf die Codonnumerierung von *B*. *amyloliquefaciens* (SEQ ID NO. 3).

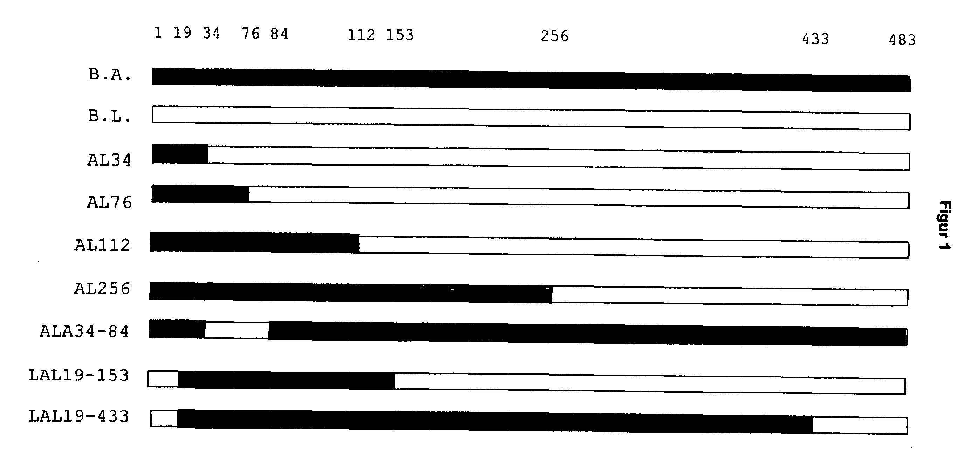

Die Hybrid-Amylasen, die bevorzugte Ausführungsformen der vorliegenden Anmeldung kennzeichnen, sind in Figur 1 schematisch dargestellt. Sie bestehen aus mindestens einem mindestens zwei Aminosäuren umfassenden Sequenzbereich der α-Amylase von *B. amyloliquefaciens* und mindestens einem ebenfalls mindestens zwei Aminosäuren umfassenden Sequenzbereich der α-Amylase von *B. licheniformis.*

Vorzugsweise sind die auf die Ausgangsmoleküle zurückzuführenden Teilsequenzen der Hybrid-Amylasen länger als 7, vorzugsweise länger als 14, besonders bevorzugt 21 bis 462 Aminosäuren lang. Denn wie beispielsweise aus der Figur 1 hervorgeht, sind die Teilsequenzen der besonders bevorzugten Vertreter (siehe unten) zwischen 21 und 462 Aminosäuren lang. Aus beliebigen Kombinationen der dort angegebenen Fusionsstellen ergeben sich erfindungsgemäße Proteine mit mindestens 8 (Fusionspunkte 76 und 84) dann 15 (Fusionspunkte 19 und 34), und schließlich zwischen 21 und 462 Aminosäuren Länge.

Vorzugsweise ist das Hybridprotein aus drei oder zwei entsprechend den Ausgangssequenzen einander ergänzenden Teilsequenzen zusammengesetzt. Das heißt, es weist eine oder zwei Fusionsstellen auf und kann einer der Hybridklassen AL, LA, LAL oder ALA zugeordnet werden.

Die Herstellung derartiger Hybridamylasen nach einem *In* vivo-Verfahren wird eingehend in der Publikation "Hybrid Bacillus amyloliquefaciens X Bacillus licheniformis α-Amylases. Construction, properties and sequence determinats" (1995) von B. Conrad, V. Hoang, A. Polley und J. Hofemeister, Eur. J. Biochem., 230, S. 481-490, beschrieben. Darin werden sie durch *In*-*vivo*-Rekombination erhalten. Diese ist beispielsweise dadurch möglich, daß beide Gene hintereinander in denselben Vektor kloniert und in eine Wirtszelle transformiert werden (vgl. H. Weber, C. Weissmann (1983): "Formation of genes coding for hybrid proteins by recombination between related, cloned genes in E. coli", Nucleic Acid Res., 11, S. 5661-5669, und EP 208491). Daraus kann sich eine Schar von Hybridamylasen ergeben, die unabhängig von bestimmten Restriktionsschnittstellen auf Rekombinationsereignisse zwischen diesen beiden Genen zurückzuführen sind. Die erhaltenen Proteine sind schematisch in Figur 2 der Arbeit von Conrad et al. angegeben.

Zur Durchführung von mehr als einer Fusion sind jedoch im Stand der Technik etablierte *In vitro-*Methoden erfolgversprechender. Die Bildung von Hybrid-Amylasen ist demnach sowohl durch Fusion an bereits vorhandenen als auch an nachträglich eingeführten Restriktionsschnittstellen möglich. So lassen sich zusätzliche Schnittstellen in beiden betreffenden Gene (SEQ ID NO. 1 und 3) einführen, indem nach an sich bekannten Punktmutagenese-Verfahren, beispielsweise über Mismatch-Primer, die gewünschten Nukleotide ausgetauscht werden. Hierbei kann die Degeneriertheit des genetischen Codes dazu genutzt werden, lediglich synonyme Codons zu erzeugen, d.h. solche Mutationen zu setzen, die die abgeleitete Aminosäuresequenz nicht beeinflussen. Man ist in der Wahl der erzeugten Restriktionsschnittstelle frei, da für die *In vitro*-Fusion jeweils auch auf ein geeignetes Restriktionsenzym abgestimmt werden kann; dies gilt insbesondere für die Erzeugung singulärer Schnittstellen. Alternativ können auch PCR-Reaktionen über Teilsequenzen, vorzugsweise mit Schnittstellen-enthaltenden Primern durchgeführt und die erhaltenen Produkte miteinander ligiert werden.

Weitere Möglichkeiten, derartige Fusionsproteine zu erhalten, sind beispielsweise: (a) die statistische Rekombination entsprechender Fragmente über Verfahren, die der Recursive-Sequence-Recombination vergleichbar sind, wie sie beispielsweise in den Patentanmeldungen WO 98/05764, WO 97/35966, EP 590689 oder EP 229046 dargestellt sind; und (b) statistische Rekombination entsprechender Fragmente über PCR-basierte Verfahen; solch eines stellt beispielsweise die StEP-Methode dar, wie sie in der Anmeldung WO 98/42832 beschrieben worden ist.

Die fusionierten DNA-Moleküle können anschließend nach an sich bekannten Methoden kloniert, amplifiziert und in Wirtszellen zur Expression gebracht werden. Hierfür geeignete Systeme, wie beispielsweise Vektoren und/oder Wirtzellen sind ebenfalls aus dem Stand der Technik bekannt und werden in großer Zahl kommerziell angeboten. Leicht kultivierbare Wirtszellen mit hohen Produktbildungsraten sind für die großtechnische Gewinnung der betreffenden Hybrid-Amylasen besonders geeignet.

Für beide Ausgangsenzyme ist bekannt, daß sie einen Beitrag zur Wasch-, beziehungsweise Reinigungsleistung entsprechender Mittel leisten können, insbesondere was Stärke- und stärkeähnliche Anschmutzungen angeht. So stellen beide Wildtyp-Moleküle die Ausgangspunkte für die kommerziell erhältlichen α-Amylasen BAN^{®}, beziehungsweise Termamyl^{®} (Hersteller: Fa. Novozymes A/S, Bagsværd, Dänemark) dar. Allerdings weisen sie unterschiedliche enzymatische Eigenschaften auf, insbesondere was die Thermostabilität angeht. So ist die Thermostabilität derα-Amylase aus *B. amyloliquefaciens* deutlich niedriger als die aus *B. licheniformis* (Tomazic, S.J., und Klibanov, A.M., (1988), J. Biol. Chem., 263, S. 3086-3091 und S. 3092-3096).

Hybridamylasen, die auf die beschriebene Weise aus den beiden Ausgangsenzymen erhalten werden können, dürften, wie anhand der enzymatischen Aktivitäten der Ausgangsenzyme und aufgrund der Arbeit von Conrad *et al.* erwartet werden kann, über amylolytische Aktivität verfügen. Jedoch ist dies nicht unbedingt gleichbedeutend mit einer Steigerung der Wasch-, beziehungsweise Reinigungsleistung entsprechender Mittel gegenüber stärkehaltigen und stärkeähnlichen Anschmutzungen. Beispielsweise können auch Enzyme erhalten werden, die wesentlich instabiler als die Ausgangsenzyme und damit als Wasch-, beziehungsweise Reinigungsmittelkomponente ungeeignet sind. Wenn sie jedoch in der Lage sind, die Wasch- und/oder Reinigungsleistung mindestens eines entsprechend mit ihnen angereicherten Wasch-, beziehungsweise Reinigungsmittels unter definierten Bedingungen zu verbessern, so werden sie bevorzugt eingesetzt.

Dies kann experimentell dadurch überprüft werden, daß eine Wasch- oder Reinigungsmittelrezeptur mit, beziehungsweise zum Vergleich ohne eine entsprechende Hybridamylase zubereitet und auf seine Waschleistung hin überprüft wird, und zwar insbesondere auf Stärke-enthaltende Verunreinigungen. Derartige Versuche werden in den Ausführungsbeispielen zur vorliegenden Patentanmeldung angegeben.

Die in Figur 1 der vorliegenden Anmeldung, beziehungsweise Figur 2 der erwähnten Publikation von Conrad et al. gezeigten Fusionsproteine sind durch Bruch- und Neufusion an den Stellen innerhalb der Ausgangsgene hervorgegangen, die auf Proteinebene folgenden Positionen entsprechen: 17, 34, 76, 108, 112, 142, 147, 149, 151, 163, 174, 179, 185, 191, 198, 207, 231, 234, 244, 256, 263, 276, 431, 84, 99, 429, 201, 19, 433 und 153 gemäß der Numerierung der Sequenz der α-Amylase aus *B. amyloliquefaciens* (SEQ ID NO. 2). Das bedeutet, daß jeweils C-terminal der Aminosäure, die diese Position einnimmt, eine Aminosäure aus der jeweils anderen Sequenz folgt. Bevorzugte Ausführungsformen der vorliegenden Erfindung enthalten solche Hybridproteine, deren Fusionsstellen innerhalb eines Bereichs von 10 Aminosäuren vor bis 10 Aminosäuren nach diesen Positionen liegen. Diesen Ausführungsformen gegenüber sind zunehmend solche bevorzugt, deren Fusionsstellen innerhalb eines Bereichs von 9, 8, 7, 6, 5, 4, 3, 2 oder 1 Aminosäuren vor bis 9, 8, 7, 6, 5, 4, 3, 2 oder 1 Aminosäuren nach diesen Positionen liegen. Und ganz besonders bevorzugt sind solche, deren Fusionspunkte exakt an diesen Positionen liegen, d.h. C-terminal davon.

Wie oben ausgeführt, bestimmen die Identität der in den Hybridproteinen enthaltenen verschiedenen Teilsequenzen und die Fusionsorte auch ihren Namen. Welche Aminosäuresequenz sich im individuellen Fall ergibt, kann anhand der im Sequenzprotokoll unter den Nummern SEQ ID NO. 2 und SEQ ID NO. 4 angegebenen Wildtyp-Aminosäuresequenzen der beiden Ausgangsenzyme sowie anhand von Figur 2 nachvollzogen werden.

Für folgende speziellen Hybridamylasen ist bereits in der Publikation von Conrad *et al.* eine gewisse amylolytische Aktivität nachgewiesen worden: AL17, AL108, AL142, AL147, AL149, AL151, AL163, AL174, AL179, AL185, AL191, AL198, AL207, AL231, AL234, AL244, AL263, AL276, AL431, ALA17-151, ALA76-151, ALA99-429, ALA12-151, ALA112-201, LA19 und LA431. Sie kennzeichnen somit bevorzugte Ausführungsformen der vorliegenden Erfindung.

Hybrid-Amylasen, die besonders bevorzugte Ausführungsformen dieses Erfindungsgegenstands kennzeichnen, weisen Fusionspunkte nahe den Positionen 34, 256, 84, 19 und/oder 153 gemäß der Numerierung der SEQ ID NO. 4 auf. Zunehmend bevorzugt liegen diese innerhalb eines Bereiches von 10, 9, 8, 7, 6, 5, 4, 3, 2 und 1 Aminosäuren vor bis zunehmend bevorzugt 10, 9, 8, 7, 6, 5, 4, 3, 2 und 1 Aminosäuren nach diesen Positionen.

Hierunter sind insbesondere Mittel mit den Hybrid-Amylasen AL34 (SEQ ID NO. 6), AL256 (SEQ ID NO. 12), ALA34-84 (SEQ ID NO. 14) und/oder LAL19-153 (SEQ ID NO. 18) bevorzugt. Denn sie vermögen die Wasch-, beziehungsweise Reinigungsleistungen von entsprechenden Rezepturen gegenüber stärkehaltigen oder stärkeähnlichen Anschmutzungen zu verbessern. Zum Teil zeigen sie, wie aus den Anwendungseispielen der vorliegenden Anmeldung entnommen werden kann, Wasch-, beziehungsweise Reinigungsleistungen die denen von im Stand der Technik etablierten Enzymen ebenbürtig sind.

Hybrid-Amylasen, die besonders bevorzugte Ausführungsformen dieses Erfindungsgegenstands kennzeichnen, weisen Fusionspunkte nahe den Positionen 19, 76, 112 und/oder 433 gemäß der Numerierung der SEQ ID NO. 4 auf. Zunehmend bevorzugt liegen diese innerhalb eines Bereiches von 10, 9, 8, 7, 6, 5, 4, 3, 2 und 1 Aminosäuren vor bis zunehmend bevorzugt 10, 9, 8, 7, 6, 5, 4, 3, 2 und 1 Aminosäuren nach diesen Positionen.

Hierunter sind insbesondere Mittel mit solchen Hybrid-Amylasen bevorzugt, die als Teilsequenz die Teilsequenz der Aminosäure-Positionen 19 bis 76 der α-Amylase aus *Bacillus amyloliquefaciens* (SEQ ID NO. 4) und als weitere Teilsequenz die Teilsequenz der Aminosäure-Positionen 433 bis 483 der α-Amylase aus *Bacillus licheniformis* (SEQ ID NO. 2) aufweisen.

Denn beispielsweise aus der Arbeit von Conrad et al. ist bekannt, daß der zwischen den Positionen 34 und 76 der α-Amylase von *B. amyloliquefaciens* für das Gesamtmolekül eine thermostabilisierende Funktion ausübt. Wahrscheinlich ist jedoch, daß nicht allein diese Region für diese Eigenschaft verantwortlich ist, sondern die Thermostabilität auf dem Wechselspiel mit weiteren Regionen der Amylase beruhen kann. So haben die Enzyme, die gleichzeitig die Teilsequenz 19 bis 76 der α-Amylase aus *B. amyloliquefaciens* und die der Aminosäurepositionen 433 bis 483 der α-Amylase aus *B. licheniformis* aufweisen, gute Beiträge zu den Wasch-, beziehungsweise Reinigungsleistungen entsprechender Rezepturen gezeigt. Das kann daraus resultieren, daß gewisse, möglicherweise noch unbekannte Strukturmerkmale den Enzymen unter entsprechenden Bedingungen (Temperatur, pH-Wert, Ionenstärke, mechanische Belastung) die Fähigkeit zur Ablösung von Stärke oder stärkeähnlichen Anschmutzungen auf Fasern oder harten Oberflächen verleihen. Dies belegen die Beispiele der vorliegenden Anmeldung.

Aus diesem Grund sind ganz besonders solche Mittel bevorzugt, die Hybrid-Amylasen enthalten, die zu der von AL76 (SEQ ID NO. 8) oder zu der von AL112 (SEQ ID NO. 10) oder zu der von LAL19-433 (SEQ ID NO. 16) mindestens zu 98%, und zunehmend bevorzugt zu 98,25%, 98,5%, 98,75%, 99%, 99,25%, 99,5%, 99,75% und zu 100% identisch sind.

Denn diese Enzyme haben in den ausgeführten Beispielen die Wasch-, beziehungsweise Reinigungsleistung der dort getesteten Mittel gegenüber stärkehaltigen oder stärkeähnlichen Anschmutzungen verbessert. Teilweise lag ihr Beitrag ebenso hoch wie der von etablierten Wasch- oder Reingungsmittelamylasen oder sogar darüber.

Die guten Wasch- und Reinigungsleistungen der Enzyme AL76, AL112 und LAL19-433 lassen vermuten, daß die Kombination der Bereiche von Position 19 bis 76 der α-Amylase aus *B amyloliquefaciens* mit der C-terminalen Domäne der α-Amylase *B licheniformis,* d.h. ab Position 433 nicht nur auf die Stabilität der betreffenden Varianten, sondern auch auf die Wasch-, beziehungsweise Reinigungsleistungen besonders günstig wirkt.

In den Schutz des Homologiebereichs dieser besonders bevorzugten Enzyme AL76 (SEQ ID NO. 8), AL112 (SEQ ID NO. 10) und LAL19-433 (SEQ ID NO. 16) fallen jeweils auch solche, die über einzelne Punktmutationen von diesen abgeleitet werden können. Hierzu gehören zunehmend bevorzugt solche mit 1, 2 oder 3 Punktmutationen, vorzugsweise Substitutionen in den Aminosäure-Positionen, die den Positionen 134, 320 und 412 in der Zählung der α-Amylase aus *B. licheniformis,* beziehungsweise den Positionen 132, 320 und 412 in der Zählung der α-Amylase aus *B. amyloliquefaciens* entsprechen.

Darunter sind insbesondere die AL76-Varianten R132L, A318S und/oder T410A (gemäß der Zählung von SEQ ID NO. 8), die AL112-Varianten R132L, A318S und/oder T410A (gemäß der Zählung von SEQ ID NO. 10), beziehungsweise die LAL19-433-Varianten Q134L, A322S und/oder T414A (gemäß der Zählung von SEQ ID NO. 16) bevorzugt. Denn beispielsweise die Variante AL76 R132L/A318S/T410A hat in Waschversuchen, die denen der Beispiele 2 bis 7 der vorliegenden Anmeldung entsprechen, überraschend positive Ergebnisse erbracht, die denen von AL76 vergleichbar sind.

Weitere und/oder andere Punktmutationen können auch in anderen Positionen der verschiedenen, entsprechend dem oben Gesagten zunehmend bevorzugten Hybrid-Amylasen durchgeführt werden. Hierunter sind Substitutionen einzelner Aminosäuren bevorzugt und darunter ganz besonders solche, die eine Eigenschaft der Amylasen hinsichtlich ihres Einsatzes in Wasch- oder Reinigungsmitteln verbessern. Dazu gehören beispielsweise: Stabilität gegenüber oxidierenden Verhältnissen, gegenüber erhöhten Temperaturen, insbesondere zwischen 40 und 95°C, gegenüber denaturierenden Agentien wie Tensiden oder Komplexbildnern, Verbesserung der Calcium-Bindung, Anpassung des pH-Optimums, Wechselwirkung mit dem zu hydrolysierenden Substrat oder Veränderung der spezifischen oder unspezifischen Bindung an die Oberfläche des Reinigungsguts.

Die Herstellung derartiger Variationen kann durch zielgerichtete Mutagenese (site directed mutagenesis) beispielsweise über sogenannte Mismatch-Primer erfolgen, wie sie dem Fachmann aus dem Stand der Technik vertraut ist. Alternativ können auch statistische Verfahren (random mutagenesis) kombiniert mit anschließender Selektion auf einen Beitrag zur Waschleistung einer entsprechenden Wasch- oder Reinigungsmittelrezeptur zum Einsatz kommen. Derartige Mutagenseverfahren werden für Hybrid-Amylasen beispielsweise in den Anmeldungen WO 96/23874, WO 97/41213 und WO 00/60059 offenbart. Die Vorselektion der erhaltenen Varianten kann wie in Beispiel 1 in der vorliegenden Anmeldung beschrieben und ihr weiterer Test gemäß den nachfolgenden Beispielen erfolgen.

Weitere Ausführungsformen dieses Erfindungsgegenstand sind Wasch- oder Reinigungsmittel, die dadurch gekennzeichnet sind, daß sie eine durch Deletion von jeweils nicht mehr als 5 zusammenhängenden Aminosäuren, und zunehmend bevorzugt von jeweils nicht mehr als 4, 3, 2, und besonders bevorzugt von jeweils nur einer Aminosäure erhaltene erfindungsrelevante Hybrid-Amylase enthalten.

Solche Hybrid-Amylasen können beispielsweie dadurch erhalten werden, daß vorzugsweise bei der oben beschriebenen Fusion der Ausgangssequenzen oder auch unabhängig davon kürzere Sequenzbereiche gezielt weggelassen worden sind. Hierbei kann es sich beispielsweise um kleinere destabilisierende Elemente handeln oder um nicht die Wasch-, beziehungsweise Reinigungsleistung beeinträchtigende Verluste von einzelnen Aminosäuren bei der Fusion.

Weitere Ausführungsformen dieses Erfindungsgegenstand sind Wasch- oder Reinigungsmittel, die dadurch gekennzeichnet sind, daß sie als Hybrid-Amylase ein amylolytisches durch Insertionsmutation erhaltenes oder amylolytisches chimäres Protein enthalten, welches wenigstens in einem, die amylolytische Aktivität verleihenden Teil aus einer der zuvor beschriebenen Hybrid-Amylase identisch ist.

So ist es beispielsweise in Anwendung der Lehre von WO 99/57250 möglich, solch ein Enzym zur Erhöhung der Wechselwirkung mit dem Substrat mit einer Cellulose-Bindungsdomäne zu koppeln. In analoger Weise können beispielsweise auch andere wasch- oder reinigungsaktive Enzyme mit einer erfindungsrelevanten Hybrid-Amylase fusioniert werden. Es ist dabei prinzipiell unerheblich, ob die Fusion N- oder C-terminal oder über Insertion erfolgt; entscheidend ist lediglich die Erreichung des Ziels, ein leistungsverbessertes Mittel zur Verfügung zu stellen.

Weitere Ausführungsformen dieses Erfindungsgegenstand sind Wasch- oder Reinigungsmittel, die dadurch gekennzeichnet sind, daß sie ein amylolytisches Derivat einer der oben beschriebenen Hybrid-Amylasen enthalten.

Dabei handelt es sich insbesondere um Moleküle, die durch chemische Kopplung von niedermolekularen Verbindungen oder von Polymeren erhalten werden können. Der Sinn einer derartigen Modifikation, etwa in Anlehnung an die Lehre von WO 00/22103, kann in der Reduzierung der allergenen Wirkung, gemäß WO 99/58651 in einer Optimierung der enzymatischen Parameter oder gemäß EP 1088887 in der Erhöhung der Stabilität liegen. Beispielsweise in Anwendung der Lehre von WO 00/26354 können die Proteine auch über Glykosylierung modifiziert werden.

Weitere Ausführungsformen dieses Erfindungsgegenstand sind Wasch- oder Reinigungsmittel, die dadurch gekennzeichnet sind, daß sie ein amylolytisches Protein oder Derivat enthalten, das wenigstens eine durch die Hybridbildung entstandene antigene Determinante mit einem der zuvor genannten Proteine oder Derivate gemeinsam hat.

Denn die erfindungsrelevanten Übergänge von einer Teilsequenz zur anderen charakterisieren die erfindungsgemäß verbesserten Mittel und die dementsprechend bevorzugten Ausführungsformen. Zum anderen kann über die immunologische Kreuzreaktion leicht der Nachweis erbracht werden, daß eine erfindungswesentliche Hybrid-Amylase in einem entsprechenden Mittel tatsächlich aktiv ist.

Erfindungsgemäße Mittel enthalten die erfindungswesentlichen Hybrid-Amylasen oder deren Derivate vorzugsweise in Mengen von 0,000001 Gewichts-Prozent bis 5 Gew.-%, und zunehmend bevorzugt von 0,00005 bis 4 Gew.-%, von 0,00001 bis 3 Gew.-%, von 0,0001 bis 2 Gew.-%, und besonders bevorzugt von 0,001 bis 1 Gew.-%.

Mit den erfindungsgemäßen Wasch- oder Reinigungsmitteln sind alle denkbaren Reinigungsmittelarten gemeint, sowohl Konzentrate, als auch unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Reinigungsmittelart stellt eine Ausführungsform der vorliegenden Erfindung dar, sofern sie um ein erfindungsgemäßes Protein bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Neben einem erfindungswesentlichen Enzym enthält das erfindungsgemäße Mittel gegebenenfalls weitere Inhaltsstoffe wie Tenside, zum Beispiel nichtionische, anionische und/oder amphotere Tenside, und/oder Bleichmittel, und/oder Builder, sowie gegebenenfalls weitere übliche Inhaltsstoffe.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₆-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden können, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II), 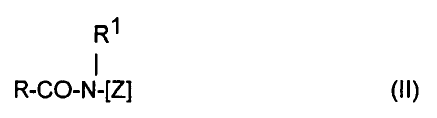 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 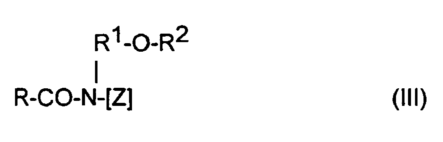 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische. Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.-%, bezogen auf das fertige Mittel, enthalten sein.

Erfindungsgemäße Mittel können Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.-% und insbesondere 10 bis 20 Gew.-%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart.

Um beim Waschen bei Temperaturen von 60°C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere *n*-Nonanoyl- oder Isononanoyloxybenzolsulfonat (*n*-beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 4443 177 bekannten Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, *n*-Nonanoyl- oder Isononanoyloxybenzolsulfonat (*n*- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Aminkomplexe sind als Bleichkatalysatoren geeignet, wobei solche Verbindungen bevorzugt eingesetzt werden, die in der DE 197 09 284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅-yH₂O bevorzugt. Im Handel befinden sich derartige Verbindungen beispielsweise unter der Bezeichnung SKS^{®} (Fa. Clariant). So handelt es sich bei SKS-6^{®} vorwiegend um ein δ-Natriumdisilicat mit der Formel Na₂Si₂O₅·yH₂O, bei SKS-7^{®} vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O₅·yH₂O, im Handel unter den Bezeichnungen SKS-9^{®} beziehungsweise SKS-10^{®} (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 19601 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Beide Substanzen sind farblose, in Wasser mit alkalischer Reaktion lösliche Kristalle. Na₄P₂O₇ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇. existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=₃. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei systemunbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472 042, WO 97/25399, und EP 755 944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50.Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder - ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können erfindungsgemäßen Zusammensetzungen ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuern und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- und Reinigungsmittel kann gegebenenfalls als weitere übliche Inhaltsstoffe Sequestrierungsmittel, Elektrolyte und weitere Hilfsstoffe, wie optische Aufheller, Vergrauungsinhibitoren, Silberkorrosionsinhibitoren, Farbübertragungsinhibitoren, Schauminhibitoren, Abrasivstoffe, Farb- und/oder Duftstoffe, sowie mikrobielle Wirkstoffe und/oder UV-Absorbenzien enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppenendverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0 241984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272 033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethylendgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl,. Neroliol, Orangenschalenöl und Sandelholzöl. Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von K. H. Wallhäußer in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, Iod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅)-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-O-chlorophenyldiguanido- N₅,N₅')-hexan-dihydrochlorid, 1,6-Di-(N₁,N₁'-2,6-dichlorophenyldiguanido-N₅, N₅')hexan-dihydrochlorid, 1,6-Di-[N₁, N₁'-beta-(p-methoxyphenyl) diguanido-N₅,N₅']-hexane-dihydrochlorid, 1,6-Di-(N₁,N_{1'}-alpha-methyl-beta-phenyldiguanido-N₅, N_{5'})-hexan-dihydrochlorid, 1,6-Di-(N₁,N₁'-p-nitrophenytdiguanido-N₅,N₅')hexan-dihydrochlorid, omega:omega-Di-(N₁,N_{1'}-phenyldiguanido-N₅,N₅')-di-n-propy)ether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chforophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecantetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅`) hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormeta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, Iodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²)(R³)(R⁴) N⁺ X- auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X- Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzylammoniumchlorid, CAS No. 8001-54-5), Benzalkon B (m,p-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethylammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammonium-chloride wie Di-*n*-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklylbenzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1 ,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal 122 (1996), S. 543 zu entnehmen.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Ein erfindungswesentliches Protein kann besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Bei Proteinen, die aus Mikroorganismen erhalten werden, ist eine Inhibierung der Proteolyse besonders kritisch, weil die meisten Mikroorganismen verschiedene Proteasen als Verdauungsenzyme in die umgebenden Medien sekretieren. Diese können die interessierenden Proteine während der nachfolgenden Aufreinigungstufen erheblich schädigen. Auch in Wasch- und Reinigungsmitteln können erfindungswesentliche Proteine mit Proteasen vergesellschaftet sein, und bedürfen deshalb eines besonderen Schutzes.

Auch erfindungsgemäße Mittel können zu diesem Zweck Stabilisatoren enthalten. Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa gemäß WO 95/12655 ortho-substituierte, gemäß WO 92/19707 meta-substituierte und gemäß US 5 972 873 para-substituierte Phenylboronsäuren, beziehungsweise deren Salze oder Ester. In den Anmeldungen WO 98/13460 und EP 583 534 werden Peptidaldehyde, das heißt Oligopeptide mit reduziertem C-Terminus, und zwar solche aus 2-50 Monomeren, zur reversiblen Inhibierung von Wasch- und Reinigungsmittelproteasen offenbart. Zu den peptidischen reversiblen Proteaseinhibitoren gehört unter anderem Ovomucoid (WO 93/00418). Beispielsweise mit der Anmeldung WO 00/01826 werden spezifische, reversible Peptid-Inhibitoren für die Protease Subtilisin zur Verwendung in Protease-haltigen Mitteln offenbart, und mit WO 00/01831 entsprechende Fusionsproteine aus Protease und Inhibitor.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise aus den Anmeldungen EP 0 378 261 und WO 97/05227 bekannt ist, wie Bernsteinsäure, andere Dicarbonsäuren oder Salze der genannten Säuren. Mit der Anmeldung DE 196 50 537 werden für diesen Zweck endgruppenverschlossene Fettsäureamidalkoxylate offenbart. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Nach einer neueren Anmeldung (EP 0 965 268) schützt auch Di-Glycerinphosphat gegen Denaturierung durch physikalische Einflüsse. Ebenso werden Calciumsalze verwendet, wie beispielsweise Calciumacetat oder das in der Patentschrift EP 0 028 865 für diesen Zweck offenbarte Calcium-Formiat, und Magnesiumsalze, etwa gemäß der europäischen Anmeldung EP 0 378 262.

Polyamid-Oligomere (WO 99/43780) oder polymere Verbindungen wie Lignin (WO 97/00932), wasserlösliche Vinyl-Copolymere (EP 828 762) oder, wie in EP 702 712 offenbart, Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere (EP 587 550 und EP 581751) wirken gleichzeitig als Enzymstabilisatoren und als Farbübertragungsinhibitoren. Andere polymere Stabilisatoren sind die in WO 97/05227 neben anderen Bestandteilen offenbarten linearen C₈-C₁₈ Polyoxyalkylene. Alkylpolyglycoside könnten wie in den Anmeldungen WO 97/43377 und WO 98/45396 die enzymatischen Komponenten des erfindungsgemäßen Mittels stabilisieren und sogar in ihrer Leistung steigern. Vernetzte N-haltige Verbindungen, wie in WO 98/17764 offenbart, erfüllen eine Doppelfunktion als Soil-release-Agentien und als Enzym-Stabilisatoren. Hydrophobes, nichtionisches Polymer wirkt in einer Mischung mit anderen Stabilisatoren gemäß der Anmeldung WO 97/32958 stabilisierend auf eine Cellulase, so daß sich solche oder ähnliche Bestandteile auch für das erfindungswesentliche Enzym eignen könnten.

Reduktionsmittel und Antioxidantien erhöhen, wie unter anderem in EP 780 466 offenbart, die Stabilität der Enzyme gegenüber oxidativem Zerfall. Schwefelhaltige Reduktionsmittel sind beispielsweise aus den Patentschriften EP 0 080748 und EP 0 080 223 bekannt. Andere Beispiele sind Natrium-Sulfit (EP 533 239) und reduzierende Zucker (EP 656 058).

Vielfach werden auch Kombinatonen von Stabilisatoren verwendet, beispielsweise aus Polyolen, Borsäure und/oder Borax in der Anmeldung WO 96/31589, die Kombination von Borsäure oder Borat, reduzierenden Salzen und Bernsteinsäure oder anderen Dicarbonsäuren in der Anmeldung EP 126 505 oder die Kombination von Borsäure oder Borat mit Polyolen oder Polyaminoverbindungen und mit reduzierenden Salzen, wie in der Anmeldung EP 080 223 offenbart. Die Wirkung von Peptid-Aldehyd-Stabilisatoren wird gemäß WO 98/13462 durch die Kombination mit Borsäure und/oder Borsäurederivaten und Polyolen gesteigert und gemäß WO 98/13459 durch die zusätzliche Verwendung von Calcium-Ionen noch weiter verstärkt.

Mittel mit stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Enzyme wie Proteasen, Amylasen, Lipasen oder Cellulasen werden seit Jahrzehnten als aktive Komponenten in Wasch- und Reinigungsmitteln eingesetzt. Ihr jeweiliger Beitrag zur Wasch-, beziehungsweise Reinigungsleistung des betreffenden Mittels ist im Fall von Protease die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es günstigenfalls zu Synergieeffekten zwischen den Enzymen und den übrigen Bestandteilen kommt.

Erfindungsgemäße Mittel können zusätzlich zu dem erfindungswesentlichen Protein andere amylolytische Enzyme, insbesondere α-Amylasen enthalten. Darunter können sich auch die für die Verwendung in Wasch- und Reinigungsmitteln etablierten Enzyme befinden. Beispiele für kommerziell erhältliche Amylasen sind BAN^{®}, Termamyl^{®}, Purastar^{®}, Amylase-LT^{®}, Maxamyl^{®}, Duramyl^{®} und/oder Purafect^{®} OxAm. Dies ist dann angebracht, wenn sich die verschiedenen Enzyme einander zu ergänzen vermögen. Solch eine Ergänzung kann beispielsweise in regulatorischer Hinsicht erfolgen, etwa durch gegenseitige Aktivierung oder durch Inaktivierung. Sie kann sich beispielsweise dadurch ergeben, daß mindestens ein nicht zu den bekannten α-Amylasen homologer Teil des erfindungswesentlichen Enzyms Einfluß auf die nicht erfindungswesentlichen amylolytischen Aktivitäten nimmt. Die gemeinsame Verwendung kann aber auch aufgrund abweichender Substratspezifitäten sinnvoll sein. Beides sind Ausführungsformen der vorliegenden Erfindung.

Insbesondere an chemisch diversen Anschmutzungen kann es vorteilhaft sein, amylolytische Enzyme in Wasch- und Reinigungsmitteln zusammen mit anderen wasch- und/oder reinigungsaktiven Enzymen einzusetzen. Somit stellen Wasch- oder Reinigungsmittel, die über ein erfindungsgemäßes Protein hinaus durch zusätzlich weitere Enzyme gekennzeichnet sind, bevorzugte Ausführungsformen der vorliegenden Erfindung dar.

Dazu gehören neben weiteren Amylasen beispielsweise Proteasen, aber auch Lipasen, Cutinasen, Esterasen, Pullulanasen, Cellulasen, Hemicellulasen und/oder Xylanasen, sowie deren Gemische. Besonders bevorzugt sind Proteasen, Lipasen, β-Glucanasen und/oder Cellulasen. Weitere Enzyme erweitern die Reinigunsgleistung entsprechender Mittel um ihre jeweils spezifische enzymatische Leistung. Dazu gehören beispielsweise Oxidoreductasen oder Peroxidasen als Komponenten von enzymatischen Bleichsystemen, zum Beispiel Laccasen (WO 00/39306), β-Glucanasen (WO 99/06515 und WO 99/06516) oder Pektin-lösende Enzyme (WO 00/42145), die insbesondere in Spezialwaschmitteln zum Einsatz kommen.

Beispiele für kommerziell erhältliche Enzyme zum Gebrauch in erfindungsgemäßen Mitteln sind Proteasen wie Subtilisin BPN', Properase^{®}, BLAP^{®}, Optimase^{®}, Opticlean^{®}, Maxatase^{®}, Maxacal^{®}, Maxapem^{®}, Alcalase^{®}, Esperase^{®}, Savinase^{®}, Durazym^{®}, Everlase^{®} und/oder Purafect^{®}G oder Purafect^{®}OxP und Lipasen wie Lipolase^{®}, Lipomax^{®}, Lumafast^{®} und/oder Lipozym^{®}.

Die Proteaseaktivität in derartigen Mitteln kann nach der in Tenside, Bd. 7 (1970), S. 125-132 beschriebenen Methode ermittelt werden. Sie wird dementsprechend in PE (Protease-Einheiten) angegeben. Die Proteaseaktivität bevorzugter Mittel kann bis zu 1.500.000 Proteaseeinheiten pro Gramm Zubereitung betragen (PE, bestimmt nach der in Tenside, Bd. 7 (1970), S. 125-132 beschriebenen Methode).

Hinsichtlich ihrer Gewinnung kommen unter den verwendbaren Enzymen in erster Linie die aus Mikroorganismen, wie Bakterien oder Pilzen, gewonnenen in Frage, beispielsweise aus *Bacillus subtilis, Bacillus licheniformis, Streptomyces griseus, Humicola lanuginosa, Humicola insolens, Pseudomonas pseudoalcaligenes* oder *Pseudomonas cepacia,* insbesondere die von diesen Stämmen natürlicherweise gebildeten Enzymgemische, beziehungsweise Gemische mit denen anderer Stämme. Sie werden in bekannter Weise durch Fermentationsprozesse aus geeigneten Mikroorganismen gewonnen, die zum Beispiel in den deutschen Offenlegungsschriften DE 19 40 488 und DE 21 21 397, den US-amerikanischen Patentschriften US 3 623957 und US 4 264 738, der europäischen Patentanmeldung EP 006 638, sowie der internationalen Patentanmeldung WO 91/02792 beschrieben sind.

Auch diese gegebenenfalls zusätzlich verwendeten Enzyme können, wie zum Beispiel in der europäischen Patentschrift EP 0 564 476 oder in der internationalen Patentanmeldungen WO 94/23005 beschrieben, an Trägerstoffen adsorbiert und/oder in Hüllsubstanzen eingebettet sein, um sie gegen vorzeitige Inaktivierung zu schützen. Sie sind in Waschmitteln vorzugsweise in Mengen bis zu 10 Gew.%, insbesondere von 0,2 Gew.-% bis 2 Gew.-%, enthalten, wobei besonders bevorzugt gegen oxidativen Abbau stabilisierte Enzyme, wie zum Beispiel aus den internationalen Patentanmeldungen WO 94/18314 bekannt, eingesetzt werden.

Erfindungsgemäße Mittel können, beispielsweise um die enthaltenen Wirkstoffe zeitlich oder räumlich voneinander getrennt freizusetzen, aus mehreren Phasen bestehen. Dabei kann es sich um Phasen in verschiedenen Aggregatzuständen, insbesondere aber um zwei Phasen in denselben Aggregatzuständen handeln.

Erfindungsgemäße Mittel, die aus mehreren festen Komponenten zusammengesetzt sind, können auf einfache Weise dadurch hergestellt werden, daß verschiedene feste Komponenten, insbesondere Pulver, Granulate oder Extrudate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 199 18 267 offenbart. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden. Ein solches Verkapselungsverfahren wird mit der deutschen Anmeldung DE 199 56 382 beschrieben.

Eine andere Möglichkeit, ein festes erfindungsgemäßes Mittel zur Verfügung zu stellen, ist das Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kN/cm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Besonders vorteilhaft ist es, wenn in mehrphasigen Mitteln wenigstens eine der Phasen ein Amylase-sensitives Material, insbesondere Stärke enthält oder von diesem zumindest teilweise umgeben oder beschichtet ist. Auf diese Weise wird diese Phase mechanisch stabilisiert und/oder gegen Einflüsse von außen geschützt und gleichzeitig über eine in der Waschflotte wirksame Amylase angegriffen, so daß die Freisetzung der Inhaltsstoffe erleichtert wird.

Flüssigen, gelförmigen oder pastösen erfindungsgemäßen Mitteln können die Enzyme, und auch ein erfindungswesentliches Protein ausgehend von einer nach dem Stand der Technik durchgeführten Proteingewinnung und Präparation in konzentrierter wäßriger oder nichtwäßriger Lösung beispielsweise in flüssiger Form, etwa als Lösung, Suspension oder Emulsion, aber auch in Gelform oder verkapselt oder als getrocknetes Pulver zugesetzt werden. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform der vorliegenden Erfindung sind solche flüssigen, gelförmigen oder pastösen Mittel, denen ein erfindungswesentliches Protein und/oder eines der anderen enthaltenen Proteine und/oder einer der anderen enthaltenene Inhaltsstoffe in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und dem erfindungswesentlichen amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Der Einsatz des erfindungswesentlichen Enzyms für diesen Zweck stellt somit eine bevorzugte Ausführungsform der vorliegenden Erfindung dar.

Die Inhaltsstoffe von Wasch- und Reinigungsmitteln vermögen sich geeigneterweise gegenseitig in ihrer Leistung zu unterstützen. Die synergistische Verwendung von Amylase und Farbübertragungsinhibitoren zur Steigerung der Reinigungsleistung wird beispielsweise mit der Anmeldung WO 99/63035 offenbart. Es ist auch bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigern können, so aus der Anmeldung WO 98/45396. Ebenso ist es möglich, daß auch eine erfindungswesentliche amylolytische Aktivität durch einen der übrigen, oben aufgeführten Bestandteile modifiziert, insbesondere stabilisiert und/oder gesteigert wird. Entsprechend abgestimmte Rezepturen für erfindungsgemäße Mittel stellen somit besonders bevorzugte Ausführungsformen der vorliegenden Erfindung dar.

Entsprechend den bisherigen Ausführungen können auch Verfahren zur Reinigung von Textilien oder von harten Oberflächen, dadurch verbessert werden, daß in wenigstens einem der Verfahrensschritte eine erfindungswesentliche Hybrid-Amylase oder ein Derivat davon aktiv wird. Es handelt sich dann um erfindungsgemäße Verfahren.

Vorzugsweise sind derartige Verfahren dadurch gekennzeichnet, daß in wenigstens einem der Verfahrensschritte ein Mittel gemäß der bisherigen Beschreibung eingesetzt wird.

Besonders bevorzugt sind derartige Verfahren dadurch gekennzeichnet, daß das amylolytische Protein oder Derivat, beispielsweise in gängigen Haushaltsgeschirrspülmaschinen oder Haushaltswaschmaschinen vorzugsweise von 0,01 mg bis 400 mg, bevorzugt von 0,02 mg bis 200 mg, besonders bevorzugt von 0,02 mg bis 100 mg des eingesetzt wird.

Günstigenfalls ergeben sich dabei Konzentrationswerte von 0,0005 bis 20 mg pro I, bevorzugt 0,005 bis 10 mg pro I, besonders bevorzugt 0,005 bis 8mg des amylolytischen Proteins pro I Waschflotte. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem. 177 (1948), S. 751-766) bestimmt werden.

Entsprechend den bisherigen Ausführungen stellt auch die Verwendung einer erfindungswesentlichen Hybridamylase oder eines Derivats davon allein oder zusammen mit mindestens einem anderen reinigungsaktiven oder die Reinigungswirkung unterstützenden Wirkstoff zur Reinigung von Textilien oder von harten Oberflächen eine Ausführungsform der vorliegenden Erfindung dar.

Vorzugsweise geschieht dies durch Verwendung eines erfindungsgemäßen Mittels.

Das erfindungsgemäße Mittel oder ein erfindungswesentliches amylolytisches Protein wird bevorzugt in den oben angegebenen Mengenbereichen eingesetzt, so daß sich günstigerweise in der Waschflotte die oben angegebenen Konzentrationswerte für das amylolytische Protein ergeben. Diese Dosierung kann, je nach Reinigungsproblem vom Hersteller des Mittels oder vom Endverbraucher vorgenommen werden.

Eine weitere Verwendungsmöglichkeit für eine erfindungswesentliche Hybrid-Amylase oder ein Derivat davon stellt die zur Aktivierung der eigenen oder anderer Phasen dar, wenn sie allein oder zusammen mit mindestens einem anderen reinigungsaktiven oder die Reinigungswirkung unterstützenden Wirkstoff in einem aus mehr als einer Phase bestehenden Wasch- oder Reinigungsmittel bereitgestellt wird.

Eine weitere Verwendungsmöglichkeit für eine erfindungswesentliche Hybrid-Amylase oder ein Derivat davon stellt die zur Freisetzung der Inhaltsstoffe aus den Kapseln dar, wenn sie allein oder zusammen mit mindestens einem anderen reinigungsaktiven oder die Reinigungswirkung unterstützenden Wirkstoff in einem Wasch- oder Reinigungsmittel mit verkapselten Inhaltsstoffen bereitgestellt wird.

In einem weiteren Aspekt der vorliegenden Erfindung werden Verfahren aufgezeigt, nach denen die Wasch-, beziehungsweise Reinigungsleistung eines Wasch- oder Reinigungsmittels durch Entwicklung neuer Amylasen verbessert werden kann. Diese bestehen darin, daß jeweils mindestens mehr als eine Aminosäure umfassende Teilsequenzen der α-Amylasen aus *Bacillus amyloliquefaciens* und *Bacillus licheniformis* in jeweils homologer Lage zu einer amylolytisch wirksamen Hybrid-Amylase fusioniert und diese dem Mittel zugegeben wird.

Derartige Verfahren sind an sich aus dem Stand der Technik bekannt und basieren auf molekularbiologischen Techniken, wie sie beispielsweise aus dem Handbuch von Fritsch, Sambrook und Maniatis "Molecular cloning: a laboratory manual", Cold Spring Harbour Laboratory Press, New York, 1989 bekannt sind oder in Nachschlagewerken wie dem "Lexikon der Biochemie", Spektrum Akademischer Verlag, Berlin, 1999 zusammengestellt sind. Vorzugsweise bauen sie auf den Nukleotidsequenzen der Ausgangsmoleküle auf, die im Sequenzprotokoll unter SEQ ID NO. 1 und 3 angegeben sind. Beispielsweise in der Arbeit von Conrad et al. (siehe oben) wird die Erstellung einer Schar von Molekülen über *In vivo*-Rekombination der zugehörigen Gene vorgestellt. Weitere Möglichkeiten zur Erzeugung erfindungsrelevanter Hybride sind ebenfalls bereits oben vorgestellt worden.

Entsprechend den oben gemachten Angaben sind jene Verfahren bevorzugt, bei denen die auf die Ausgangsmoleküle zurückzuführenden Teilsequenzen der Hybrid-Amylasen länger als 7, vorzugsweise länger als 14, besonders bevorzugt 21 bis 462 Aminosäuren lang sind.

Entsprechend den oben gemachten Angaben sind jene Verfahren bevorzugt, bei denen das Hybridprotein aus 3 oder aus 2 entsprechend den Ausgangssequenzen einander ergänzenden Teilsequenzen zusammengesetzt ist.

Entsprechend den oben gemachten Angaben sind jene Verfahren zunehmend bevorzugt, bei denen die Fusionspunkte der Hybrid-Amylase innerhalb eines Bereiches von 10, 9, 8, 7, 6, 5, 4, 3, 2 und 1 Aminosäure vor bis 10, 9, 8, 7, 6, 5, 4, 3, 2 und 1 Aminosäuren nach und ganz besonders genau an einer oder mehreren der Positionen 17, 34, 76, 108, 112, 142, 147, 149, 151, 163, 174, 179, 185, 191, 198, 207, 231, 234, 244, 256, 263, 276, 431, 84, 99, 429, 201, 19, 433 und 153 gemäß der Numerierung der SEQ ID NO. 4 liegen.

Entsprechend den oben gemachten Angaben sind jene Verfahren zunehmend bevorzugt, bei denen die erhaltenen Hybrid-Amylasen zusätzlich eine oder mehrere Deletionen von jeweils nicht mehr als 5, 4, 3 und 2 zusammenhängenden Aminosäuren, besonders bevorzugt von jeweils nur einer Aminosäure erhalten.

Derartige Mutationen können mit an sich bekannten Teilschritten erzeugt werden, beispielsweise in Zusammenhang mit der Fusion. Sie können aber auch anderen Positionen als den Fusionsstellen eingeführt werden.

Entsprechend den oben gemachten Angaben sind jene Verfahren bevorzugt, bei denen die erhaltenen Hybrid-Amylasen zusätzlich in mindestens einer Position einen Aminosäureaustausch erfahren. Hierunter sind Substitutionen in 1, 2 oder 3 der Positionen 132, 320 und 412 gemäß der Zählung von SEQ ID NO. 4 zunehmend bevorzugt.

Entsprechend den oben gemachten Angaben sind jene Verfahren bevorzugt, bei denen die erhaltenen Hybrid-Amylasen zusätzlich Insertionen erhalten oder ein amylolytisches chimäres Protein darstellen.

Entsprechend den oben gemachten Angaben sind jene Verfahren bevorzugt, bei denen die erhaltenen Hybrid-Amylasen zusätzlich derivatisiert werden, beispiesweise durch Kopplung an ein anderes Protein, an ein Polymer oder durch eine sonstige chemische Modifikation.

Besonders bevorzugt sind jeweils solche Verfahren, die dadurch gekennzeichnet sind, daß zur Bildung der Hybrid-Amylasen Nukleinsäuren eingesetzt werden, die in den entsprechenden Teilbereichen die in SEQ ID NO. 1 und SEQ ID NO. 3 angegebenen Nukleotidsequenzen oder zu diesen synonyme Nukleotidsequenzen aufweisen. Wie oben ausgeführt kann der Austausch synonymer Codons dann besonders sinnvoll sein, wenn unter Beibehaltung der Aminosäuresequenz die Nukleotidsequenz geändert werden soll, um bestimmte Restriktiosschnittstellen einzuführen. Die erhaltenen mutierten Gene können in an sich bekannter Weise amplifiziert, kloniert und zur Produktion der entsprechenden Varianten eingesetzt werden.

### Beispiele

### Beispiel 1

### Gewinnung der Hybrid-Amylasen AL 34, AL76, AL112, AL 256, ALA 34-84, LAL 19-153 und LAL 19-433 sowie die Bestimmung deren Enzymaktivitäten

Alle molekularbiologischen und mikrobiologischen Schritte folgen Standardmethoden, wie sie beispielsweise in dem handbuch von Fritsch, Sambrook und Maniatis "Molecular cloning: a laboratory manual", Cold Spring Harbour Laboratory Press, New York, 1989, beschrieben worden sind.

Bakterienstämme, die die betreffenden Enzyme bilden, können wie in in der Publikation "Hybrid Bacillus amyloliquefaciens X Bacillus licheniformis α-Amylases. Construction, properties and sequence determinats" (1995) von B. Conrad, V. Hoang, A. Polley und J. Hofemeister, Eur. J. Biochem., 230, S. 481-490, beschrieben ist erhalten werden.

Die Anzucht der Bakterienstämme, die die Hybrid-Amylasen AL 34, AL76, AL112, AL 256, ALA 34-84, LAL 19-153 und LAL 19-433 exprimieren, erfolgte in 250 ml-Kulturen in 1 l-Schüttelkolben bei 37°C und 200 upm. Als Medium diente: MLBSP (10 g/l Casiton; 20 g/l Trypton, 10 g/l Hefeextrakt, jeweils von der Fa. Becton Dickinson, Cockeysville; 5 g/l NaCl; 27 g/l Natrium-Succinat; 100 mg/l MgSO₄*7 H₂O; 75 mg/l CaCl₂*2 H₂O; 0,5 µM MnCl₂; 0,5 µM FeSO₄; 2 %(w/v) Glucose; 50 mM PIPES-Puffer (aus einer 1 M Stammlösung mit pH 7,2); 75 mM KPO₄ (aus einer 1,5 M Stammlösung mit pH 7,0); pH= 7,0, eingestellt mit KOH) und 5 µg/ml Chloramphenicol. Als Inokulum diente jeweils 1 % des Zielvolumens einer 24 h-Vorkultur in demselben Medium.

Nach 64 h wurde der Überstand durch Zentrifugation bei 4°C gewonnen. Der Überstand wurde mit 50% Propylenglycol stabilisiert und daraus die Amylase-Aktivität in TAU bestimmt. Dafür wird das Substrat p-Nitrophenyl-α-D-Maltoheptaglucosid verwendet, dessen endständige Glucoseeinheit durch eine Benzylidengruppe blockiert ist. Dieses wird durch Amylase zu freiem p-Nitrophenyl-Oligosaccharid gespalten, welches seinerseits mittels der Hilfesenzyme Glucoamylase und alpha-Glucosidase zu Glucose und p-Nitrophenol umgesetzt wird. Damit ist die Menge an freigesetztem p-Nitrophenol der Amylase-Aktivität proportional. Die Messung erfolgt beispielsweise mit dem Quick-Start^{®}-Testkit der Fa. Abbott, Abott Park, Illinois, USA. Die Absorptionszunahme (405 nm) im Testansatz wird bei 37°C über 3 min gegen einen Blank-Wert mittels Photometer detektiert. Die Kalibrierung erfolgt über einen Enzymstandard von bekannter Aktivität (zum Beispiel Maxamyl^{®}/Purastar^{®} 2900 der Firma Genencor mit 2900 TAU/g). Die Auswertung erfolgt mittels Auftragung der Absorptionsdifferenz dE (405 nm) pro min gegen die Enzymkonzentration des Standards. 1 TAU ist die Menge an Enzym, die unter den gegebenen Bedingungen in der Lage ist, in einer Minute 1 µmol des Substrates zu spalten.

Es wurden die in folgender Tabelle 1 angegebenen Aktivitätswerte erhalten:

**Tabelle 1: Aktivitätswerte ausgewählter Hybrid-Amylasen.**

| **Hybrid-Amylase** | **Aktivität (TAU/ml)** |
|---|---|
| AL 34 | 2,5 |
| AL76 | 2,2 |
| AL112 | 4,3 |
| AL 256 | 4,9 |
| ALA 34-84 | 9,5 |
| LAL 19-153 | 2,3 |
| LAL 19-433 | 1,7 |

### Beispiel 2

Baumwolltextilien wurden standardisiert mit folgenden Anschmutzungen versehen: A: Mousse au chocolat; B: Haferflocken mit Kakao, C: Kartoffelstärke, und anhand des auf diese Weise präparierten Materials verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 30 °C gewaschen. Die Dosierung lag bei 5,88 g des jeweiligen Waschmittels pro I Waschflotte. Die Wasserhärte betrug 16° deutscher Härte.

Als Kontroll-Waschmittel diente für A und B eine Waschmittel-Basis-Rezeptur folgender Zusammensetzung (alle Angaben in Gewichts-Prozent): 4 % lineares Natrium-Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, 1 % Proteasegranulat, Rest: Wasser, optischer Aufheller, Parfüm, Salze. Sie wurde für die verschiedenen Versuchsreihen mit verschiedenen Amylasen versetzt, so daß sich jeweils eine Endkonzentration von 44 TAU an amylolytischem Enzym pro I Waschflotte ergab.

Mit den erfindungswesentlichen amylolytischen Enzymen AL76, AL112 und LAL19-433 wurden Termamyl^{®}, Duramyl^{®} und BAN^{®} (Hersteller jeweils: Novo Nordisk A/S, Bagsvaerd, Dänemark) verglichen. Für die Verunreinigung C wurde dieselbe Basis-Rezeptur verwendet, allerdings ohne Protease, und wie A und B als Kontrolle verwendet, beziehungsweise mit den Amylasen versetzt.

Der Weißheitsgrad der Textilien wurde im CIELAB-System mit dem Meßgerät Minolta CR 310 vor und nach dem Waschen im Vergleich zu einem Standard gemessen, der auf 100 % normiert wurde. Die Differenzen aus den erhaltenen Werten werden für die jeweiligen Versuche in folgender Tabelle 2 zusammengestellt. Angegeben sind die Mittelwerte aus jeweils 5 Messungen. Sie erlauben einen unmittelbaren Rückschluß auf den Beitrag des enthaltenen Enzyms auf die Waschleistung des verwendeten Mittels.

**Tabelle 2:**

| **Basis-Waschmittel mit** | **A** | **B** | **C** |
|---|---|---|---|
| **AL76** | 28,7 | 27,5 | 11,7 |
| **AL112** | 24,1 | 24,0 | 14,3 |
| **LAL19-433** | 26,4 | 26,3 | 12,5 |
| **Termamyl^{®}** | 25,9 | 22,7 | 12,3 |
| **Duramyl^{®}** | 28,6 | 23,4 | 14,3 |
| **BAN®** | 22,5 | 22,0 | 13,2 |
| **Kontrolle ohne Amylase** | 22,9 | 22,2 | 10,0 |
| Standardabw. | 1,3 | 0,6 | 2,2 |

Man erkennt, daß im Falle der Verschmutzung A die Hybridamylase AL76 mindestens ebensogut ist wie das beste der drei Vergleichsenzyme; im Fall der Verschmutzung B sind AL76 und LAL19-433 diesen deutlich überlegen. Im Fall der Verschmutzung C ist die Hybrid-Amylase AL112 genausogut wie das beste der drei Vergleichsenzyme. Die jeweils übrigen untersuchten erfindungswesentlichen Enzyme zeigen Waschleistungen, die denen der etablierten Enzyme zumindest vergleichbar sind. Diese Ergebnisse sind umso bemerkenswerter, als in allen Mitteln ein Bleichmittel und in A und B zusätzlich eine Protease enthalten sind, worauf enthaltene Enzyme im allgemeinen sehr empfindlich reagieren.

### Beispiel 3

Baumwoll-Textilien wurden standardisiert mit den Anschmutzungen B (Haferflocken mit Kakao) und D (Haferflocken mit Kakao und wenig Milch) verunreinigt. Die launderometrische Untersuchung erfolgte wie in Beispiel 2, allerdings unter Verwendung einer Bleichmittel-freien Waschmittel-Basis-Rezeptur; sie enthielt jeweils in Gewichts-Prozent: 14 % Na-Alkylbenzolsulfonat, 6 % Na-Fettalkoholsulfonat, 6 % 7-fach ethoxylierter C₁₂-C₁₈-Fettalkohol, 1 % Seife, 25 % Zeolith Na-A, 10 % Na-Carbonat, 5 % polymeres Polycarboxylat (Sokalan CP5), 11 % Trinatriumcitrat-dihydrat, 4 % Citronensäure, 1 % teilchenförmig konfektionierter Schauminhibitor, 1 % Proteasegranulat, 5 % Natriumsulfat, Rest: Wasser und Salze. Diese Basis-Rezeptur wurde für die verschiedenen Versuchsreihen mit den verschiedenen Amylasen versetzt, so daß sich jeweils eine Endkonzentration von 33,5 TAU an amylolytischem Enzym pro I Waschflotte ergab. Mit den erfindungswesentlichen Hybridamylasen AL76, AL112 und LAL19-433 wurden Termamyl®, Duramyl® und BAN® (Hersteller jeweils: Novo Nordisk A/S, Bagsværd, Dänemark) verglichen. Die Dosierung lag bei 4,45 g des jeweiligen Waschmittels pro I Waschflotte.

Nach dem Waschen wurde der Weißheitsgrad der gewaschenen Textilien wie in dem vorangegangenen Beispiel bestimmt. Die jeweils erhaltenen Differenzwerte werden in folgender Tabelle 3 zusammengestellt. Es handelt sich jeweils um die Mittelwerte aus 5 Messungen, welche wiederum einen unmittelbaren Rückschluß auf den Beitrag des jeweiligen Enzyms auf die Waschleistung des Mittels zulassen.

**Tabelle 3:**

| **Basis-Waschmittel mit** | **B** | **D** |
|---|---|---|
| **AL76** | 29,5 | 17,3 |
| **AL112** | 30,8 | 17,0 |
| **LAL19-433** | 31,8 | 18,9 |
| **Termamyl^{®}** | 29,3 | 15,0 |
| **Duramyl^{®}** | 29,2 | 16,7 |
| **BAN^{®}** | 28,9 | 15,6 |
| **Kontrolle ohne Amylase** | 28,5 | 14,5 |
| Standardabw. | 0,6 | 1,2 |

In beiden untersuchten Fällen zeigen die drei erfindungswesentlichen Enzyme, insbesondere LAL19-433, auch in dieser bleichmittelfreien Waschmittel-Rezeptur solche Beiträge zu den Waschleistungen der betreffenden Mitteln, die besser als die der drei Vergleichsenzyme oder ihnen innerhalb der Fehlergrenze zumindest ebenbürtig sind.

### Beispiel 4

Baumwoll-Textilien wurden standardisiert mit zwei verschiedenen Typen kommerziell erhältlicher Arten Milchkakao (E und F) verunreinigt. Damit erfolgte die launderometrische Untersuchung wie in Beispiel 2 beschrieben. Als Kontroll-Waschmittel diente die Waschmittel-Basis-Rezeptur des Beispiels 3, jedoch ohne Protease. Sie wurde für die verschiedenen Versuchsreihen wie in Beispiel 3 mit den verschiedenen Amylasen versetzt und in gleicher Dosierung eingesetzt.

Nach dem Waschen wurde der Weißheitsgrad der gewaschenen Textilien im Vergleich zu dem von Bariumsulfat gemessen, der auf 100 % normiert wurde. Die Messung erfolgte an einem Spektrometer Datacolor SF500-2 bei 460 nm (UV-Sperrfilter 3), 30 mm Blende, ohne Glanz, Lichtart D65, 10°, d/8°. Die erhaltenen Ergebnisse werden als Prozent an Remission, das heißt als Prozentangaben im Vergleich zu Bariumsulfat in folgender Tabelle 4 zusammengestellt; dort sind ebenfalls die jeweiligen Anfangswerte angegeben. Angegeben sind die Mittelwerte aus jeweils 5 Messungen. Sie erlauben einen unmittelbaren Rückschluß auf den Beitrag des jeweils enthaltenen amylolytischen Enzyms auf die Waschleistung des verwendeten Mittels.

**Tabelle 4:**

| **Basis-Waschmittel mit** | **E** | **F** |
|---|---|---|
| **AL 76** | 70,8 | 41,6 |
| **AL112** | 70,3 | 39,2 |
| **LAL 19-433** | 71,4 | 40,9 |
| **Termamyl^{®}** | 67,3 | 39,7 |
| **Duramyl^{®}** | 68,3 | 40,5 |
| **BAN^{®}** | 68,7 | 39,8 |
| **Kontrolle ohne Amylase** | 61,1 | 31,4 |
| Anfangswert | 21,1 | 25,0 |
| Standardabw. | 1,0 | 1,2 |

Im Fall der Anschmutzung E sind alle drei getesteten erfindungswesentlichen Hybridamylasen den drei etablierten Enzymen eindeutig überlegen; im Fall der Anschmutzung F sind sie den etablierten Enzymen innerhalb der Fehlergrenze ungefähr gleichwertig.

### Beispiel 5

Baumwoll-Textilien wurden standardisiert mit den Anschmutzungen G (Kartoffelpüree mit Tomatenmark als Farbindikator), H (Milchkakao), D (Haferflocken mit Kakao und wenig Milch), E und F (zwei Typen kommerziell erhältlicher Arten Milchkakao) versehen und unter den in Beispiel 2 ausgeführten Versuchsbedingungen launderometrisch untersucht. Die Konzentration des jeweils verwendeten Waschmittels lag wiederum bei 5,88 g pro I Waschflotte.

Dafür wurde wiederum die in Beispiel 2 angegebene Waschmittel-Grundrezeptur verwendet, mit Bleiche, aber ohne sonstige Enzyme. Die α-Amylasen wurden jedoch in einer Konzentration von 125 TAU pro I Waschflotte eingesetzt. Im Vergleich zu den drei bekannten Referenzenzymen Termamyl^{®}, Duramyl^{®} und BAN^{®} (Hersteller jeweils: Novo Nordisk A/S, Bagsværd, Dänemark) wurden hier die beiden Hybridamylasen AL76 und LAL untersucht.

Wie in Beispiel 2 beschrieben wurde für die Anschmutzungen G, H und D der erreichte Weißheitsgrad der Textilien in Prozent über das CIELAB-System ermittelt; für die Anschmutzungen E und F wurden, wie in Beispiel 4 beschrieben, Vergleichsmessungen zu Bariumsulfat angestellt. Die aus jeweils 5 Messungen erhaltenen Mittelwerte sind in folgender Tabelle 5 zusammengestellt.

**Tabelle 5:**

| **Basis-Waschmittel mit** | **G** | **H** | **D** | **E** | **F** |
|---|---|---|---|---|---|
| **AL76** | 20,9 | 27,9 | 18,6 | 70,8 | 46,9 |
| **LAL19-433** | 19,7 | 24,3 | 11,8 | 69,3 | 44,8 |
| **Termamyl^{®}** | 20,2 | 22,5 | 16,3 | 69,7 | 46,6 |
| **Duramyl^{®}** | 20,7 | 22,2 | 13,2 | 70,5 | 45,8 |
| **BAN^{®}** | 20,5 | 22,7 | 15,4 | 68,2 | 44,7 |
| **Kontrolle ohne Amylase** | 9,9 | 20,8 | 8,7 | 55,0 | 34,6 |
| Anfangswert | - | - | - | 21,1 | 23,5 |
| Standardabw. | 2,4 | 0,7 | 2,2 | 1,2 | 1,9 |

Bei der Anschmutzung G liegt der Wert für die Hybridamylase AL76 innerhalb des Fehlerbalkens über dem höchsten der drei Vergleichsenzyme, ist also als mindestens gleichwertig anzusehen. Bei den Anschmutzungen H und D zeigt AL76 mit Abstand die besten Werte, im Fall von H gefolgt von der Hybridamylase LAL und dann erst den zum Vergleich herangezogenen etablierten Waschmittelamylasen.

Auch bei den Anschmutzungen E und F zeigt AL76 keine niedrigeren Werte als die Vergleichsenzyme, und LAL19-433 weist Werte auf, die nur geringfügig schlechter sind als die der drei Vergleichsenzyme. Auch dieses Beispiel belegt also, daß die Hybridamylasen, insbesondere AL76 und LAL19-433 hinsichtlich ihrer Beiträge zu den Waschleistungen entsprechender Waschmittel den etablierten Enzymen durchaus vergleichbar sind.

### Beispiel 6

Gefäße mit harten, glatten Oberflächen wurden standardisiert mit in Wasser gequollenen Haferflocken angeschmutzt und anschließend bei 45°C mit dem Normalprogramm einer Haushaltsgeschirrspülmaschine Typ Miele^{®} G 575 gespült. Pro Spülgang wurden 20 g Spülmittel verwendet; die Wasserhärte betrug 16° deutscher Härte.

Als Spülmittel diente folgende Basis-Rezeptur (alle Angaben jeweils in Gewichts-Prozent): 55 % Natriumtripolyphosphat (berechnet als wasserfrei), 4 % amorphes Natriumdisilikat (berechnet als wasserfrei), 22 % Natriumcarbonat, 9 % Natriumperborat, 2 % TAED, 2 % nichtionisches Tensid, 1,4 % Proteasegranulat, Rest: Wasser, Farbstoffe, Parfüm. Diese Basis-Rezeptur wurde für die verschiedenen Versuche mit verschiedenen Amylasen versetzt, nämlich Termamyl^{®}, Duramyl^{®}, BAN^{®} (Hersteller jeweils: Novo Nordisk A/S, Bagsværd, Dänemark), beziehungsweise jeweils mit einem erfindungswesentlichen amylolytischen Enzym. Die Enzyme wurde in wirksamen Mengen von jeweils, wie gemäß der in Beispiel 2 angegebenen Methode bestimmt, 150 TAU an amylolytischer Aktivität pro Reinigungsgang eingesetzt. Bei den eingesetzten Vertretern erfindungswesentlicher Hybridamylasen handelte es sich um: ALA34-84, AL34, AL76, AL112, AL256, LAL19-153, LAL19-433.

Nach dem Spülen wurde der Abtrag der Anschmutzung nach Anfärbung mit Iod mittels der lod-Stärke-Reaktion visuell auf einer Skala von 0 (= unverändert, d.h. sehr starke Anschmutzung) bis 10 (= keinerlei Anschmutzung erkennbar) benotet. Die erhaltenen Ergebnisse werden in folgender Tabelle 6 zusammengestellt. Angegeben sind dort die Mittelwerte aus jeweils 9 Messungen. Sie erlauben einen unmittelbaren Rückschluß auf den Beitrag des enthaltenen Enzyms auf die Reinigungsleistung des verwendeten Mittels.

**Tabelle 6:**

| **Basis-Waschmittel mit** | **1** |
|---|---|
| **ALA34-84** | 1, 8 |
| **AL34** | 2,1 |
| **AL76** | 2,1 |
| **AL112** | 2,1 |
| **AL256** | 2,0 |
| **LAL19-153** | 1,6 |
| **LAL19-433** | 2,0 |
| **Termamyl**^{®} | 1,8 |
| **Duramyl^{®}** | 2,0 |
| **BAN^{®}** | 1,7 |
| **Kontrolle ohne Amylase** | 1,3 |

An der dieser stärkehaltigen Anschmutzung zeigen die drei Hybridamylasen AL34, AL76 und AL112 Beiträge zu den Reinigungsleistungen der Mittel, die über dem Wert für das beste der drei Referenzenzyme liegen. Nur die α-Amylase LAL19-153 zeigt einen Wert, der unter dem schlechtesten der Referenzenzyme, aber oberhalb der Kontrolle liegt. Damit zeigen die erfindungswesentlichen Hybridamylasen bei 45°C Beiträge zu Reinigungsleistungen erfindungsgemäßer Mittel, die denen der für diesen Zweck etablierten Enzymen vergleichbar, beziehungsweise überlegen sind.

### Beispiel 7

Gefäße mit harten, glatten Oberflächen wurden standardisiert mit folgenden Anschmutzungen versehen: J (DIN Haferflocken), I (in Wasser gequollene Haferflocken) und K (Mix-Stärke).

An diesen erfolgte die Untersuchung zum Beitrag der betreffenden amylolytischen Enzyme zur Reinigungsleistung einer Geschirrspülmittelrezeptur wie im vorangegangenen Beispiel beschrieben. Der einzige Unterschied bestand darin, daß bei 55°C gespült wurde.

Untersucht wurden in entsprechender Weise die Hybridamylasen AL112, LAL 19-433 und AL76, wiederum im Vergleich zu den α-Amylasen Termamyl^{®}, Duramyl^{®} und BAN^{®} (Hersteller jeweils: Novo Nordisk A/S, Bagsvaerd, Dänemark). Für die Anschmutzungen J und I erfolgte die Auswertung, wie im vorangegangen Beispiel beschrieben, visuell auf einer Skala von 0 bis 10. Der Abtrag der Anschmutzung K wurde gravimetrisch in Prozent ermittelt. Dafür wurde die Differenz aus dem Gewicht des verunreinigten und anschließend gespülten Gefäßes und dem Anfangsgewicht des Gefäßes in Relation zu der Gewichtsdifferenz des nicht gespülten Gefäßes zum Anfangsgewicht gesetzt. Diese Relation kann als Prozent Abtrag angesehen werden. Das Ergebnis ist in folgender Tabelle 7 dargestellt.

**Tabelle 7:**

| **Basis-Waschmittel mit** | **J** | **I** | **K** |
|---|---|---|---|
| **AL112** | 7,0 | 92,9 | 79,0 |
| **LAL1 9-433** | 5,9 | 92,5 | 73,0 |
| **AL76** | 7,5 | 96,9 | 90,1 |
| **Termamyl^{®}** | 6,7 | 94,5 | 53,4 |
| **Duramyl^{®}** | 6,9 | 95,1 | 88,7 |
| **BAN^{®}** | 6,2 | 92,3 | 77,3 |
| **Kontrolle ohne Amylase** | 5,3 | 70,5 | 27,2 |

Bei diesem Versuch leistet AL76 an der Anschmutzung J den deutlich größten Beitrag zur Waschleistung des betreffenden Mittels, gefolgt von AL112 und dann erst den Vergleichsenzymen. Besonders bei Reinigung bei 55°C liegen die Beiträge der Hybridamylase AL76 an der Anschmutzung I deutlich über denen aller drei Referenzenzyme; die der beiden anderen getesteten Hybridamylasen liegen bei vergleichbaren Werten. Das gleiche gilt für die Anschmutzung K.

### Beschreibung der Figuren

- **Figur 1:**: Schema zur Konstruktion der besonders erfindungswesentlichen Hybridamylasen AL34, AL76, AL112, AL256, ALA34-84, LAL19-153 und LAL19-433.

Die jeweils schwarz gekennzeichneten Bereiche leiten sich von der α-Amylase aus *B. amyloliquefaciens* (B.A.; oberster Balken) her, und die weiß gekennzeichneten von der α-Amylase aus *B. licheniformis* (B.L.; zweiter Balken). Oberhalb des ersten Balkens sind die Fusionspunkte in der Numerierung der Aminosäuresequenzen der maturen α-Amylase von *B*. *amyloliquefaciens* (SEQ ID NO. 4) angegeben.
- **Figur 2:**: Alignment der Aminosäuresequenzen der Präproteine (Precursor) der α-Amylasen aus *B*. *licheniformis* (B.L.) und *B. amyloliquefaciens.* (B.A.).
Das Leader-Peptid der α-Amylase aus *B*. *licheniformis* umfaßt 29 Aminosäuren, das der α-Amylase aus *B. amyloliquefaciens* 31. Die maturen Proteine sind jeweils 483 Aminosäuren lang.
Fett hervorgehoben sind jeweils die erste Aminosäure des maturen Proteins sowie die Aminosäuren, die in der Zählung des maturen Proteins aus *B. amyloliquefaciens* den Positionen 19, 34, 76, 84, 112, 153, 256 und 433 entsprechen. Wechsel von der einen zur anderen Sequenz hinter einer, beziehungsweise zweier dieser Positionen kennzeichnen die jeweils besonders bevorzugten Ausführungsformen der vorliegenden Erfindung.

## Patentansprüche

1. Wasch- oder Reinigungsmittel, **dadurch gekennzeichnet, daß** es ein amylolytisches Hybrid-Protein enthält, wobei es sich bei dem Hybrid-Protein um ein solches handelt, das zu AL76 (SEQ ID NO. 8) mindestens zu 98 %, vorzugsweise zu 99 %, besonders bevorzugt zu 100 % identisch ist.

2. Wasch- oder Reinigungsmittel gemäß Anspruch 1, **dadurch gekennzeichnet, daß** es als amylolytisches Protein eine durch Deletion von jeweils nicht mehr als 5 zusammenhängenden Aminosäuren, vorzugsweise von jeweils nicht mehr als 3 zusammenhängenden Aminosäuren, besonders bevorzugt von jeweils nur einer Aminosäure oder durch Substitution einer Aminosäure erhaltene Hybrid-Amylase gemäß Anspruch 1 enthält.

3. Wasch- oder Reinigungsmittel gemäß Anspruch 1, **dadurch gekennzeichnet, daß** es als amylolytisches Protein ein amylolytisches durch Insertionsmutation erhaltenes oder amylolytisches chimäres Protein enthält, welches durch Einfügen eines Proteinfragments in die Ausgangssequenz gemäß Anspruch 1 erhalten worden ist.

4. Mittel gemäß einem der Ansprüche 1 bis 3, **dadurch gekennzeichnet, daß** es 0,000001 Gewichts-Prozent bis 5 Gew.-%, insbesondere 0,00001 bis 3 Gew.-% des amylolytischen Proteins enthält.

5. Mittel gemäß einem der Ansprüche 1 bis 4, **dadurch gekennzeichnet, daß** es zusätzlich eine oder mehrere andere amylolytische Proteine, insbesondere α-Amylasen enthält.

6. Mittel gemäß einem der Ansprüche 1 bis 5, **dadurch gekennzeichnet, daß** es zusätzlich andere Enzyme, insbesondere eine oder mehrere Proteasen, Lipasen, β-Glucanasen und/oder Cellulasen enthält.

7. Mittel gemäß einem der Ansprüche 1 bis 6, **dadurch gekennzeichnet, daß** es aus mehr als einer Phase besteht.

8. Mittel gemäß einem der Ansprüche 1 bis 7, **dadurch gekennzeichnet, daß** es fest ist und mindestens zwei verschiedene feste Komponenten, insbesondere Pulver, Granulate oder Extrudate, in insgesamt loser Form miteinander vermischt vorliegen.

9. Mittel gemäß einem der Ansprüche 1 bis 8, **dadurch gekennzeichnet, daß** mindestens zwei feste Phasen miteinander verbunden vorliegen, insbesondere nach einem gemeinsamen Kompaktierungsschritt.

10. Mittel gemäß einem der Ansprüche 7 bis 9, **dadurch gekennzeichnet, daß** wenigstens eine der Phasen ein Amylase-sensitives Material, insbesondere Stärke enthält oder von diesem zumindest teilweise umgeben oder beschichtet ist.

11. Mittel gemäß einem der Ansprüche 1 bis 10, **dadurch gekennzeichnet, daß** es insgesamt flüssig, gelförmig oder pastös ist und das enthaltene Protein und/oder wenigstens eines der enthaltenen Enzyme und/oder wenigstens eine der sonstigen enthaltenen Komponenten einzeln oder zusammen mit anderen Bestandteilen verkapselt vorliegt, bevorzugt in Mikrokapseln, besonders bevorzugt in solchen aus einem Amylase-sensitiven Material.

12. Mittel gemäß einem der Ansprüche 1 bis 11, **dadurch gekennzeichnet, daß** die amylolytische Aktivität durch einen der sonstigen Bestandteile des Mittels modifiziert, insbesondere stabilisiert und/oder in ihrem Beitrag zur Wasch-, beziehungsweise Reinigungsleistung des Mittels gesteigert wird.

13. Verfahren zur Reinigung von Textilien oder von harten Oberflächen, **dadurch gekennzeichnet, daß** in wenigstens einem der Verfahrensschritte ein amylolytisches Protein gemäß einem der Ansprüche 1 bis 3 aktiv wird.

14. Verfahren zur Reinigung von Textilien oder von harten Oberflächen, **dadurch gekennzeichnet, daß** in wenigstens einem der Verfahrensschritte ein Mittel gemäß einem der Ansprüche 1 bis 12 eingesetzt wird.

15. Verfahren nach Anspruch 13 oder 14, **dadurch gekennzeichnet, daß** das amylolytische Protein in dem betreffenden Verfahrensschritt in einer Menge von 0,01 mg bis 400 mg, vorzugsweise von 0,02 mg bis 200 mg, besonders bevorzugt von 0,02 bis 100 mg pro Anwendung eingesetzt wird.

16. Verwendung eines amylolytischen Proteins gemäß einem der Ansprüche 1 bis 3 allein oder zusammen mit mindestens einem anderen reinigungsaktiven oder die Reinigungswirkung unterstützenden Wirkstoff zur Reinigung von Textilien oder von harten Oberflächen.

17. Verwendung eines Mittels gemäß einem der Ansprüche 1 bis 12 zur Reinigung von Textilien oder von harten Oberflächen.

18. Verwendung nach Anspruch 16 oder 17, **dadurch gekennzeichnet, daß** pro Anwendung, vorzugsweise pro Anwendung in einer Geschirrspülmaschine oder einer Waschmaschine 0,01 mg bis 400 mg, vorzugsweise von 0,02 mg bis 200 mg, besonders bevorzugt von 0,02 bis 100 mg des amylolytischen Proteins eingesetzt werden.

19. Verwendung eines amylolytischen Proteins gemäß einem der Ansprüche 1 bis 3 allein oder zusammen mit mindestens einem anderen reinigungsaktiven oder die Reinigungswirkung unterstützenden Wirkstoff in einem aus mehr als einer Phase bestehenden Wasch- oder Reinigungsmittel zur Aktivierung der eigenen oder anderer Phasen.

20. Verwendung eines amylolytischen Proteins gemäß einem der Ansprüche 1 bis 3 allein oder zusammen mit mindestens einem anderen reinigungsaktiven oder die Reinigungswirkung unterstützenden Wirkstoff in einem Wasch- oder Reinigungsmittel mit verkapselten Inhaltsstoffen zur Freisetzung der Inhaltsstoffe aus den Kapseln.

21. Verfahren zur Verbesserung der Wasch- oder Reinigungsleistung eines Wasch- oder Reinigungsmittels, **dadurch gekennzeichnet, daß** eine amylolytisch wirksame Hybrid-Amylase dem Mittel zugegeben wird, wobei es sich bei dem Hybrid-Protein um ein solches handelt, das zu AL76 (SEQ ID NO. 8) mindestens zu 98 %, vorzugsweise zu 99 %, besonders bevorzugt zu 100 % identisch ist.

22. Verfahren nach Anspruch 21, **dadurch gekennzeichnet, daß** zur Bildung der Hybrid-Amylase Nukleinsäuren eingesetzt werden, die in den entsprechenden Teilbereichen die in SEQ ID NO. 1 und SEQ ID NO. 3 angegebenen Nukleotidsequenzen oder zu diesen synonyme Nukleotidsequenzen aufweisen.

## Claims

1. Detergent or cleaning agent, **characterized in that** it comprises an amylolytic hybrid protein, wherein the hybrid protein is one which is at least 98%, preferably 99%, particularly preferably 100%, identical to AL76 (SEQ ID No. 8) .

2. Detergent or cleaning agent according to Claim 1, **characterized in that** it comprises as amylolytic protein a hybrid amylase as claimed in Claim 1, obtained by deletion of in each case no more than 5 contiguous amino acids, preferably of in each case no more than 3 contiguous amino acids, particularly preferably of in each case only one amino acid, or by substitution of an amino acid.

3. Detergent or cleaning agent according to Claim 1, **characterized in that** it comprises as amylolytic protein an amylolytic protein obtained by insertion mutation or an amylolytic chimeric protein which has been obtained by inserting a protein fragment into the starting sequence according to Claim 1.

4. Agent according to any of Claims 1 to 3, **characterized in that** it comprises from 0.000001 percent by weight to 5% by weight, in particular from 0.00001 to 3% by weight, of the amylolytic protein.

5. Agent according to any of Claims 1 to 4, **characterized in that** it additionally comprises one or more other amylolytic proteins, in particular α-amylases.

6. Agent according to any of Claims 1 to 5, **characterized in that** it additionally comprises other enzymes, in particular one or more proteases, lipases, β-glucanases and/or cellulases.

7. Agent according to any of Claims 1 to 6, **characterized in that** it comprises more than one phase.

8. Agent according to any of Claims 1 to 7, **characterized in that** it is solid and that at least two different solid components, in particular powders, granules or extrudates, are present in an overall loose mixture.

9. Agent according to any of Claims 1 to 8, **characterized in that** at least two solid phases bonded together are present, in particular after a joint compacting step.

10. Agent according to any of Claims 7 to 9, **characterized in that** at least one of the phases comprises an amylase-sensitive material, in particular starch, or is, at least partly, surrounded by or coated with said material.

11. Agent according to any of Claims 1 to 10, **characterized in that** it is overall in liquid, gel or paste form and that the protein present and/or at least one of the enzymes present and/or at least one of the other components present is, either individually or together with other components, in encapsulated form, preferably in microcapsules, particularly preferably in those made of an amylase-sensitive material.

12. Agent according to any of Claims 1 to 11, **characterized in that** any of the other components of the agent modifies, in particular stabilizes, the amylolytic activity and/or increases the contribution thereof to the washing or cleaning performance of the agent.

13. Method for cleaning textiles or hard surfaces, **characterized in that** in at least one of the method steps an amylolytic protein as claimed in any of Claims 1 to 3 becomes active.

14. Method for cleaning textiles or hard surfaces, **characterized in that** in at least one of the method steps an agent as claimed in any of Claims 1 to 12 is used.

15. Method according to Claim 13 or 14, **characterized in that** the amylolytic protein is used in the method step in question in an amount of from 0.01 mg to 400 mg, preferably from 0.02 mg to 200 mg, particularly preferably from 0.02 to 100 mg, per application.

16. Use of an amylolytic protein as claimed in any of Claims 1 to 3 alone or together with at least one other cleaning-active ingredient or active ingredient supporting the cleaning action for cleaning textiles or hard surfaces.

17. Use of an agent according to any of Claims 1 to 12 for cleaning textiles or hard surfaces.

18. Use according to Claim 16 or 17, **characterized in that** per application, preferably per application in a dishwasher or a washing machine, 0.01 mg to 400 mg, preferably from 0.02 mg to 200 mg, particularly preferably from 0.02 to 100 mg, of the amylolytic protein are used.

19. Use of an amylolytic protein according to any of Claims 1 to 3 alone or together with at least one other cleaning-active ingredient or active ingredient supporting the cleaning action in a detergent or cleaning agent comprising more than one phase for activating its own or other phases.

20. Use of an amylolytic protein according to any of Claims 1 to 3 alone or together with at least one other cleaning-active ingredient or active ingredient supporting the cleaning action in a detergent or cleaning agent containing encapsulated ingredients for releasing the ingredients from the capsules.

21. Method for improving the washing or cleaning performance of a detergent or cleaning agent, **characterized in that** an amylolytically active hybrid amylase is added to the agent, with the hybrid protein being at least 98%, preferably 99%, particularly preferably 100%, identical to AL76 (SEQ ID NO. 8).

22. Method according to Claim 21, **characterized in that** the hybrid amylases are formed by using nucleic acids which have in the corresponding partial regions the nucleotide sequences indicated in SEQ ID No. 1 and SEQ ID No. 3 or nucleotide sequences synonymous thereto.

## Revendications

1. Agent de lavage et de nettoyage, **caractérisé en ce qu'**il contient une protéine hybride amylolytique, où il s'agit, pour la protéine hybride, d'une protéine qui est identique à AL76 (SEQ ID NO. 8) à raison d'au moins 98%, de préférence à raison de 99%, de manière particulièrement préférée à raison de 100%.

2. Agent de lavage et de nettoyage selon la revendication 1, **caractérisé en ce qu'**il contient, comme protéine amylolytique une amylase hybride selon la revendication 1 obtenue par délétion d'à chaque fois pas plus de 5 acides aminés assemblés, de préférence d'à chaque fois pas plus de 3 acides aminés assemblés, de manière particulièrement préférée d'à chaque fois seulement un acide aminé ou par substitution d'un acide aminé.

3. Agent de lavage et de nettoyage selon la revendication 1, **caractérisé en ce qu'**il contient comme protéine amylolytique une protéine amylolytique obtenue par mutation par insertion ou une protéine amylolytique chimère, qui a été obtenue par insertion d'un fragment de protéine dans la séquence de départ selon la revendication 1.

4. Agent selon l'une quelconque des revendications 1 à 3, **caractérisé en ce qu'**il contient 0,000001 % en poids à 5% en poids, en particulier 0,00001 à 3% en poids de la protéine amylolytique.

5. Agent selon l'une quelconque des revendications 1 à 4, **caractérisé en ce qu'**il contient en outre une ou plusieurs autres protéines amylolytiques, en particulier des α-amylases.

6. Agent selon l'une quelconque des revendications 1 à 5, **caractérisé en ce qu'**il contient en outre d'autres enzymes, en particulier une ou plusieurs protéases, lipases, β-glucanases et/ou cellulases.

7. Agent selon l'une quelconque des revendications 1 à 6, **caractérisé en ce qu'**il est constitué par plus d'une phase.

8. Agent selon l'une quelconque des revendications 1 à 7, **caractérisé en ce qu'**il est solide et qu'au moins deux composants solides différents, en particulier des poudres, des granulats ou des produits extrudés se trouvent sous une forme globalement en vrac, mélangés les uns aux autres.

9. Agent selon l'une quelconque des revendications 1 à 8, **caractérisé en ce qu'**au moins deux phases solides se trouvent sous une forme assemblée l'une à l'autre, en particulier après une étape de compactage commune.

10. Agent selon l'une quelconque des revendications 7 à 9, **caractérisé en ce qu'**au moins une des phases contient un matériau sensible aux amylases, en particulier de l'amidon, ou est entourée ou revêtue, au moins partiellement, par celui-ci.

11. Agent selon l'une quelconque des revendications 1 à 10, **caractérisé en ce qu'**il est globalement liquide, en forme de gel ou de pâte et la protéine contenue et/ou au moins une des enzymes contenues et/ou au moins un des autres composants contenus se trouvent, individuellement ou avec d'autres composants, sous forme encapsulée, de préférence dans des microcapsules, de manière particulièrement préférée dans des microcapsules en un matériau sensible aux amylases.

12. Agent selon l'une quelconque des revendications 1 à 11, **caractérisé en ce que** l'activité amylolytique est modifiée par un des autres constituants de l'agent, en particulier stabilisée et/ou augmentée en ce qui concerne sa contribution à la puissance de lavage resp. de nettoyage de l'agent.

13. Procédé pour le nettoyage de textiles ou de surfaces dures, **caractérisé en ce que** dans au moins une des étapes de procédé, une protéine amylolytique selon l'une quelconque des revendications 1 à 3 devient active.

14. Procédé pour le nettoyage de textiles ou de surfaces dures, **caractérisé en ce que** dans au moins une des étapes de procédé, on utilise un agent selon l'une quelconque des revendications 1 à 12.

15. Procédé selon la revendication 13 ou 14, **caractérisé en ce que** la protéine amylolytique dans l'étape de procédé concernée est utilisée en une quantité de 0,01 mg à 400 mg, de préférence de 0,02 mg à 200 mg, de manière particulièrement préférée de 0,02 à 100 mg par utilisation.

16. Utilisation d'une protéine amylolytique selon l'une quelconque des revendications 1 à 3, seule ou ensemble avec au moins une autre substance active en nettoyage ou soutenant l'effet de nettoyage pour le nettoyage de textiles ou de surfaces dures.

17. Utilisation d'un agent selon l'une quelconque des revendications 1 à 12 pour le nettoyage de textiles ou de surfaces dures.

18. Utilisation selon la revendication 16 ou 17, **caractérisée en ce qu'**on utilise par utilisation, de préférence par utilisation dans un lave-vaisselle ou un lave-linge 0,01 mg à 400 mg, de préférence 0,02 mg à 200 mg, de manière particulièrement préférée 0,02 à 100 mg de la protéine amylolytique.

19. Utilisation d'une protéine amylolytique selon l'une quelconque des revendications 1 à 3, seule ou ensemble avec au moins une autre substance active en nettoyage ou soutenant l'effet de nettoyage dans un agent de lavage ou de nettoyage constitué de plus d'une phase pour l'activation de ses phases ou d'autres phases.

20. Utilisation d'une protéine amylolytique selon l'une quelconque des revendications 1 à 3, seule ou ensemble avec au moins une autre substance active en nettoyage ou soutenant l'effet de nettoyage dans un agent de lavage ou de nettoyage avec des constituants encapsulés pour la libération des constituants des capsules.

21. Procédé pour améliorer la puissance de lavage ou de nettoyage d'un agent de lavage ou de nettoyage **caractérisé en ce qu'**on ajoute une amylase hybride à activité amylolytique à l'agent, où il s'agit, pour la protéine hybride, d'une protéine qui est identique à AL76 (SEQ ID NO. 8) à raison d'au moins 98%, de préférence à raison de 99%, de manière particulièrement préférée à raison de 100%.

22. Procédé selon la revendication 21, **caractérisé en ce qu'**on utilise, pour la formation de l'amylase hybride, des acides nucléiques qui présentent, dans les zones partielles correspondantes, les séquences de nucléotides indiquées dans les SEQ ID NO. 1 et SEQ ID NO. 3 ou des séquences de nucléotides synonymes de celles-ci.
